# EUROPEAN PATENT SPECIFICATION

(11) **EP 1 272 484 B1**
(45) Date of publication and mention of the grant of the patent: **20.07.2005**
(21) Application number: 01919702.9
(22) Date of filing: 06.04.2001
(51) Int. Cl.: C07D 401/14, C07D 413/14, C07D 405/14, C07D 471/10, C07D 491/10, C07D 401/12, A61K 31/5395, A61P 25/00, A61P 11/00, A61P 1/00

(54) **BENZOAMIDE PIPERIDINE COMPOUNDS AS SUBSTANCE P ANTAGONISTS**
BENZOAMID-PIPERIDIN VERBINDUNGEN ALS SUBSTANZ P-ANTAGONISTEN
COMPOSES CONTENANT BENZOAMIDE PIPERIDINE COMME DES ANTAGONISTES SUBSTANCE P

(30) Priority: 10.04.2000 US 195922 P; 20.06.2000 US 212922 P
(43) Date of publication of application: 08.01.2003
(73) Proprietor: Pfizer Products Inc., Groton, Connecticut 06340 (US)
(72) Inventor: ARNOLD, Eric Platt, Pfizer Global Res. and Dev., Groton, CT 06340 (US); CHAPPIE, Thomas Allen, Pfizer Global Res. and Dev., Groton, CT 06340 (US); HUANG, Jianhua, Pfizer Global Res. and Dev., Groton, CT 06340 (US); HUMPHREY, John Michael, Pfizer Global Res. and Dev, Groton, CT 06340 (US); NAGEL, Arthur Adam, Pfizer Global Res. and Dev., Groton, CT 06340 (US); O'NEILL, Brian Thomas, Pfizer Global Res. and Dev., Groton, CT 06340 (US); SOBOLOV-JAYNES, Susan B., Pfizer Global Res. & Dev, Groton, CT 06340 (US); VINCENT, Lawrence Albert, Pfizer Global Res. & Dev, Groton, CT 06340 (US)
(74) Representative: Duncan, Garreth Andrew
(86) International application number: PCT/IB2001/000629
(87) International publication number: WO 2001/077100

(56) References cited:
- EP-A- 0 122 494
- WO-A-00/73312
- WO-A-00/73313
- WO-A-92/06079
- WO-A-97/03066
- WO-A-99/58500
- US-A- 3 539 584
- US-A- 5 604 252
- CORDERO, FRANCA M. ET AL: "Syntheses of 3-phenyl substituted indolizidin-2-ones and a pyrrolizidin-2-one on the route to constrained potential NK1 receptor antagonists" TETRAHEDRON (1994), 50(44), 12713-26 , 1994, XP002181978

## Description

The present invention relates to certain benzoamide piperidine containing compounds and related compounds that exhibit activity as NK-1 receptor antagonists, (e.g., substance P receptor antagonists), to pharmaceutical compositions containing them, and to their use in the treatment and prevention of central nervous system disorders, inflammatory disorders, cardiovascular disorders, ophthalmic disorders, gastrointestinal disorders, disorders caused by *helicobacter pylori,* disorders of the immune system, urinary incontinence, pain, migraine, emesis, angiogenesis and other disorders.

Substance P is a naturally occurring undecapeptide belonging to the tachykinin family of peptides, the latter being so-named because of their prompt stimulatory action on smooth muscle tissue. More specially, substance P is a pharmaceutically active neuropeptide that is produced in mammals (having originally been isolated from gut) and possesses a characteristic amino acid sequence that is illustrated by D. F. Veber et al. in US Pat. 4,680,283. The wide involvement of substance P and other tachykinins in the pathophysiology of numerous diseases has been amply demonstrated in the art.

World Patent Application WO 97/03066, published January 30, 1997, and U.S. Patent Application 08/98004, filed May 9, 1996, refer to substituted benzolactam and cyclicthioamide compounds that exhibit activity as substance P receptor antagonists. Other substance P receptor antagonists containing a fused bicyclic moiety are referred to in the following: U.S. Patent Application 09/011,271, filed June 10, 1996; U.S. Provisional Patent Application 60/132,858, filed May 6, 1999; U.S. Patent Application 09/402,630, filed October 26, 1998; and World Patent Application WO 99/13663, published June 23, 1994.

### SUMMARY OF THE INVENTION

The present invention relates to compounds of the formula 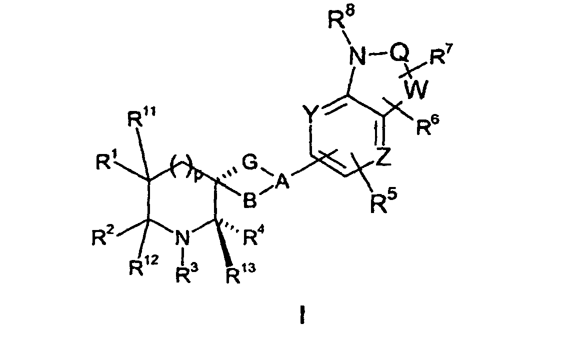
wherein Q is C=S or C=O;
A is CH, CH₂, C(C₁-C₆)alkyl, CH(C₁-C₆)alkyl, C(CF₃) or CH(CF₃), with the proviso that when B is present, A must be either CH, C(C₁-C₆)alkyl or C(CF₃);
B is absent or is methylene or ethylene;
Y is N and Z is CH, or Y is CH and Z is N;
G is NH(CH₂)_{q}, S(CH₂)_{q} or O(CH₂)_{q}, wherein q is zero or one;
with the proviso that when q is zero, G is NHS or O;
W is a one carbon linking group (i.e., methylene) or a saturated or unsaturated two or three carbon linking group, wherein each of the foregoing W groups can optionally be substituted with one substituent R⁷ or two substituents R⁷ and R⁶, or W is a one carbon linking group that forms, together with a 2, 3, 4 or 5 carbon chain, a 3, 4, 5 or 6 membered spiro ring, respectively;
or W is a saturated two carbon chain linking group that forms, together with a separate 1, 2 or 3 carbon chain, a fused 3, 4 or 5 membered ring, respectively;
or W is a saturated two carbon chain linking group, wherein one of the two carbons in the chain forms, together with a separate 2, 3, 4 or 5 carbon chain, a 3, 4, 5 or 6 membered spiro ring, respectively;
p is zero, one or two;
R³ is selected from hydrogen, COR⁹, CO₂R⁹, optionally substituted phenyl, optionally substituted heterocyclic rings, and optionally substituted (C₁-C₈)alkyl wherein one of the CH₂ groups of said (C₁-C₈) alkyl may optionally be replaced with a sulfur, oxygen or carbonyl group and wherein said (C₁-C₈)alkyl can optionally be substituted with from one to three substituents, preferably with zero substituents or one substituent, independently selected from hydroxy, oxo, phenyl-(C₁-C₃)alkoxy, phenyl, cyano, halo, optionally substituted heterocyclic rings, NR⁹COR¹⁰, NR⁹CO₂R¹⁰, CONR⁹R¹⁰, COR⁹, CO₂R⁹, NR⁹R¹⁰, and (C₁-C₆)alkoxy optionally substituted with from one to seven fluorine atoms, preferably with from zero to three fluorine atoms;
and wherein the heterocyclic rings of R³ and the heterocyclic ring substituents on the alkyl groups of R³ are selected, independently, from 3 to 7 membered saturated or unsaturated monocyclic rings containing from 1 to 4 ring heteroatoms, and 8 to 12 membered saturated or unsaturated bicyclic rings containing from 1 to 4 ring heteroatoms, wherein said heteroatoms are selected, independently, from oxygen, nitrogen and sulfur, with the proviso that there can not be two adjacent ring oxygen atoms or two adjacent ring sulfur atoms in either the monocyclic or bicyclic heterocyclic rings, and with the proviso that heterocyclic rings formed from NR⁹R¹⁰ or CONR⁹R¹⁰ must contain at least one nitrogen atom;
and wherein the heterocyclic rings of R³ and the heterocyclic ring substituents on the alkyl groups of R³ can optionally be substituted with one or more substituents, preferably with zero, one or two substituents, independently selected from oxo, hydroxy, thioxc, halo, cyano, phenyl, (CH₂)ₘNR⁹R¹⁰, NR⁹COR¹⁰, (CH₂)ₘOR⁹, wherein m is zero, one or two, and (C₁-C₆)alkyl optionally substituted with one or more substituents, preferably with from zero to two substituents, independently selected from halo, CF₃, methoxy and phenyl;
and wherein the phenyl groups of R³ and the phenyl substituents in the alkyl groups of R³ can optionally be substituted with one or more substitutents, preferably with from zero to two substituents, independently selected from the group consisting of halo, cyano, nitro, CF₃, (CH₂)ₘNR⁹R¹⁰, wherein m is zero, one or two, NR⁹COR¹⁰, NR⁹CO₂R¹⁰, CONR⁹R¹⁰, CO₂NR⁹R¹⁰, COR⁹, CO₂R⁹, (C₁-C₆)alkyl optionally substituted with from one to seven fluorine atoms, preferably with from zero to three fluorine atoms, (C₁-C₆)alkoxy optionally substituted with from one to seven fluorine atoms, preferably with from zero to three fluorine atoms, and (C₂-C₆)alkenyl optionally substituted with from one to seven fluorine atoms, preferably with from zero to three fluorine atoms;
each of R¹, R², R¹¹, R¹² and R¹³ are selected, independently, from hydrogen and (C₁-C₆)alkyl optionally substituted with one or more substituents, preferably with zero, one or two substituents, that are selected, independently, from hydroxy, oxo, (C₁-C₆)alkoxy and cyano;
or R¹ and R², together with the carbon atoms to which they are attached, or R² and R³, together with the carbon and nitrogen to which they are attached, respectively, form a 5 or 6 membered saturated heterocyclic ring containing one or two heteroatoms that are selected, independently, from nitrogen, oxygen and sulfur, with the proviso that said ring can not contain two adjacent oxygen atoms or two adjacent sulfur atoms; or R¹ and R², together with the carbons to which they are attached, form a 5 or 6 membered, saturated or unsaturated carbocyclic ring, and wherein said heterocyclic and carbocyclic rings formed by R¹ and R² or by R² and R³ can be substituted with one or more substituents, preferably with zero substituents or one substituent, independently selected from halo, oxo, NR⁹R¹⁰, (C₁-C₆)alkyl optionally substituted with from one to seven fluorine atoms, preferably with from zero to three fluorine atoms, and (C₁-C₆)alkoxy optionally substituted with from one to seven fluorine atoms, preferably with from zero to three fluorine atoms;
or R¹² and R¹³, together with the carbon atoms to which they are attached, form a 5 or 6 membered saturated heterocyclic ring containing one or two heteroatoms that are selected, independently, from nitrogen, oxygen and sulfur, with the proviso that said ring can not contain two adjacent oxygen atoms or two adjacent sulfur atoms, or R¹² and R¹³, together with the carbons to which they are attached, form a 5 or 6 membered, saturated or unsaturated carbocyclic ring, and wherein said heterocyclic and carbocyclic rings formed by R¹² and R¹³ can be substituted with one or more substituents, preferably with zero substituents or one substituent, independently selected from NR⁹R¹⁰, halo, phenyl-S-, phenyl-SO-, phenyl-SO₂-,oxo, (C₁₋C₆)alkoxy optionally substituted with from one to seven fluorine atoms, preferably with from zero to three fluorine atoms, and (C₁-C₆)alkyl optionally substituted with from one to seven fluorine atoms, preferably with from zero to three fluorine atoms:
with the proviso that no more than one of R¹ and R², R² and R³, and R¹² and R¹³ can form a ring;
R⁴ is selected from phenyl, 2-, 3- or 4-pyridyl, 2- or 3-thienyl, and pyrimidyl, wherein R⁴ can be optionally substituted with one or more substituents, preferably with zero or one substituent, selected, independently, from halo, (C₁-C₆)alkyl optionally substituted with from one to seven fluorine atoms, preferably with from zero to three fluorine atoms, (C₁-C₆)alkoxy optionally substituted with from one to seven fluorine atoms, preferably with from zero to three fluorine atoms, and (C₂-C₆) alkenyl optionally substituted with from one to seven fluorine atoms, preferably with from zero to three fluorine atoms;
R⁵ is selected from hydrogen, -SO(C₁-C₆)alkyl, -SO₂-(C₁-C₆)alkyl, -SO-aryl, -SO₂-aryl, CF₃, halo, phenyl, phenyl-(C₁-C₂)alkyl, hydroxy, aryloxy, heteroaryloxy, pyridyl, tetrazolyl, oxazolyl, thiazolyl, (C₁-C₆)alkoxy optionally substituted with from one to seven fluorine atoms, preferably with from zero to three fluorine atoms, (C₁-C₆)alkyl optionally substituted with from one to seven fluorine atoms, preferably with from zero to three fluorine atoms, and (C₁-C₆)alkyl substituted with one or more substituents, preferably with from zero to two substituents selected, independently, from hydroxy, oxo, (C₁-C₆)alkoxy, phenyl-(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
R⁶ and R⁷ are selected, independently, from -SO(C₁-C₆)alkyl, -SO₂-(C₁-C ₆)alkyl, -SO-aryl, -SO₂-aryl, CF₃, halo, phenyl, phenyl-(C₁-C₂)alkyl, hydroxy, aryloxy, heteroaryloxy, pyridyl, tetrazolyl, oxazolyl, thiazolyl, (C₁-C₆)alkoxy optionally substituted with from one to seven fluorine atoms, preferably with from zero to three fluorine atoms, (C₁-C₆)alkyl optionally substituted with from one to seven fluorine atoms, preferably with from zero to three fluorine atoms, and (C₁-C₆)alkyl substituted with one or more substituents, preferably with from zero to two substituents selected, independently, from hydroxy, oxo, (C₁-C₆)alkoxy, phenyl-(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
R⁸ is selected from hydrogen, -SO-(C₁-C₆)alkyl, -SO₂-(C₁-C₆)alkyl, -SO-aryl, -SO₂-aryl, CF₃, phenyl, phenyl-(C₁-C₂)alkyl, pyridyl, tetrazolyl, oxazolyl, thiazolyl, and (C₁-C₆)alkyl substituted with one or more substituents, preferably from zero to two substituents selected, independently, from hydroxy, oxo, (C₁-C₆)alkoxy, phenyl-(C₁-C₃)alkoxy, phenyl, cyano, chloro, bromo, iodo, NR⁹ R¹⁰, NR⁹ COR¹⁰, NR⁹CO₂R¹⁰, CONR⁹R¹⁰, COR⁹ and CO₂R⁹;
each R⁹ and each R¹⁰ is selected, independently, from hydrogen, (C₁-C₆)alkyl, hydroxy(C₁-C₆)alkyl, phenyl and CF₃;
or R⁹ and R¹⁰, when R³ is NR⁹R¹⁰ or CONR⁹R¹⁰, can form, together with the nitrogen to which they are attached, an optionally substituted heterocyclic ring that contains at least one nitrogen atom;
and wherein the phenyl groups in the definition of R⁵, R⁶, R⁷ and R⁸ and the phenyl moiety of phenyl (C₁-C₂)alkyl in the definition of R⁵, R⁶, R⁷ and R⁸ can optionally be substituted with one or more substituents, preferably with from zero to two substituents, that are selected, independently, from halo, hydroxy, (C₁-C₆)alkoxy optionally substituted with from one to seven fluorine atoms, preferably with from zero to three fluorine atoms, and (C₁-C₆)alkyl optionally substituted with from one to seven fluorine atoms, preferably with from zero to three fluorine atoms;
and the pharmaceutically acceptable salts of such compounds.

Examples of the optionally substituted heterocyclic rings of R³ and the optionally substituted heterocyclic ring substitutents on the alkyl groups of R³ are the following: pyrimidinyl, benzoxazolyl, 2,3-dihydro-3-oxobenzisosulfonazol-2-yl, morpholin-1-yl, thiomorpholin-1-yl, benzofuranyl, benzothienyl, indolyl, isoindolyl, isoquinolinyl, furyl, pyridyl, isothiazolyl, oxazolyl, triazolyl, tetrazolyl, quinolyl, thiazolyl, and thienyl, and groups of the formulas 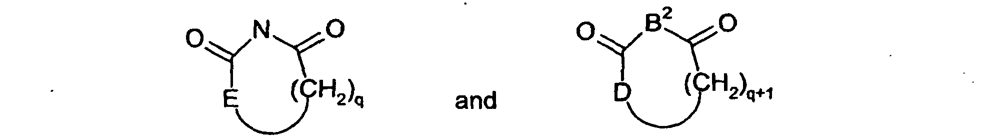 wherein B² and D are selected from carbon, oxygen and nitrogen, and at least one of B² and D is other than carbon; E is carbon or nitrogen; q is an integer from 1 to 5; any one of the carbon atoms of said (CH₂)_{q} and (CH₂)_{q+1} may be optionally substituted with (C₁-C₆)alkyl or (C₁-C₆) spiroalkyl; and either any one pair of the carbon atoms of said (CH₂)_{q} and (CH₂)_{q+1} may be bridged by a one or two carbon atom linkage, or any one pair of adjacent carbon atoms of said (CH₂)_{q} and (CH₂)_{q+1} may form, together with from one to three carbon atoms that are not members of the carbonyl containing ring, a (C₃-C₅) fused carbocyclic ring.

Compounds of formula I may contain chiral centers and therefore may exist in different enantiomeric and diastereomeric forms. This invention relates to all optical isomers and all stereoisomers of compounds of the formula I, both as racemic mixtures and as individual enantiomers and diastereoismers of such compounds, and mixtures thereof, and to all pharmaceutical compositions and methods of treatment defined above that contain or employ them, respectively.

As the compounds of formula I of this invention possess at least two asymmetric centers, they are capable of occurring in various stereoisomeric forms or configurations. Hence, the compounds can exist in separated (+)- and (-)-optically active forms, as well as mixtures thereof. The present invention includes all such forms within its scope. Individual isomers can be obtained by known methods, such a s optical resolution, optically selective reaction, or chromatographic separation in the preparation of the final product or its intermediate.

In so far as the compounds of formula I of this invention are basic compounds, they are all capable of forming a wide variety of different salts with various: inorganic and organic acids. Although such salts must be pharmaceutically acceptable for administration to animals, it is often desirable in practice to initially isolate the base compound from the reaction mixture as a pharmaceutically unacceptable salt and then simply convert to the free base compound by treatment with an alkaline reagent and thereafter convert the free base to a pharmaceutically acceptable acid addition salt. The acid addition salts of the base compounds of this invention are readily prepared by treating the base compound with a substantially equivalent amount of the chosen mineral or organic acid in an aqueous solvent or in a suitable organic solvent, such as methanol or ethanol. Upon careful evaporation of the solvent, the desired solid salt is readily obtained. The acids which are used to prepare the pharmaceutically acceptable acid addition salts of the a forementioned base compounds of this invention are those which form non-toxic acid addition salts, i.e., salts containing pharmaceutically acceptable anions, such as the hydrochloride, hydrobromide, hydroiodide, nitrate, sulfate or bisulfate, phosphate or acid phosphate, acetate, lactate, citrate or acid citrate, tartrate or bi-tartrate, succinate, maleate, fumarate, gluconate, saccharate, benzoate, methanesulfonate, ethanesulfonate, benzenesulfonate, p-toluenesulfonate and pamoate (i.e., 1,1'-methytene-bis-(2-hydroxy-3-naphthoate))salts.

Individual enantiomers of the compounds of formula I may have advantages, as compared with the racemic mixtures of these compounds, in the treatment of various disorders or conditions. For example, the compounds prepared from the 2S-phenyl-piperidin-3S-ylamino template are preferred.

The present invention also includes isotopically labelled compounds, which are identical to those recited in formula I, but for the fact that one or more atoms are replaced by an atom having an atomic mass or mass number different from the atomic mass or mass number usually found in nature. Examples of isotopes that can be incorporated into compounds of the present invention include isotopes of hydrogen, carbon, nitrogen, oxygen, phosphorous, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. Compounds of the present invention, prodrugs thereof, and pharmaceutically acceptable salts of said compounds or of said prodrugs which contain the aforementioned isotopes and/or other isotopes of other atoms are within the scope of t his invention. Certain isotopically labelled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and delectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of formula I of this invention and prodrugs thereof can generally be prepared by carrying out the procedures disclosed in the Schemes and/or in the Examples and Preparations below, by substituting a readily available isotopically labelled reagent for a non-isotopically labelled reagent.

The term "alkyl", as used herein, unless otherwise indicated, includes saturated monovalent hydrocarbon radicals having straight, branched or cyclic moieties or combinations thereof. Examples of "alkyl" groups include, but are not limited to, methyl, ethyl, propyl, isopropyl, butyl, iso- sec- and tert-butyl, pentyl, hexyl, heptyl, 3-ethylbutyl, cyclopropyl, cyclobutyl, cyclopentyl, cyclohexyl, cycloheptyl, norbomyl, and the like.

The term "alkoxy", as used herein, unless otherwise indicated, means "alkyl-O-", wherein "alkyl" is as defined above. Examples of "alkoxy" groups include, but are not limited to, methoxy, ethoxy, propoxy, butoxy and pentoxy.

The term "alkenyl", as used herein, unless otherwise indicated, includes unsaturated hydrocarbon radicals having one or more double bonds connecting two carbon atoms, wherein said hydrocarbon radical may have straight, branched or cyclic moieties or combinations thereof. Examples of "alkenyl" groups include, but are not limited to, ethenyl, propenyl, butenyl, pentenyl, and dimethylpentyl, and include E and Z forms where applicable.

The term "aryl", as used herein, unless otherwise indicated, includes an aromatic ring system with no heteroatoms, which can be either unsubstituted or substituted with one, two or three substituents selected from the group consisting of halo, (C₁-C₄)alkyl optionally substituted with from one to three fluorine atoms and (C₁-C₄)alkoxy optionally substituted with from one to three fluorine atoms.

The term "aryloxy", as used herein, unless otherwise indicated, means "aryl-O-", wherein "aryl" is as defined above.

The term "heteroaryl", as used herein, unless otherwise indicated, includes an aromatic heterocycle containing five or six ring members, of which from 1 to 4 can be heteroatoms selected, independently, from N, S and O, and which rings can be unsubstituted, monosubstituted or disubstituted with substituents selected, independently, from the group consisting of halo, (C₁-C₄)alkyl, and (C₁-C₄)alkoxy, optionally substituted with from one to three fluorine atoms;

The term "heteroaryloxy", as used herein, unless otherwise indicated, means "heteroaryl-O", wherein heteroaryl is as defined above.

The term "one or more substituents", as used herein, refers to a number of substituents that equals from one to the maximum number of substituents possible based on the number of available bonding sites.

The terms "halo" and "halogen", as used herein, unless otherwise indicated, include, fluoro, chloro, bromo and iodo.

The term "treating", as used herein, refers to reversing, alleviating, inhibiting the progress of, or preventing the disorder or condition to which such term applies, or preventing one or more symptoms of such condition or disorder. The term "treatment", as used herein, refers to the act of treating, as "treating" is defined immediately above.

The term "methylene", as used herein, means ―CH₂-.

The term "ethylene", as used herein, means ―CH₂CH₂-.

The term "propylene", as used herein, means -―CH₂CH₂CH₂-.

More specific embodiments of the invention include compounds of the formula I wherein B is absent and A is CH₂.

Other more specific embodiments of the invention include compounds of the formula I wherein Q is a carbonyl group.

Other more specific embodiments of the invention include compounds of the formula I wherein B is ethylene, A is CH and G is NHCH₂.

Other more specific embodiments of the invention include compounds of the formula I wherein B is ethylene, A is CH and G is SCH₂.

Other more specific embodiments of the invention include compounds of the formula I wherein R³ is hydrogen.

Other more specific embodiments of the invention include compounds of the formula I wherein B is ethylene, A is CH and G is NHCH₂.

Other more specific embodiments of the invention include compounds of the formula I wherein R³ is CO₂R⁹.

Other more specific embodiments of the invention include compounds of the formula I wherein B is absent, G is NH and A is CH₂.

Other more specific embodiments of the invention include compounds of the formula I wherein W is ethylene.

Other more specific embodiments of the invention include compounds of the formula I wherein R⁴ is phenyl.

Other more specific embodiments of the invention include compounds of the formula I wherein R⁴ is phenyl and R⁸ is hydrogen.

Other more specific embodiments of the invention include compounds of the formula I wherein R⁴ is phenyl and R⁸ is methyl.

Other more specific embodiments of the invention include compounds of the formula I wherein p is one.

Other more specific embodiments of the invention include compounds of the formula I wherein R² is (C₁-C₆)alkyl.

Other more specific embodiments of the invention include compounds of the formula I wherein R² is (C₁-C₆)alkyl w herein the stereochemical configuration at the chiral carbon to which R² is attached is "S".

Other more specific embodiments of the invention include compounds of the formula I wherein R⁴ is 2-, 3- or 4-pyridyl.

Other more specific embodiments of the invention include compounds of the formula I wherein R² and R¹² are selected, independently, from hydrogen, methyl, ethyl and propyl.

Other more specific embodiments of the invention include compounds of the formula I wherein both R² and R¹² are other than hydrogen.

Other more specific embodiments of the invention include compounds of the formula I wherein Y is CH and Z is nitrogen.

Other more specific embodiments of the invention include compounds of the formula I wherein Q is C=O and W is methylene optionally substituted with one or two substituents independently selected from (C₁-C₆)alkyl and CF₃.

Other more specific embodiments of the invention include compounds of the formula I wherein Q is C=O and W is ethylene optionally substituted with one or two substituents independently selected from (C₁-C₆)alkyl and CF₃.

Other more specific embodiments of the invention include compounds of the formula I wherein Y is nitrogen and Z is CH.

Other more specific embodiments of the invention include compounds of the formula I wherein Q is C=S.

Other more specific embodiments of the invention include compounds of the formula I wherein R⁸ is hydrogen.

Other more specific embodiments of the invention include compounds of the formula I wherein R⁸ is methyl.

Other more specific embodiments of the invention include compounds of the formula I wherein R³ is a heterocyclic ring.

Other more specific embodiments of the invention include compounds of the formula I wherein R³ is an alkyl group substituted with a heterocyclic ring.

Other more specific embodiments of the invention include compounds of the formula I wherein R³ is an alkyl group substituted with a heterocyclic ring selected from imidazolyl, 5-oxo-4,5-dihydro-1H-[1,2,4]triazol-3-yl, benzoxazol-2-yl, and 5-oxo-pyrrolidin-2-yl.

Other more specific embodiments of the invention include compounds of the formula I wherein R⁸ is a cycloalkyl group.

Other more specific embodiments of the invention include compounds of the formula I wherein R⁸ is a cyclopropyl group.

Preferred compounds of the invention include compounds of the formula I wherein R⁴ is optionally substituted pyridyl.

Other preferred compounds of the invention include compounds of the formula I wherein R² and R¹² are selected from (C₁-C₃)alkyl.

Examples of preferred compounds of this invention are the isomers of the following compounds that have the stereochemistry depicted in structural formula I, and their pharmaceutically acceptable salts:
6-Methoxy-1-methyl-7-[(2-phenyl-piperidin-3-ylamino)-methyl]-3,4-dihydro-1H-[1,5]naphthyridin-2-one;
6-Methoxy-1-methyl-7-[(6-methyl-2-phenyl-piperidin-3-ylamino)-methyl]-3,4-dihydro-1 H-[1,5]naphthyridin-2-one;
7-[(6-Ethyl-2-phenyl-piperidin-3-ylamino)-methyl]-6-methoxy-1-methyl-3,4-dihydro-1H-[1,5]naphthyridin-2-one; and
6-Methoxy-1-methyl-7-[(2-phenyl-6-propyl-piperidin-3-ylamino)-methyl]-3,4-dihydro-1 H-[1,5]naphthyridin-2-one.

Other examples of preferred compounds of this invention are the following compounds of the formula I and their pharmaceutically acceptable salts:
7-[((2S,3S,6S)-6-Isopropyl-2-phenyl-piperidin-3-ylamino)-methyl]-6-methoxy-1-methyl-3,4-dihydro-1H-[1,5]naphthyridin-2-one.

The compounds of formula I of this invention have useful pharmaceutical and medicinal properties. The compounds of formula I of this invention exhibit significant substance P receptor-binding activity and therefore are of value in the treatment of a wide variety of clinical conditions that are characterized by the presence of an excess of tachykinin, in particular, substance P, activity. Thus, for example, an excess of tachykinin, and in particular, substance P, activity is implicated in a variety of disorders of the central nervous system. Such disorders include, but are not limited to those enumerated in the paragraphs below.

This invention also relates to use of a compound of formula I, or a pharmaceutically acceptable salt thereof, in the manufacture of a medicament for treating a disorder or condition selected from the group consisting of mood disorders, such as depression, or more particularly, depressive disorders, for example, single episodic or recurrent major depressive disorders, dysthymic disorders, depressive neurosis and neurotic depression, melancholic depression, including anorexia, weight loss, insomnia, early morning waking and psychomotor retardation, atypical depression (or reactive depression), including increased appetite, hypersomnia, psychomotor agitation or irritability, seasonal affective disorder and pediatric depression; or bipolar disorders or manic depression, for example, bipolar I disorder, bipolar II disorder and cyclothymic disorder; conduct disorder and disruptive behavior disorder; anxiety disorders, such as panic disorder with or without agoraphobia, agoraphobia without history of panic disorder, specific phobias, for example, specific animal phobias, social anxiety, social phobia, obsessive-compulsive disorder, stress disorders, including post-traumatic stress disorder and acute stress disorder, and generalized anxiety disorders; borderline personality disorder; schizophrenia and other psychotic disorders, for example, schizophreniform disorders, schizoaffective disorders, delusional disorders, brief psychotic disorders, shared psychotic disorders, psychotic disorders with delusions or hallucinations, psychotic episodes of anxiety, anxiety associated with psychosis, psychotic mood disorders such as severe major depressive disorder; mood disorders associated with psychotic disorders such as acute mania and depression associated with bipolar disorder, mood disorders associated with schizophrenia; behavioral disturbances associated with mental retardation, autistic disorder, and conduct disorder in a mammal, including a human.

This invention also relates to use of a compound of formula I, or a pharmaceutically acceptable salt thereof, in the manufacture of a medicament for treating a disorder or condition selected from the group consisting of delerium, dementia, and amnestic and other cognitive or neurodegenerative disorders, such as Parkinson's disease (PD), Huntington's disease (HD), Alzheimer's disease, senile dementia, dementia of the Alzheimer's type, memory disorder, vascular dementia, and other dementias, for example, due to HIV disease, head trauma, Parkinson's disease, Huntington's disease, Pick's disease, Creutzfeldt-Jakob disease, or due to multiple aetiologies; movement disorders such as akinesias, dyskinesias, including familial paroxysmal dyskinesias, spasticities, Tourette's syndrome, Scott syndrome, PALSYS and akinetic-rigid syndrome; extra-pyramidal movement disorders such as medication-induced movement disorders, for example, neuroleptic-induced Parkinsonism, neuroleptic malignant syndrome, neuroleptic-induced acute dystonia, neuroleptic-induced acute akathisia, neuroleptic-induced tardive dyskinesia and medication-induced postural tremour; substance-related disorders arising from the use of alcohol, amphetamines (or amphetamine-like substances) caffeine, cannabis, cocaine, hallucinogens, inhalants and aerosol propellants, nicotine, opioids, phenylglycidine derivatives, sedatives, hypnotics, and anxiolytics, which substance-related disorders include dependence and abuse, intoxication, withdrawal, intoxication delerium and withdrawal delerium; addictive behaviors such as gambling; epilepsy; Down's syndrome; acute pain, chronic pain and migraine; demyelinbting diseases such as multiple sclerosis (MS) and amylolateral sclerosis (ALS), peripheral neuropathy, for example diabetic and chemotherapy-induced-neuropathy, and postherpetic neuralgia, trigeminal neuralgia, segmental or intercostal neuralgia and other neuralgias; and cerebral vascular disorders due to acute or chronic cerebrovascular damage such as cerebral infarction, subarachnoid haemorrhage or cerebral oedema in a mammal, including a human.

Examples of the types of pain that can be treated with the compounds of formula I of the present invention and their pharmaceutically acceptable salts include pain resulting from soft tissue and peripheral damage, such as acute trauma, pain associated with osteoarthritis and rheumatoid arthritis, musculo-skeletal pain, such as pain experienced after trauma; spinal pain, dental pain, myofascial pain syndromes, episiotomy pain, and pain resulting from bums; deep and visceral pain, such as heart pain, muscle pain, eye pain, orofacial pain, for example, odontalgia, abdominal pain, gynaecological pain, for example, dysmenorrhoea, labour pain and pain associated with endometriosis; pain associated with nerve and root damage, such as pain associated with peripheral nerve disorders, for example, nerve entrapment and brachial plexus avulsions, amputation, peripheral neuropathies, tic douloureux, atypical facial pain, nerve root damage, trigeminal neuralgia, neuropathic lower back pain, HIV related neuropathic pain, cancer related neuropathic pain, diabetic neuropathic pain, and arachnoiditis; neuropathic and non-neuropathic pain associated with carcinoma, often referred to as cancer pain; central nervous system pain, such as pain due to spinal cord or brain stem damage; lower back pain; sciatica; phantom limb pain, headache, including migraine and other vascular headaches, acute or chronic tension headache, cluster headache, temperomandibular pain and maxillary sinus pain; pain resulting from ankylosing spondylitis and gout; pain caused by increased blader contractions; post operative pain; scar pain; and chronic non-neuropathic pain such as pain associated with fibromyalgia, HIV, rheumatoid and osteoarthritis, anthralgia and myalgia, sprains, strains and trauma such as broken bones; and post surgical pain .

This invention also relates to use of a compound of formula I, or a pharmaceutically acceptable salt thereof, in the manufacture of a medicament for treating a disorder or condition selected from the group consisting of respiratory diseases, particularly those associated with excess mucus secretion, such as chronic obstructive airways disease, bronchopneumonia, chronic bronchitis, cystic fibrosis, adult respiratory distress syndrome, and bronchospasm; inflammatory diseases such as inflammatory bowel disease, psoriasis, Reiter's syndrome, Raynaud's syndrome, anthropathies, fibrositis, osteoarthritis, rheumatoid arthritis, psoriatic arthritis, asthma, pruritis and sunburn; human immunodeficiency virus (HIV) infections; allergies such as eczema and rhinitis, and other allergies; hypersensitivity disorders such as poison ivy; ophthalmic diseases such as conjunctivitis, vernal conjunctivitis, and the like; ophthalmic conditions associated with cell proliferation such as proliferative vitreoretinopathy; cutaneous diseases such as contact dermatitis, atopic dermatitis, urticaria, and other eczematoid dermatitis in a mammal, including a human.

This invention also relates to use of a compound of formula I, or a pharmaceutically acceptable salt thereof, in the manufacture of a medicament for treating a disorder or condition selected from the group consisting of neoplasms, including breast tumours, gastric carcinomas, gastric lymphomas, neuroganglioblastomas and small cell carcinomas such as small cell lung cancer in a mammal, including a human.

This invention also relates to use of a compound of formula I, or a pharmaceutically acceptable salt thereof, in the manufacture of a medicament for treating a disorder or condition selected from the group consisting of gastrointestinal (GI) disorders, including inflammatory gastrointestinal disorders such as inflammation bowel disease, disorders caused by *helicobacter pylori* and diseases of the GI tract such as gastritis, gastroduodenal ulcers, disorders associated with the neuronal control of viscera, ulcerative colitis, Crohn's disease, irritable bowel syndrome and emesis, including post operative nausea and post operative vomiting, and including acute, delayed or anticipatory emesis in a mammal, including a human.

Emesis, as referred to above, includes emesis induced by chemotherapy, radiation, toxins, viral or bacterial infections, pregnancy, vestibular disorders, for example, motion sickness, vertigo, dizziness and Meniere's disease, surgery, migraine, variations in intercranial pressure, gastro-oesophageal reflux disease, acid indigestion, over indulgence in food or drink, acid stomach, waterbrash or regurgitation, heartburn, for example, episodic, nocturnal or meal-induced heartburn, and dyspepsia.

This invention also relates to use of a compound of formula I, or a pharmaceutically acceptable salt thereof, in the manufacture of a medicament for treating a disorder or condition selected from the group consisting of stress related somatic disorders; reflex sympathetic dystrophy such as shoulder/hand syndrome; adverse immunological reactions such as rejection of transplanted tissues and disorders related to immune enhancement or suppression such as systemic lupus erythematosus; plasma extravasation resulting from cytokine chemotherapy; disorders of bladder function such as cystitis, bladder detrusor hyper-reflexia, inflammation of the urinary tract and incontinence, including urinary urge incontinence, overactive bladder, stress incontinence and mixed incontinence; fibrosing and collagen diseases such as scleroderma and eosinophilic fascioliasis; blood flow disorders caused by vasodilation and vasospastic diseases such as angina and Reynaud's disease; angiogenesis; cardiovascular disorders; eating disorders, such as anorexia nervosa and bulimia nervosa; attention deficit hyperactivity disorder; chronic fatigue syndrome; sexual dysfunctions including premature ejaculation and male erectile dysfunction; premenstrual syndrome and premenstrual dysphoric disorder; fibromyalgia; and rheumatic diseases such as fibrositis in a mammal, including a human.

The compounds of formula I are also of value in the treatment of a combination of the above conditions, in particular in the treatment of combined post-operative pain and post-operative nausea and vomiting.

The compounds of formula I are particularly useful in the treatment of emesis, including acute, delayed or anticipatory emesis, wherein the emetic agent or condition is chemotherapy, radiation, toxins, pregnancy, vestibular disorders, motion, surgery, migraine, variations in intercranial pressure, or any other emetic agent or condition. Most especially, the compounds of formula I are of use in the treatment of emesis induced by antineoplastic (cytotoxic) agents including those routinely used in cancer chemotherapy, and emesis induced by other pharmacological agents, for example, rolipram. Examples of such chemotherapeutic agents include alkylating agents, for example, nitrogen mustards, ethyleneimine compounds, alkyl sulphonates and other compounds with an alkylating action such as nitrosoureas, cisplatin and dacarbazine; antimetabolites, for example, folic acid, purine or pyrimidine antagonists; mitotic inhibitors, for example, vinca alkaloids and derivatives of podophyllotoxin; and cytotoxic antibiotics. Particular examples of chemotherapeutic agents are described, for instance, by D. J. Stewart in Nausea and Vomiting: Recent Research and Clinical Advances, Eds. J. Kucharczyk et al., CRC Press Inc., Boca Raton, Florida, USA (1991) pages 177-203, especially page 188. Commonly used chemotherapeutic agents include cisplatin, dacarbazine (DTIC). dactinomycin, mechlorethamine (nitrogen mustard), streptozocin, cyclophosphamide, carmustine (BCNU), lomustine (CCNU), doxorubicin (adriamycin), daunorubicin, procarbazine, mitomycin, cytarabine, etoposide, methotrexate, 5-fluorouracil, vinblastine, vincristine, bleomycin and chlorambucil (R. J. Gratia et al. in Cancer Treatment Reports (1984) 68(I), 163-172). The compounds of formula I are also of use in the treatment of emesis induced by radiation, including radiation therapy such as in the treatment of cancer, or radiation sickness; and in the treatment of post-operative nausea and vomiting. It will be appreciated that the compounds of formula I may be presented together with another therapeutic agent as a combined preparation for simultaneous, separate or sequential use for the relief of emesis. Such combined preparations may be, for example, in the form of a twin pack.

This invention also relates to a pharmaceutical composition comprising a therapeutically effective amount of a compound of the formula I, or a pharmaceutically acceptable salt thereof, and a pharmaceutically acceptable carrier.

The present invention also relates to compounds of the formula 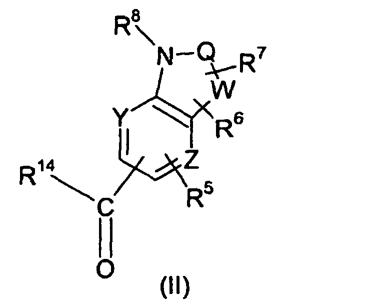
wherein Q is C=S or C=O;
Y is N and Z is CH, or Y is CH and Z is N;
W is a one carbon linking group (i.e., methylene) or a saturated or unsaturated two or three carbon linking group, wherein each of the foregoing W groups can optionally be substituted with one substituent R⁷ or two substituents R⁷ and R⁶, or W is a one carbon.linking group that forms, together with a 2, 3, 4 or 5 carbon chain, a 3, 4, 5 or 6 membered spiro ring, respectively;
or W is a saturated two carbon chain linking group that forms, together with a separate 1, 2 or 3 carbon chain, a fused 3, 4 or 5 membered ring, respectively;
or W is a saturated two carbon chain linking group, wherein one of the two carbons in the chain forms, together with a separate 2, 3, 4 or 5 carbon chain, a 3, 4, 5 or 6 membered spiro ring, respectively;
R⁵ is selected from hydrogen, -SO(C₁-C₆)alkyl, -SO₂-(C₁-C₆)alkyl, -SO-aryl, -SO₂-aryl, CF₃, halo, phenyl, phenyl-(C₁-C₂)alkyl, hydroxy, aryloxy, heteroaryloxy, pyridyl, tetrazolyl, oxazolyl, thiazolyl, (C₁-C₆)alkoxy optionally substituted with from one to seven fluorine atoms, preferably with from zero to three fluorine atoms, (C₁-C₆)alkyl optionally substituted with from one to seven fluorine atoms, preferably with from zero to three fluorine atoms, and (C₁-C₆)alkyl substituted with one or more substituents, preferably with from zero to two substituents selected, independently, from hydroxy, oxo, (C₁-C₆)alkoxy, phenyl-(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
R⁶ and R⁷ are selected, independently, from -SO(C₁-C₆)alkyl, -SO₂-(C₁-C₆)alkyl, -SO-aryl, -SO₂-aryl, CF₃, halo, phenyl, phenyl-(C₁-C₂)alkyl, hydroxy, aryloxy, heteroaryloxy, pyridyl, tetrazolyl, oxazolyl, thiazolyl, (C₁-C₆)alkoxy optionally substituted with from one to seven fluorine atoms, preferably with from zero to three fluorine atoms, (C₁-C₆)alkyl optionally substituted with from one to seven fluorine atoms, preferably with from zero to three fluorine atoms, and (C₁-C₆)alkyl substituted with one or more substituents, preferably with from zero to two substituents selected, independently, from hydroxy, oxo, (C₁-C₆)alkoxy, phenyl-(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
R⁸ is selected from hydrogen, -SO-(C₁-C₆)alkyl, -SO₂-(C₁-C₆)alkyl, -SO-aryl, -SO₂-aryl, CF₃, phenyl, phenyl-(C₁-C₂)alkyl, pyridyl, tetrazolyl, oxazolyl, thiazolyl, and (C₁-C₆)alkyl substituted with one or more substituents, preferably from zero to two substituents selected, independently, from hydroxy, oxo, (C₁-C₆)alkoxy, phenyl-(C₁-C₃)alkoxy, phenyl, cyano, chloro, bromo, iodo, NR⁹ R¹⁰, NR⁹ COR¹⁰, NR⁹CO₂R¹⁰, CONR⁹R¹⁰, COR⁹ and CO₂R⁹;
each R⁹ and each R¹⁰ is selected, independently, from hydrogen, (C₁-C₆)alkyl, hydroxy(C₁-C₆)alkyl, phenyl and CF₃;
and wherein the phenyl groups in the definition of R⁵, R⁶, R⁷ and R⁸ and the phenyl moiety of phenyl (C₁-C₂)alkyl in the definition of R⁵, R⁶, R⁷ and R⁸ can optionally be substituted with one or more substituents, preferably with from zero to two substituents, that are selected, independently, from halo, hydroxy, (C₁-C₆)alkoxy optionally substituted with from one to seven fluorine atoms, preferably with from zero to three fluorine atoms, and (C₁-C₆)alkyl optionally substituted with from one to seven fluorine atoms, preferably with from zero to three fluorine atoms; and
R¹⁴ is hydrogen, (C₁-C₆)alkyl or CF₃;
and the pharmaceutically acceptable salts of such compounds.

Compounds of the formula II are useful as intermediates in the preparation of compounds of the formula I.

Compounds of formula II may contain chiral centers and therefore may exist in different enantiomeric and diastereomeric forms: This invention relates to all optical isomers and all stereoisomers of compounds of the formula II, both as racemic mixtures and as individual enantiomers and diastereoismers of such compounds, and mixtures thereof.

Other examples of preferred compounds of this invention are the following compounds of the formula II and their pharmaceutically acceptable salts:
3-Methoxy-8-methyl-7-oxo-5,6,7,8-tetrahydro-[1,8]naphthyridine-2-carbaldehyde; and
   2-Methoxy-5-methyl-6-oxo-5,6,7,8-tetrahydro-[1,5]naphthyridine-3-carbaldehyde.

A further aspect of the present invention comprises the compounds of formula I in combination with a 5-HT₃ antagonist, such as ondansetron, granisetron or tropisetron, or other anti-emetic medicaments, for example, a dopamine antagonist such as metoclopramide or domperidone, or GABA-B receptor agonists such as baclofen. Additionally, a compound of formula I either alone or in combination with one or more other anti-emetic therapeutic agents, may be administered in combination with an anti-inflammatory corticosteroid, such as dexamethasone, betamethasone, triamcinolone, triamcinolone acetonide, flunisolide, budesonide, or others such as those disclosed in U.S. Patent Nos. 2,789,118; 2,990,401; 3,048,581; 3,126,375; 3,929,768; 3,996,359; 3,928,326; and 3,749,712. Dexamethasone (Decadron™) is particularly preferred. Furthermore, a compound of formula I may be administered in combination with a chemotherapeutic agent such as an alkylating agent, antimetabolite, mitotic inhibitor or cytotoxic antibiotic, as described above. In general, the currently available dosage forms of the known therapeutic agents for use in such combinations will be suitable. W hen tested in the ferret model of cisplatin-induced emesis described by F. D. Tattersall et al. in Eur. J. Pharmacol., (1993) 250, R5-R6, the compounds of the present invention were found to attenuate the retching and vomiting induced by cisplatin.

For the treatment of certain conditions it may be desirable to employ a compound according to the present invention in conjunction with another pharmacologically active agent. For example, for the treatment of respiratory diseases such as asthma, a compound of formula I may be used in conjunction with a bronchodilator, such as an α₂-adrenergic receptor agonist or tachykinin antagonist which acts at NK-2 receptors. The compound of formula I and the bronchodilator may be administered to a patient simultaneously, sequentially or in combination. Likewise, a compound of the present invention may be employed with a leukotriene antagonist, such as a leukotriene D4 antagonist such as a compound selected from those disclosed in European Patent Application Nos. 0 480 717 and 0 604 114 and in US Patent Nos. 4,859,692 and 5,210,324. This combination is particularly useful in the treatment of respiratory diseases such as asthma, chronic bronchitis and cough.

The present invention accordingly provides use of a compound of formula I and a bronchodilator, in the manufacture of a medicament for the treatment of a respiratory disease, such as asthma. The present invention also provides a composition comprising a compound of formula I, a bronchodilator, and a pharmaceutically acceptable carrier.

It will be appreciated that for the treatment or prevention of migraine, a compound of the present invention may be used in conjunction with other anti-migraine agents, such as ergotamines or 5-HT, agonists, especially sumatriptan, naratriptan, zolmatriptan or rizatriptan. Likewise, for the treatment of behavioural hyperalgesia, a compound of the present invention may be used in conjunction with an antagonist of N-methyl D-aspartate (NMDA), such as dizocilpine.

For the treatment or prevention of inflammatory conditions in the lower urinary tract, especially cystitis, a compound of the present invention may be used in conjunction with an antiinflammatory agent such as a bradykinin receptor antagonist. Specific antiinflammatory agents include diclofenac, ibuprofen, indomethacin, ketoprofen, naproxen, piroxicam and sulindac.

It will be appreciated that for the treatment or prevention of pain or n ociception, a compound of the present invention may be used in conjunction with other analgesics, such as acetaminophen (paracetamol), aspirin and other NSAIDs and, in particular, opioid analgesics, especially morphine. Suitable opioid analgesics of use in conjunction with a compound of the present invention include morphine, codeine, dihydrocodeine, diacetylmorphine, hydrocodone, hydromorphone, levorphanol, oxymorphone, alfentanil, buprenorphine, butorphanol, fentanyl, sufentanyl, meperidine, methadone, nalbuphine, propoxyphene and pentazocine, or a pharmaceutically acceptable salt thereof. Preferred salts of these opioid analgesics include morphine sulphate, morphine hydrochloride, morphine tartrate, codeine phosphate, codeine sulphate, dihydrocodeine bitartrate, diacetylmorphine hydrochloride, hydrocodone bitartrate, hydromorphone hydrochloride, levorphanol tartrate, oxymorphone hydrochloride, alfentanil hydrochloride, buprenorphine hydrochloride, butorphanol tartrate, fentanyl citrate, meperidine hydrochloride, methadone hydrochloride, nalbuphine hydrochloride, propoxyphene hydrochloride, propoxyphene napsylate (2-naphthalenesulphonic acid (1:1) monohydrate), and pentazocine hydrochloride.

Therefore, in a further aspect of the present invention, there is provided a pharmaceutical composition comprising a compound of the present invention and an analgesic, together with at least one pharmaceutically acceptable carrier or excipient. In a further or alternative aspect of the present invention, there is provided a product comprising a compound of the present invention and an analgesic as a combined preparation for simultaneous, separate or sequential use in the treatment or prevention of pain or nociception.

It will be appreciated that for the treatment of depression or anxiety, a compound of the present invention may be used in conjunction with other antidepressant or anti-anxiety agents. Suitable classes of anti-depressant agent include norepinephrine reuptake inhibitors, selective serotonin reuptake inhibitors (SSRIs), monoamine oxidase inhibitors (MAOIs), reversible inhibitors of monoamine oxidase (RIMAs), serotonin and noradrenaline reuptake inhibitors (SNRIs), corticotropin releasing factor (CRF) antagonists, α-adrenoreceptor antagonists and atypical antidepressants. Suitable norepinephrine reuptake inhibitors include tertiary amine tricyclics and secondary amine tricyclics. Suitable examples of tertiary amine tricyclics include amitriptyline, clomipramine, doxepin, imipramine and trimipramine, and pharmaceutically acceptable salts thereof. Suitable examples of secondary amine tricyclics indude amoxapine, desipramine, maprotiline, nortriptyline and protriptyline, and pharmaceutically acceptable salts thereof. Suitable selective serotonin reuptake inhibitors include fluoxetine, fluvoxamine, paroxetine and sertraline, and pharmaceutically acceptable salts thereof. Suitable monoamine oxidase inhibitors include isocarboxazid, phenelzine, tranylcypromine and selegiline, and pharmaceutically acceptable salts thereof. Suitable reversible inhibitors of monoamine oxidase include moclobemide, and pharmaceutically acceptable salts thereof. Suitable serotonin and noradrenaline reuptake inhibitors of use in the present invention include venlafaxine, and pharmaceutically acceptable salts thereof. Suitable CRF antagonists include those compounds described in International Patent Application Nos. WO 94/13643, WO 94/13644, WO 94/13661, WO 94/13676 and WO 94/13677. Suitable atypical anti-depressants include bupropion, lithium, nefazodone, trazodone and viloxazine, and pharmaceutically acceptable salts thereof. Suitable classes of anti-anxiety agents include benzodiazepines and 5-HT_{IA} agonists or antagonists, especially 5-HTIA partial agonists, and corticotropin releasing factor (CRF) antagonists. Suitable benzodiazepines include alprazolam, chlordiazepoxide, clonazepam, chlorazepate, diazepam, halazepam, lorazepam, oxazepam, and prazepam, and pharmaceutically acceptable salts thereof. Suitable 5-HT_{IA} receptor agonists or antagonists indude, in particular, the 5-HT_{IA} receptor partial agonists buspirone, flesinoxan, gepirone and ipsapirone, and pharmaceutically acceptable salts thereof.

Therefore, in a further aspect of the present invention, there is provided a pharmaceutical composition comprising a compound of the present invention and an anti-depressant or anti-anxiety agent, together with at least one pharmaceutically acceptable carrier or excipient. In a further or alternative aspect of the present invention, there is provided a product comprising a compound of the present invention and an anti depressant or anti-anxiety agent as a combined preparation for simultaneous, separate or sequential use for the treatment or prevention of depression and/or anxiety.

It will be appreciated that for the treatment or prevention of eating disorders, including obesity, bulimia nervosa and compulsive eating disorders, a compound of the present invention may be used in conjunction with other anorectic agents. The present invention accordingly provides the use of a compound of formula I and an anorectic agent for the manufacture of a medicament for the treatment or prevention of eating disorders. In a further aspect of the present invention, there is provided a pharmaceutical composition comprising a compound of formula I and an anorectic agent, together with at least one pharmaceutically acceptable carrier or excipient. It will be appreciated that the compound of formula I and anorectic agent may be present as a combined preparation for simultaneous, separate or sequential use for the treatment or prevention of eating disorders. Such combined preparations may be, for example, in the form of a twin pack. In a further or alternative aspect of the present invention, there is therefore provided a product comprising a compound of formula I and an anorectic agent as a combined preparation for simultaneous, separate or sequential use in the treatment or prevention of eating disorders.

In a further embodiment of the present invention there is provided the use of a compound of formula I and an anorectic agent for the manufacture of a medicament for the treatment or prevention of obesity.

In an alternative embodiment of the present invention there is provided the use of a compound of formula I and an anorectic agent for the manufacture of a medicament for the treatment or prevention of bulimia nervosa.

In a further embodiment of the present invention there is provided the use of a compound of formula I and an anorectic agent for the manufacture of a medicament for the treatment or prevention of compulsive eating disorders.

In an alternative embodiment of the present invention there is provided the use of a compound of formula I and an anorectic agent for the manufacture of a medicament for reducing the total body fat mass in an obese mammal, especially a human.

Suitable anoretic agents for use in combination with a compound of the present invention include, but are not limited to, aminorex, amphechloral, amphetamine, benzphetamine, chlorphentermine, clobenzorex, cloforex, clominorex, clortermine, cyclexedrine, dexfenfluramine, dextroamphetamine, diethylpropion, diphemethoxidine, N-ethylamphetamine, fenbutrazate, fenfluramine, fenisorex, fenproporex, fludorex, fluminorex, furfurylmethylamphetamine, levamfetamine, levophacetoperane, mazindol, mefenorex, metamfepramone, methamphetamine, norpseudoephedrine, pentorex, phendimetrazine, phenmetrazine, phentermine, phenylpropanolamine, picilorex and sibutramine, and pharmaceutically acceptable salts thereof. Particularly preferred anorectic agents include amphetamine and derivatives thereof such as amphetamine, benzphetamine, chlorphentermine, clobenzorex, cloforex, clotermine, dexfenfluramine, dextroamphetamine, diethylpropion, N-ethylamphetamine, fenfluramine, fenproporex, furfurylmethylamphetamine, levamfetamine, mefenorex, metamfepramone, methamphetamine, norpseudoephedrine, pentorex, phendimetrazine, phenmetrazine, phentermine, phenylpropanolamine, picilorex and sibutramine, and pharmaceutically acceptable salts thereof. A particularly suitable class of anorectic agent are the halogenated amphetamine derivatives, including chlorphentermine, cloforex, clortermine, dexfenfluramine, fenfluramine, picilorex and sibutramine, and pharmaceutically acceptble salts thereof. Particularly preferred halogenated amphetamine derivatives of use in combination with a compound of the present invention include fenfluramine and dexfenfluramine, and pharmaceutically acceptable salts thereof.

It will be appreciated that for the treatment or prevention of obesity, the compounds of the present invention may also be used in combination with a selective serotonin reuptake inhibitor (SSRI). The present invention accordingly provides the use of a compound of formula I and an SSRI for the manufacture of a medicament for the treatment or prevention of obesity. In a further aspect of the present invention, there is provided a pharmaceutical composition for the treatment or prevention of obesity comprising a compound of formula I and an SSRI, together with at least one pharmaceutically acceptable carrier or excipient. It will be appreciated that the compound of formula I and SSRI may be present as a combined preparation for simultaneous, separate or sequential use for the treatment or prevention of obesity. Such combined preparations may be, for example, in the form of a twin pack. In a further or alternative aspect of the present invention, there is therefore provided a product comprising a compound of formula I and an SSRI as a combined preparation for simultaneous, separate or sequential use in the treatment or prevention of obesity.

In an alternative embodiment of the present invention, there is provided the use of a compound of formula I and an SSRI for the manufacture of a medicament for reducing the total body fat mass in an obese mammal, especially a human. In a further aspect of the present invention, there is provided a pharmaceutical composition for reducing the total body fat mass in an obese mammal, especially a human, comprising a compound of formula I and an SSRI, together with at least one pharmaceutically acceptable carrier or excipient. Suitable selective serotonin reuptake inhibitors for use in combination with a compound of the present invention include fluoxetine, fluvoxamine, paroxetine and sertraline, and pharmaceutically acceptable salts thereof.

As used herein "obesity" refers to a condition whereby a mammal has a Body Mass Index (BMI), which is calculated as weight per height squared (kg/m²), of at least 25.9. Conventionally, those persons with normal weight, have a BMI of 19.9 to less than 25.9. The obesity herein may be due to any cause, whether genetic or environmental. Examples of disorders that may result in obesity or be the cause of obesity include overeating and bulimia, polycystic ovarian disease, craniopharyngioma, the Prader-Willi Syndrome, Frohlich's syndrome, Type II diabetes, GH-deficient subjects, normal variant short stature, Turner's syndrome, and other pathological conditions showing reduced metabolic activity or a decrease in resting energy expenditure as a percentage of total fat-free mass, e.g., children with acute lymphoblastic leukemia. "Treatment" (of obesity) refers to reducing the BMI of the mammal to less than a bout 2 5.9, and maintaining that weight for at least 6 months. The treatment suitably results in a reduction in food or calorie intake by the mammal. "Prevention" (of obesity) refers to preventing obesity from occurring if the treatment is administered prior to the onset of the obese condition. Moreover, if treatment is commenced in already obese subjects, such treatment is expected to prevent, or to prevent the progression of, the medical sequelae of obesity, such as, e.g., arteriosclerosis, Type II diabetes, polycycstic ovarian disease, cardiovascular diseases, osteoarthritis, dermatological disorders, hypertension, insulin resistance, hypercholesterolemia, hypertriglyceridemia, and cholelithiasis. Thus, in one aspect, this invention relates to the inhibition and/or complete suppression of lipogenesis in obese mammals, i.e., the excessive accumulation of lipids in fat cells, which is one of the major features of human and animal obesity, as well as loss of total body weight. In another aspect, the invention ameliorates the conditions that are a consequence of the disease, such as preventing or arresting the progression of polycystic ovarian disease so that the patient is no longer infertile, and increasing the insulin sensitivity and/or decreasing or eliminating the need for usage of insulin in a diabetic patient, e.g., one with adult-onset diabetes or Type II diabetes.

In a further embodiment of the present invention there is provided the use of a compound of formula I and a nicotine receptor partial agonist in the manufacture of a medicament for the treatment of chronic pain, neuropathic pain and migraine. Nicotine receptor partial agonists that can be used in such embodiments of this invention include, but are not limited to, those referred to in World Patent Applications WO 98/18798, WO 99/55680 and WO 99/35131, which were published, respectively, on May 7, 1998, November 4, 1999 and July 15, 1999, and in United States Provisional Application No. 60/083,556, which was filed on April 29, 1998.

### DETAILED DESCRIPTION OF THE INVENTION

The compounds of formula I of the present invention may be prepared as described in the following reaction schemes. Unless otherwise indicated, in the reaction schemes that follow, R¹ through R¹³, Q, Z, G, B, B², A, W, E, D and Y are defined as above.

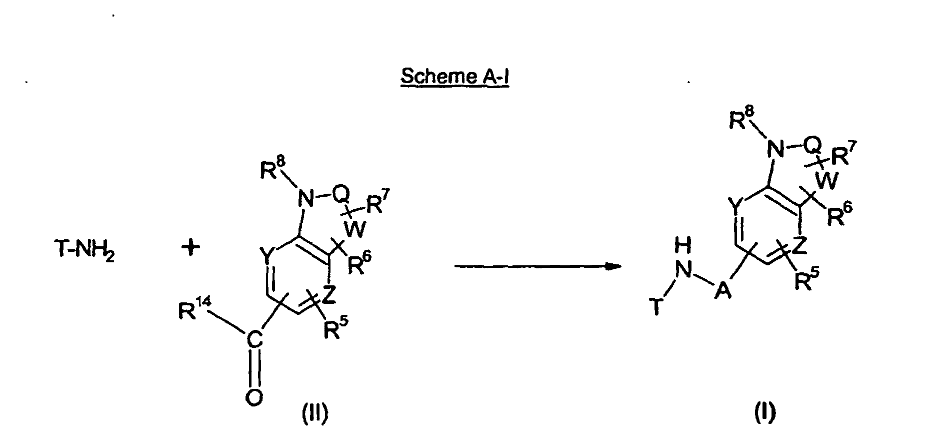

Scheme A-I illustrates a method for preparing compounds of the formula (I) wherein A is CH₂, B is absent and G is NH by reductive amination of a compound of the formula (II) with a compound of the formula T-NH₂ wherein T-NH2 is 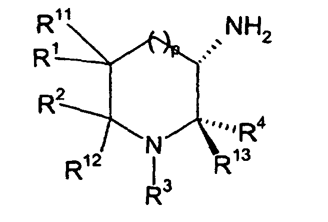 and R¹⁴ is hydrogen, (C₁-C₆)alkyl or CF₃.

The above reaction may be carried out in one vessel without isolation of the imine intermediate, or T-NH₂ and (II) may be combined in an inert solvent such as methylene chloride, dichloroethane, toluene or benzene, either at room temperature or the reflux point of the solvent, with or without removal of the byproduct water, to form the imine, which is then reduced. The reduction can be carried out by catalytic hydrogenation, or with several hydride reagents in a reaction inert solvent. The catalytic hydrogenation may be carried out in the presence of a metal catalyst such as palladium or Raney nickel. Suitable hydride reagents include borohydrides such as sodium borohydride (NaBH₄), sodium cyanoborohydride (NaBH₃CN) and sodium triacetoxyborohydride (NaB(OAc)₃H), boranes, aluminum based reagents and trialkylsilanes. Suitable solvents include polar solvents such as methanol, ethanol, methylene chloride, dichloroethane, tetrahydrofuran (THF), dioxane, toluene, benzene and ethylacetate. This reaction is typically carried out at a temperature from about -78°C to about the reflux temperature of the solvent, preferably from about 0°C to about 25°C, for a period of about 5 minutes to about 48 hours, preferably from 0.5 to 16 hours.

Alternatively, the compounds of the formula (I) of this invention may be prepared as shown in the following scheme A-II. 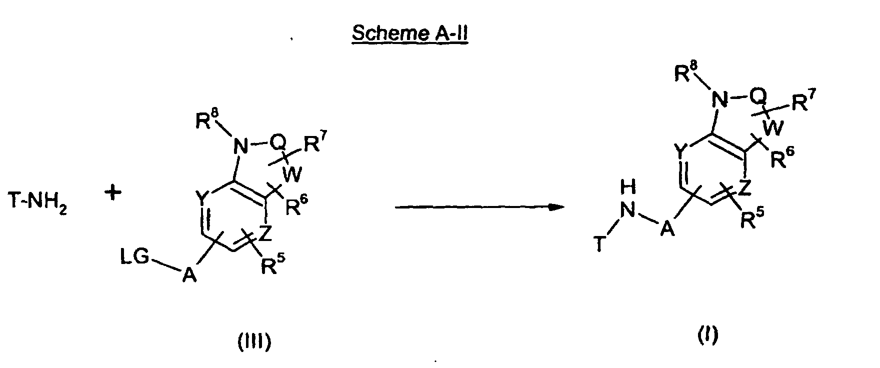 (wherein LG is a leaving group such as halo or sulfonate including tosylate, triflate or mesylate)

Referring to Scheme A-II, the compounds of the formula (I) wherein B is absent and G is NH may be prepared by a reaction of a compound of the formula (III) with a compound of the formula T-NH₂, wherein T-NH₂ is as previously defined. The compound of formula (III) is treated with T-NH₂ in the presence of a base (e.g., K₂CO₃ or Na₂CO₃) in a polar solvent (e.g., methanol, ethanol, isopropylalcohol, THF, dioxane, dimethylformamide (DMF) or dimethylsulfoxide (DMSO)). This reaction is typically carried out at a temperature from about -78°C to about the reflux temperature of the solvent, preferably from 0°C to 25°C, for a period of about 5 minutes to about 48 hours, preferably for between 0.5 and 16 hours.

The compounds of formula (III) can be prepared by reduction of an aldehyde of the formula (II), followed by conversion of a hydroxy group of the resultant compound into a leaving group, LG (e.g., halo such as chloro, bromo, iodo or fluoro, or sulfonate including tosylate or mesylate). Reduction of the aldehyde of formula (II) can be accomplished using a variety of reducing agents in a reaction inert solvent. Suitable reducing agents/solvent systems include sodium tetrahydroborate (NaBH₄) in methanol or ethanol; lithium tetrahydroborate (LiBH₄) in THF or diethyl ether; lithium tetrahydroaluminium (LiAlH₄), lithium triethoxyhydroaluminium (LiAl(OEt)₃H), lithium *tert*-butoxyhydroaluminium (LiAl(OBu*t*)₃H) or aluminium trihydride (AlH₃) in THF or diethyl ether; and *iso*-butyl aluminium hydride(*i*-BuAlH₂) or diisopropyl aluminum hydride (DIBAL-H) in dichloromethane, THF or n-hexane. This reaction is generally carried out at a temperature from about -20°C to about 25°C for a period of about 5 minutes to about 12 hours. Then, the hydroxy group of the resultant compound is converted into a leaving group LG using methods known to those skilled in the art. For example, when LG is a sulfonate such as tosylate or mesylate, the hydroxy compound is reacted with sulfonyl chloride in the presence of pyridine or triethylamine in dichloromethane. When LG is halo such as chloro or bromo, the hydroxy compound may be treated with SOX₂ (wherein X is Cl or Br) in the presence of pyridine.

Compounds of formula (II) can be prepared as illustrated in the following scheme B.

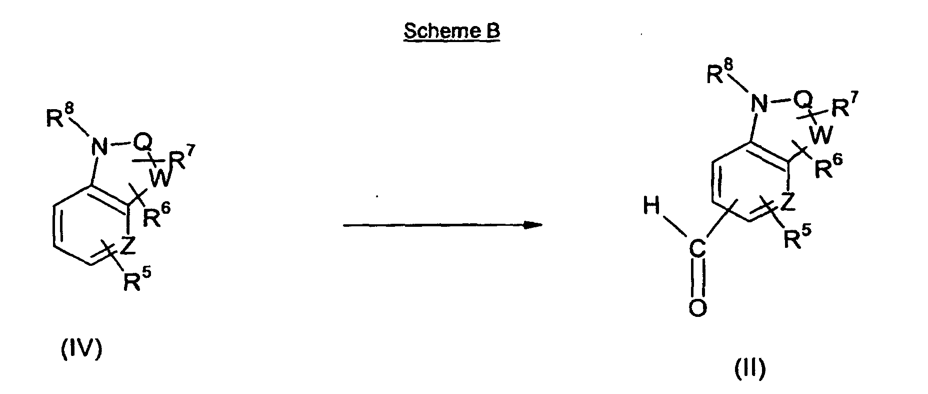

The compounds of the formula (II) may be prepared by direct or indirect formylation of a compound of the formula (IV). Any formylation methods known to those skilled in the art may be used to introduce a formyl group into a benzene ring. For example, direct formylation may be accomplished by contacting the compound of formula (IV) with a suitable formylating agent in the presence of a suitable catalyst. Suitable formylating agent/catalyst systems include dichloromethyl methyl ether/titanium (IV) chloride (Cl₂CHOCH₃/TiCl₄), dichloromethyl methyl ether/aluminum chloride (Cl₂CHOCH₃/AlCl₃), dichloromethyl methyl ether/tin (IV) chloride (Cl₂CHOCH₃/SnCl₄) dichloromethyl methyl ether/boron trifluoride etherate (Cl₂CHOCH₃/BF₃-OEt), trifluoroacetic acid (CF₃CO₂H) /hexamethylenetetramine (modified Duff's conditions) and phosphoryl trichloride (POCl₃)/DMF (Vilsmeier's conditions). Indirect formylation may be achieved by halogenating the compound of formula (IV), displacing the halogen atom introduced with a cyano group, and then subjecting the resultant cyano substituted compound to reduction. Alternatively, the halogen may be subjected to halogen metal exchange with butyl lithium. The lithium intermediate may then be treated with dimethytformamide to afford the compound of formula (II). The halogenation as used herein may be carried out according to the procedure reported in G. A. Olah et al., J. Org Chem, 58, 3194 (1993). The displacement of the halogen atom with a cyano group may be performed according to the methods reported in D. M. Tschaem et al., Smth Commun, 24, 887 (1994), and K. Takagi et al., 64 Bull Chem. Soc. Jpn. 64, 1118 (1991). The reduction can be performed in the presence of diisopropyl aluminiumhydride (DIBAL-H) in dichloromethane or Raney nickel in formic acid.

In addition, compounds of formula (II) wherein W is vinylene can be prepared by dehydrogenation of the analogous compounds of formula (II) wherein W is ethylene in a suitable solvent such as dioxane.

The starting materials of the formula (IV) either are known compounds which are commercially available, or can be prepared by known methods. For example, compounds of the formula (IV) wherein R¹ is alkyl can be prepared by *N*-alkylation of the corresponding compounds (IV) wherein R¹ is hydrogen, in the presence of a base (e.g., NaH or KH), in a suitable solvent (e.g., DMSO, DMF and THF). Compounds of the formula (IV) wherein R² or R³ is other than hydrogen, can also be prepared from the corresponding compounds of the formula (IV) wherein R² or R³, respectively, is hydrogen, using similar techniques as described above. Compounds of the formula (IV) can be also prepared by other methods as described in European Patent No. 385662 and C. Crestini et al., Synth. Commun. 24 2853 (1994) and G. W. Rewcastle et al., J. Med Chem, 37, 2033 (1994). Compounds of the formula (IV) wherein Q is S can be prepared by thionation of the corresponding compounds of formula (IV) wherein Q is O. Suitable thionation agents are Lawesson's reagent (Tetrahedron, 41, 5061 (1985)) and P₄S₁₀ (Chem. Pharm. Bull., 10, 647 (1962)).

Alternatively, compounds of the formula (I) wherein B is absent G is NH and A is CH₂ may be prepared as shown in the following Scheme A-III.

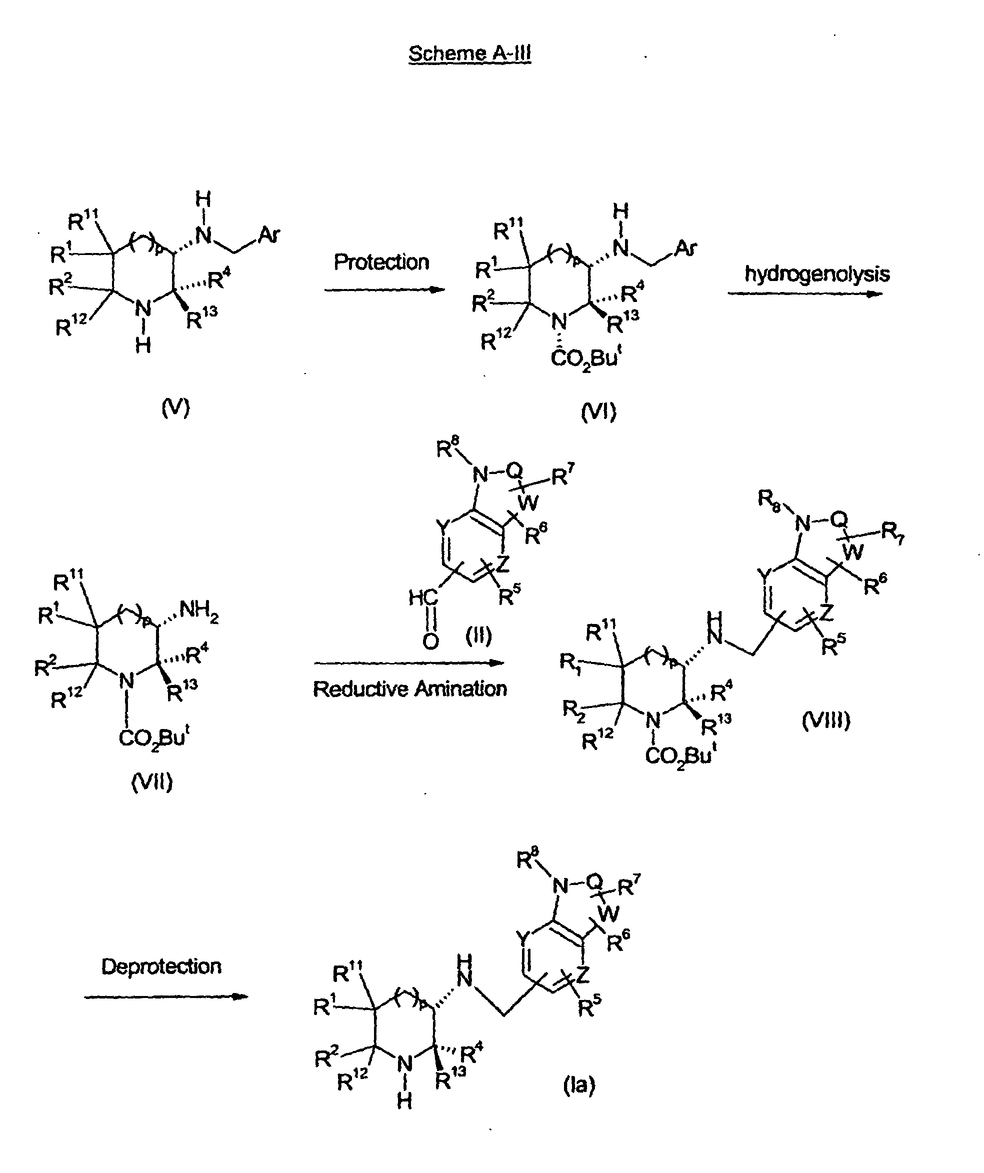

Scheme A-III illustrates the preparation of compounds of the formula (Ia) (which are compounds of the formula (I) wherein T is 2-phenylpiperidinyl).

Referring to Scheme A-III, N-protection of a compound of the formula (V) (Ar is phenyl or the like) may be carried out by treatment with (*t*-BuOCO)₂O (Boc₂O) in the presence of a base such as sodium bicarbonate (NaHCO₃) or triethylamine (Et₃N) to obtain a compound of the formula (VI). Other nitrogen protecting groups that are well known to those of skill in the art can also be used, e.g., FMOC (via reaction with FMOC-Cl), benzyl (via reaction with benzyl chloride), trifluoroacetyl (via reaction with trifluoroacetic anhydride) and benzoyl (via reaction with benzoylchloride). For a discussion of such protecting groups and methods of attaching and removing them, see Theodora W. Greene and Wuts, Peter G. M., Protective Groups In Organic Synthesis, Second Edition, John Wiley & Sons, Inc., New York, 1991. The compound of formula (VI) is subjected to hydrogenolysis to obtain a compound of the formula (VII). An alternative route for N-protection of a compound of the formula (V) may be carried out by treatment with carbobenzoxy chloride (Cbz-Cl) in the presence of a base such as sodium bicarbonate (NaHCO₃) or triethylamine (Et₃N). The hydrogenolysis may be carried out by treatment with H₂ or ammonium formate (HCO₂NH₄) in the presence of a metal catalyst such as a palladium on charcoal (e.g., 20% palladium on charcoal) in a suitable solvent. Then, the compound of formula (VII) is subjected to the reductive amination as described in Scheme A-I to form the corresponding compound of formula (VIII), which can then be converted into a compound of the formula (Ia) by treatment with an acid catalyst such as hydrochloride (HCl) in methanol, concentrated HCl in ethylacetate or CF₃CO₂H in dichloroethane.

The compounds of formula (I), and the intermediates shown in the above reaction schemes can be isolated and purified by conventional procedures, such as recrystallization or chromatographic separation.

Selected intermediates depicted as compound IV in Scheme B can be prepared by the transformations depicted in Schemes C and D.

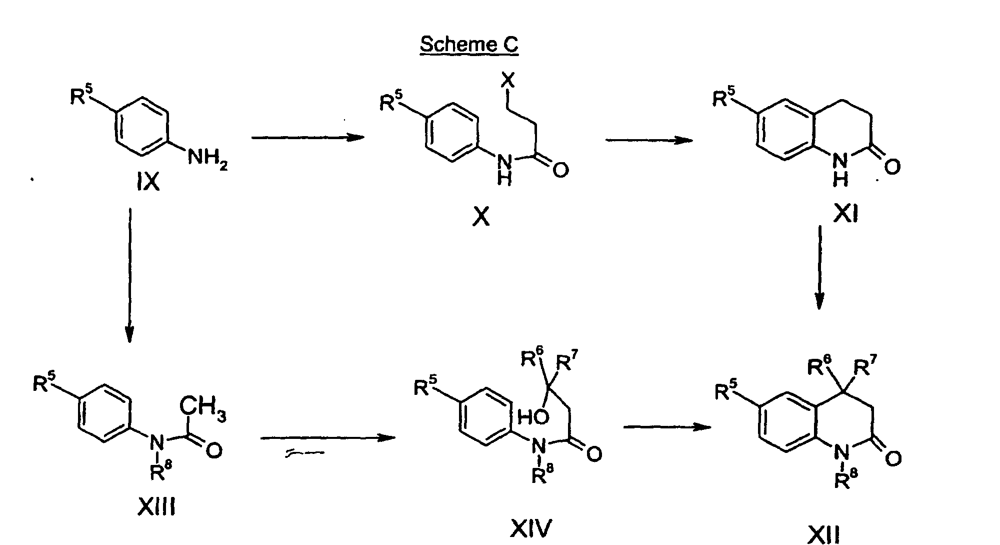

Acylation of the aniline of formula IX with an acylating agent such as acetic anhydride, acetyl bromide, acetyl chloride, acetyl sulfonates, acetyl phosphates or mixed anhydrides of acetic acid and a phenyl or alkyl chloroformate affords a compound of the formula XIII. Typically, this transformation is carried out in an inert solvent or mixtures of solvents such as methylene chloride, dichloroethane, toluene, ether, benzene, THF, dioxane, water or chloroform, or any inert solvent, in the presence of a base such as a bicarbonate or carbonate or triethyl amine, at a temperature from about -50°C to about the reflux temperature of the solvent, for a period of about 15 minutes to about 24 hours. Preferably, the compound of formula XIII is formed by the reaction of acetic anhydride in methylene chloride, using triethyl amine as the base, at about 0°C for about 2 hours.

Compounds of the formula XIII wherein R⁸ = H can be alkylated with a suitable electrophile chosen from methyl iodide, dimethyl sulfate and methyl triflate. This reaction is conducted in the presence of a base such as sodium or potassium t-butoxide or sodium or potassium hydride. A suitable inert solvent such as THF, ether or dimethoxyethane may be used. The reaction is conveniently carried out at a temperature from about -50°C to about ambient temperature. Preferred conditions involve using dimethylsulfate in THF in the presence of potassium t-butoxide, at a temperature from 0°C to ambient temperature, for 16 hours.

Alternatively, when R⁸ is hydrogen, the compound of formula XIII can be acylated with a sulfonyl or sulfinyl halide or anhydride (e.g., methanesutfonyl chloride or bromide or anhydride, trifluoromethanesulfonyl anhydride, phenyl sulfonyl chloride, bromide or anhydride, or tosyl chloride or anhydride) after treatment of such compound with a suitable base (e.g., sodium or potassium hydride, sodium or potassium carbonate, or sodium or potassium t-butoxide), in a solvent such as DMF, THF, N-methylpyrrolidinine, dichloroethane or dichloromethane. Preferably, this reaction is carried out with methane sulfonyl chloride as the reactant, using DMF as the solvent and sodium hydride as the base.

The compound of formula XIV can be prepared from the corresponding compound of formula XIII through formation of the enolate with a suitable base such as lithium diisopropyl amide, sodium, lithium or potassium hexamethyldisilazane or potassium t-butoxide, in a solvent such as THF or dimethoxyethane, at a temperature from about -100°C to about -25°C, for a period of about 15 minutes to about 5 hours. Suitable electrophiles include acetone, acetaldehyde, benzaldehyde, formaldehyde, cyclopentanone and cyclohexanone. Preferred conditions for this transformation involve using lithium diisopropyl amide in THF at about -78°C with either acetone or acetaldehyde as the electrophile, for about 2 hours.

Formation of the compound of formula XII from the corresponding compound of formula XIV can be accomplished in an acid such as sulfuric, phosphoric, triflic, hydrofluoric or polyphosphoric acid, with or without an additional inert solvent, at a temperature from about 50°C to about 150°C for a period of about 5 minutes to about 2 hours. Preferred conditions involve heating the compound of formula XIV in neat polyphosphoric acid for about 15 minutes at about 100°C.

Compounds of the formula X can be formed via acylation of the aniline of formula IX with an acylating agent such as 3-chloropropionyl chloride or chloroacetyl chloride. Typically, this transformation is carried out in an inert solvent or mixtures of solvents such as methylene chloride, dichloroethane, toluene, ether, benzene, THF, dioxane, water or chloroform, or any inert solvent, in the presence of a base such as a bicarbonate, a carbonate or triethyl amine, at a temperature between about -50°C and about the reflux temperature of the solvent, for a period of about 15 minutes to a bout 24 hours. Preferably, the compound of formula X is formed by the reaction of 3-chloropropionyl chloride in methylene chloride/water, using sodium bicarbonate as the base, at about ambient temperature for about 16 hours. The compound of formula XI is formed by reacting the corresponding compound of formula X with a Lewis acid such as aluminum chloride, stannic chloride, stannous chloride, or titanium chloride, either neat or in an inert solvent such as methylene chloride, dichloroethane, chloroform, benzene or toluene, at a temperature from about ambient temperature to about 300°C, for a period of about 5 minutes to about 2 hours. Preferably, the compound of formula XI is formed by the reaction of the corresponding compound of formula X with aluminum chloride, neat, at about 210°C for a period of about 10 minutes.

Compounds of the formula XI wherein R⁵ is hydroxy and/or R⁶ is hydrogen can be converted into the corresponding compounds wherein R⁵ or R⁸ is, respectively, an alkoxy or alkyl group by reacting them with a suitable electrophile chosen from methyl iodide, dimethyl sulfate and methyl triflate. This reaction is conducted in the presence of a base such as sodium or potassium t-butoxide or sodium or potassium hydride. A suitable inert solvent such as THF, ether or dimethoxyethane can be used. The reaction is conveniently carried out at a temperature from about -50°C to about ambient temperature. Preferred conditions involve using methyl iodide in THF, in the presence of potassium t-butoxide, at a temperature from about 0°C to about ambient temperature, for about 16 hours.

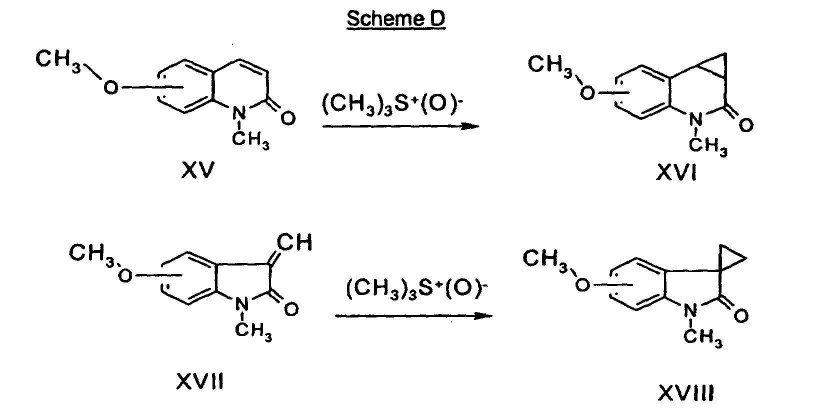

Compounds XVI and XVIII may be prepared as shown in Scheme D using conditions described by Bernard et al. CAN 67: 120195. The reaction may be conducted with trimethylsulfonium iodide, chloride or bromide, in a solvent such as DMSO, THF, DME, ether or DMF using a base such as sodium or potassium hydride, butyl or hexyl lithium at a temperature f rom a bout 0°C to about 200°C f or a bout 1 h our t o about 2 d ays. P referred conditions involve using trimethylsulfonium chloride in DMSO with sodium hydride at a temperature of about 100°C for about 1 week. More preferred conditions involve using trimethylsulfonium iodide in THF with hexyl lithium at a temperature of about 0°C to the reflux point of the solvent for about 1.5 hours. Compounds XVI and XVIII can then be transformed as above into compounds of the formula II (see Scheme B) and further transformed into compounds of the formula I (see Schemes A-I and A-III).

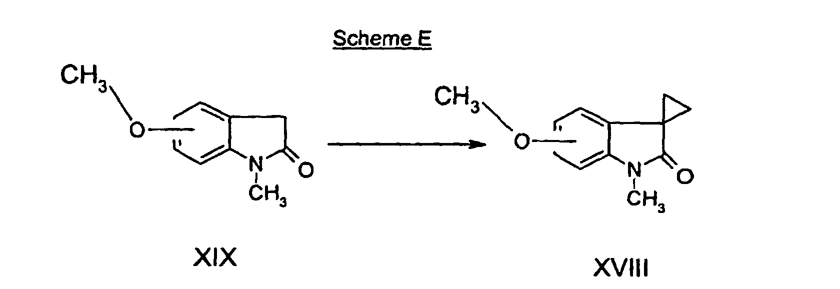

Compounds of the formula XVIII are preferably prepared directly from the corresponding compounds of formula XIX through the use of a suitable base and alkylating agent in an inert solvent. Suitable bases include but are not limited to sodium, potassium or lithium hydride, and lithium dialkylamides such as lithium diisopropyl amide, lithium hexamethyldisilazide, sodium hexamethyldisilazide or potassium hexamethyldisilazide. Suitable alkylating agents include dibromoethane, iodobromoethane, ethylene glycol dimesylate or ditosylate, iodochloroethane and the like. Suitable inert solvents include THF, ether, dimethoxyethane and DMF. The reaction can be conducted at temperatures from about 0°C to a bout the reflux temeperature of the solvent. The most preferred conditions involve reaction of the substrate XIX in DMF with sodium hydride as the base and dibromoethane as the alkylating agent at about room temperature for about 4 hours.

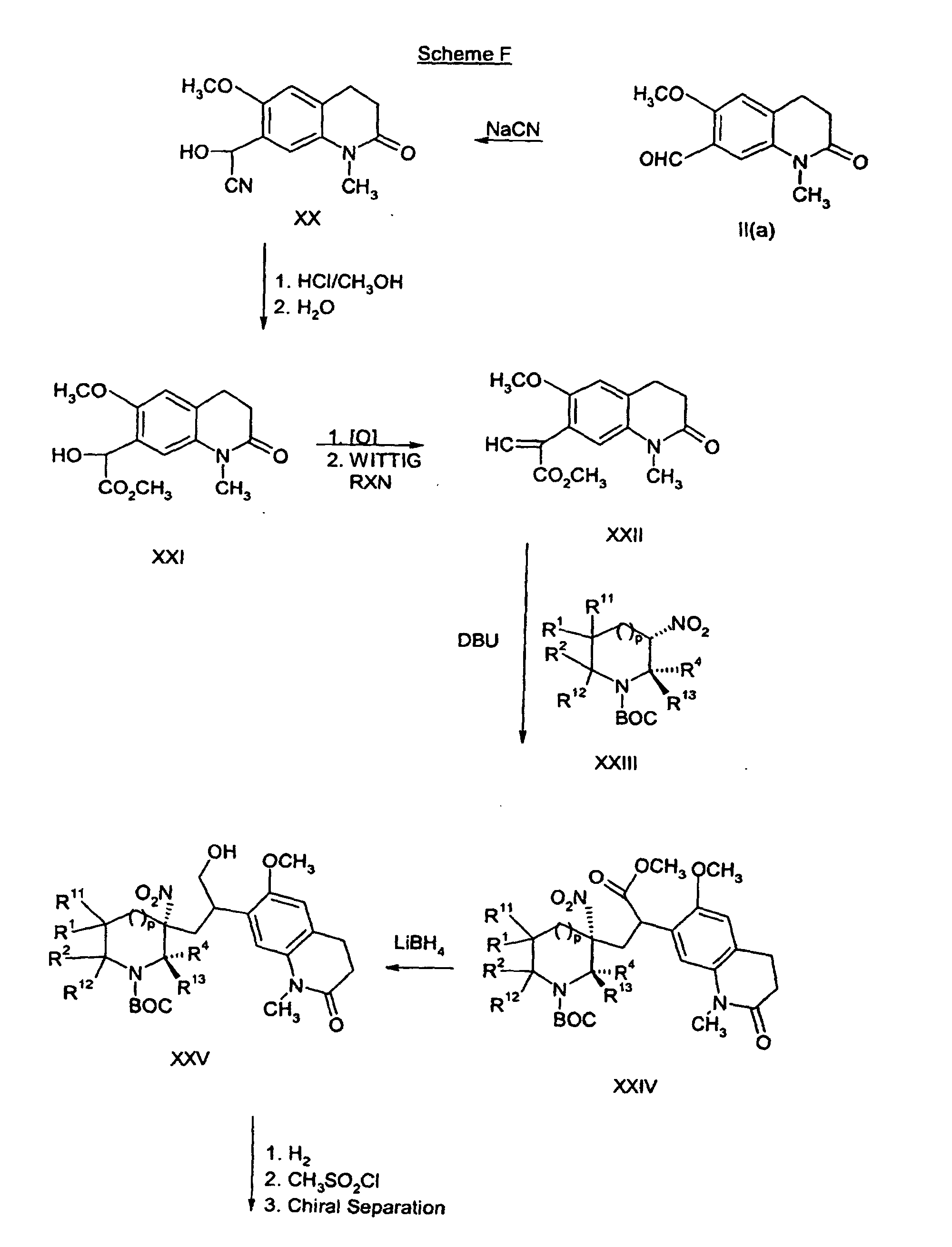

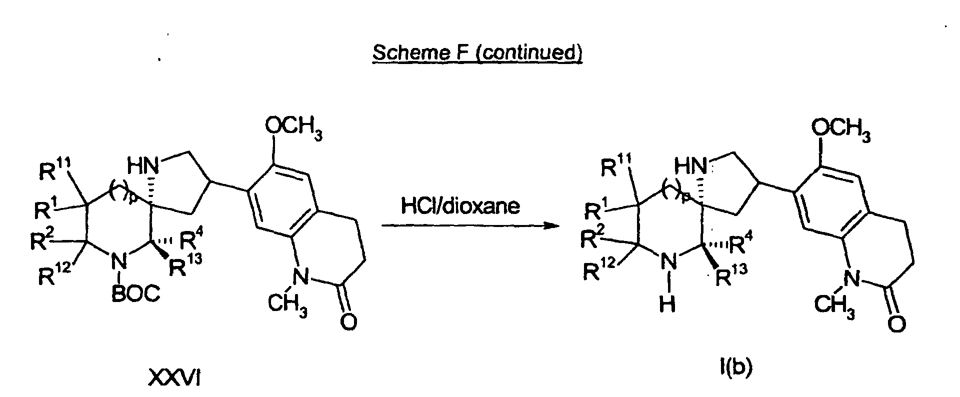

The spirocyclic compounds outlined in Scheme F can be prepared using the following general reaction sequence. The starting aldehyde of formula II(a) is dissolved in a non-polar solvent, preferably ethyl acetate, at a temperature from about -50°C to about 50°C, preferably at about room temperature, and to this solution is added aqueous sodium bisulfite, followed by aqueous sodium cyanide (NaCN). The mixture is stirred for about 1 to about 24 hours, preferably for about 4 hours, a second portion of aqueous NaCN is added, and stirring is continued for about 18 hours. The resulting cyanohydrin of formula XX is isolated and dissolved in methanol saturated with hydrogen chloride (HCl) gas. The resulting solution is then refluxed for about 3 hours. From this solution the desired hydroxy-ester of formula XX can be isolated.

The hydroxy-ester of formula XXI can be oxidized to the corresponding keto-ester using a variety of oxidizing reagents (preferably CrO₃/H₂SO₄; Jones reagent) in a non-protic solvent, preferably acetone. The keto-acid is then reacted with methyl-triphenylphosphonium bromide using standard Wittig conditions (n-Bu Li/THF; about -20°C to about room temperature) to afford the unsaturated ester derivative of formula XXII. The unsaturated ester derivative of formula XXII is then mixed with the compound of formula XXIII in a non-polar solvent, preferably tetrahydrofuran (THF), and treated with a non-aqueous base, preferably 1,8-diazabicylclo[5.4.0]undec-7-ene (DBU). The reaction is then heated to reflux for about 4 to about 36 hours, preferably for about 18 hours. The resulting ester adduct of formula XXIV can then be reduced using a variety of known reducing agents, preferably LiBH₄, in a nonprotic ether solvent, preferably ethyl ether, to afford the nitro alcohol derivative of formula XXV.

Reduction of the nitro group can be accomplished using standard methodology, preferably hydrogenation in ethanol using a Raney nickel catalyst, and cyclization to the spirocycle can be accomplished by activation of the alcohol, preferably using methanesulfonyl chloride in methylene chloride at about -20 to about 40°C, preferably about 5°C. Separation of the resulting enantiomers by chiral chromatography is a standard procedure known to those skilled in the art. Final removal of the nitrogen protecting group using standard conditions, preferably dioxane/HCl, affords the final product of formula I(b).

Compounds of the formula I wherein B is CH₂, A is CH and G is OCH₂ can be prepared using the method illustrated in Scheme G. Compounds of the formula QQ can be replaced with the appropriate compound of formula IV, as defined above for Scheme B to obtain compounds of the formula I having desired definitions of Q, W, Y, Z, R⁵, R⁶, R⁷ and R⁸.

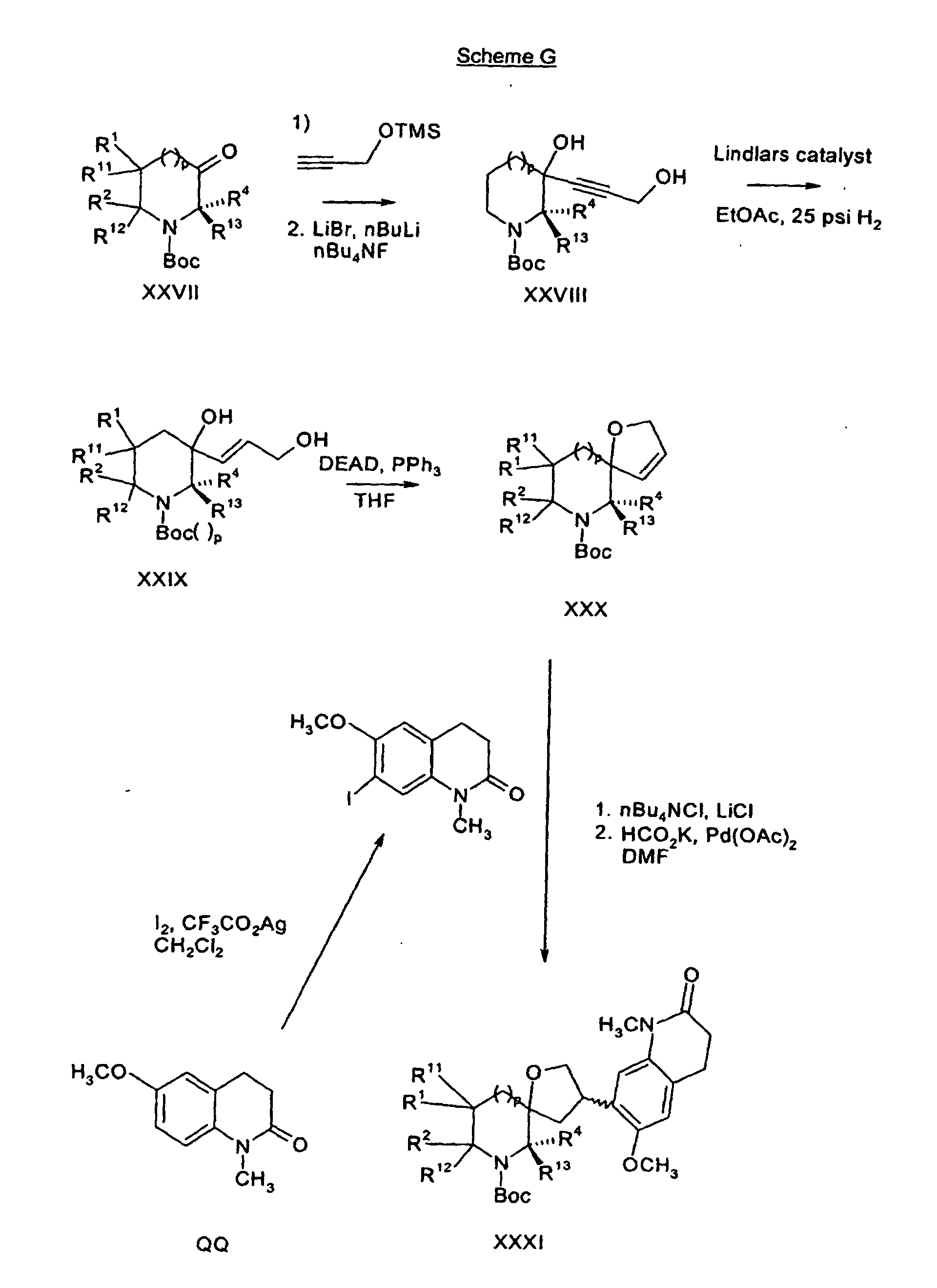

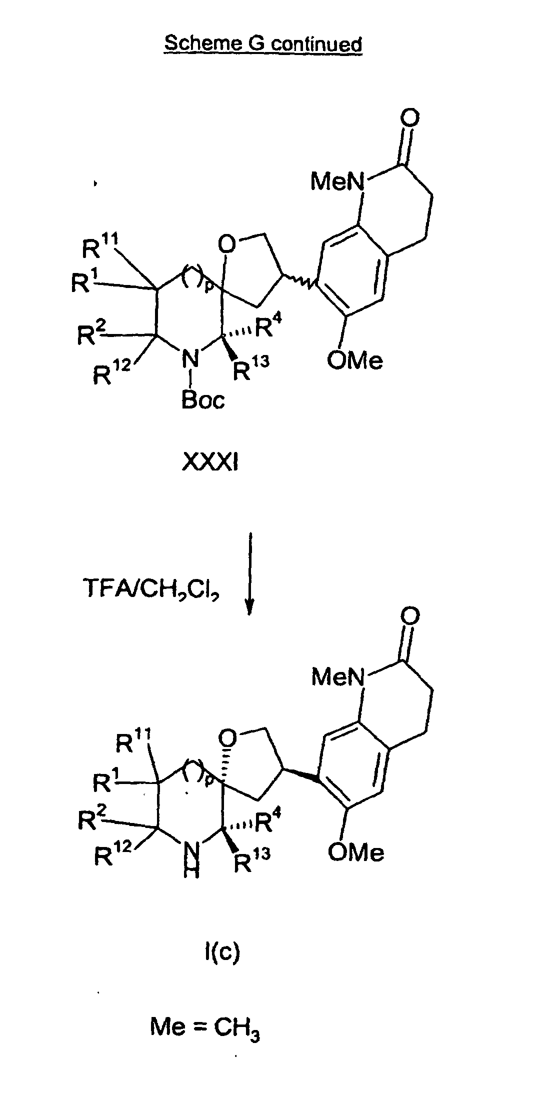

Scheme H illustrates a method of adding a Het-CH₂-C(=O)- group, as substituent R³, to an analogous compound of the formula I wherein R³ is hydrogen. (Het is defined as the heterocyclic substituents on R³ are defined above, with the proviso that Het must contain a secondary amine). Het-H is condensed with methoxy bromoacetate or ethoxy bromoacetate, in the presence of a tertiary amine hydrochloride base catalyst such as diethyl benzyl amine, pyridinium hydrochloride, or diisopropyl ethyl amine hydrochloride at a temperature of from about 0°C to about ambient temperature. The ester is hydrolyzed with a slurry of potassium carbonate in an aqueous potassium hydroxide solution at a temperature of from about 0°C to about the reflux temperature for a period of from about 16 hours to about 48 hours. The substituted acetic acid and piperidine I(d) are coupled with any standard peptide coupling agent such as Bop (benzotriazol-1-yloxytris(dimethylamino)phosphonium hexafluorophosphate), Py-Brop (bromo-tris-pyrrolidinophosphonium hexafluorophosphate) or T3P(1-propanephosphonic acid cyclic anhydride), at a temperature of from about 0°C to about ambient temperature for a period of from about 1 hour to about 24 hours, to form the final product.

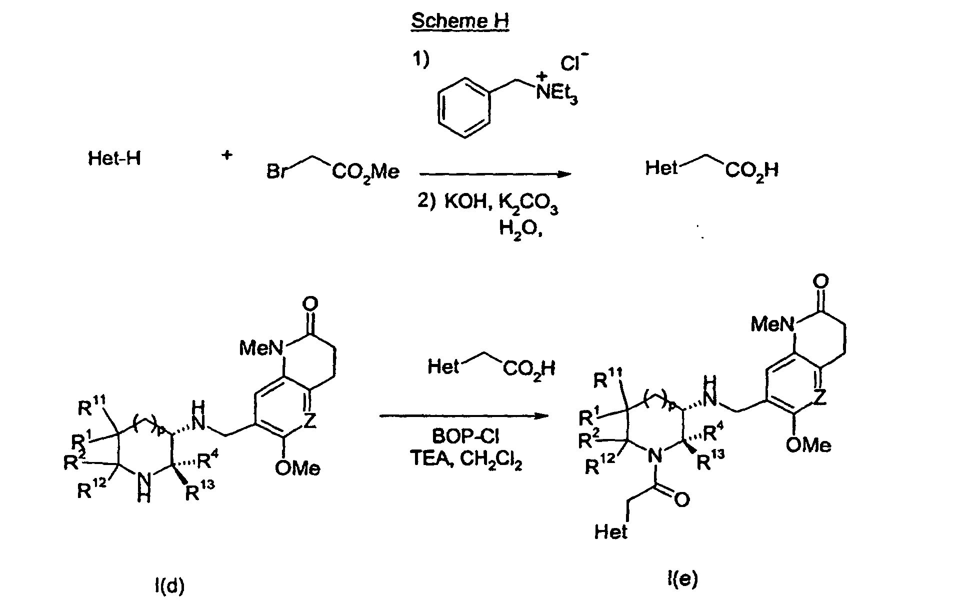

Me=CH₃, Et=CH₂CH₃

Scheme J illustrates the synthesis of certain compounds of the formula I wherein R² is alkyl.

The starting materials used in this scheme (compounds of the formula XXXII) can be prepared from commercially available alkyl vinyl ketones by reaction with nitromethane in the presence of a suitable base such as sodium or potassium alkoxide, triethylamine, diisopropylethyl amine, N-methyl morpholine, or sodium and potassium carbonate, in an organic solvent such as methanol, ethanol, propanol, isopropanol, tert-butanol, methylene chloride, chloroform, toluene, THF, DMF, DMSO, ether, oethyl acetate, at a temperature from about -78°C to about 100°C. (J. Am. Chem. Soc., 74, 3664-3668 (1952)). Preferably, the reaction is carried out using nitromethane in methanol at between about -30°C and about 0°C with sodium methoxide as the base, and methyl vinyl ketone as the electrophile. Alternatively, the reaction can be conducted under neutral aqueous conditions as described in the literature, *e*.*g*., Tetrahedron Lett. 1929-1932 (1982).

Compounds of the formula XXXIII can be prepared by using a modified Henry reaction that condenses to give substituted cyclic imines. Firstly, the ketone is protected as a ketal by use of a mineral acid such as hydrochloric acid, sulfuric acid, and nitric acid or a catalytic organic acid such as camphor sulfonic acid or toluene sulfonic acid, in an alcoholic solvent such as methanol, ethanol, propanol, or ethylene glycol, with a water scavenger such as trimethyl orthoformate, triethyl orthoformate, magnesium sulfate, or molecular sieves, at a temperature from about 0°C to about 75°C. Then, the nitroacetal is condensed *in situ* with an imine created by an amine source such as ammonium acetate, ammonium chloride, or ammonium formate, and an aldehyde such as one of variously substituted aromatic aldehydes, at a temperature from about 0°C to about 75°C. The acetal is then converted into the ketone by addition of a mineral acid such as hydrochloric acid, sulfuric acid, or nitric acid in water, at a temperature from about 0°C to about 75°C, which causes cyclization to occur, giving the compound XXXIII. Preferably. the nitroketone is dissolved in methanol at about room temperature with a catalytic amount of camphor sulfonic acid and trimethyl orthoformate. Ammonium formate is then added, followed by benzaldehyde. Aqueous sulfuric acid is added and stirred to give the cyclic imine XXXIII.

The resulting compound of formula XXXIII is dissolved in an alcoholic solvent such as methanol, ethanol, propanol, isopropanol, or t-butanol and its lithium, sodium, or potassium base and added to a solution of a mineral acid such as hydrochloric acid, sulfuric acid, or nitric acid in an alcoholic solvent with a water scavenger such as trimethyl orthoformate, triethyl orthoformate, magnesium sulfate, or molecular sieves, at a temperature from about - 30°C to about 75°C. This reaction yeilds the corresponding compound of formula XXXIV. Preferably, the nitroimine is dissolved in sodium methoxide in methanol and added to a 0°C solution of sulfuric acid and trimethyl orthoformate in methanol.

The corresponding compound of formula XXXV-a is created by the stereoselective reduction of the imine of formula XXXIV. The imine is reduced with a Lewis acid such as trimethylaluminum, triethylaluminum, and trichloroaluminum and a hydride source such as lithium aluminum hydride or diisobutyl aluminum hydride. Typically, this reaction is conducted in an organic solvent such as THF, ether, or glyme at a temperature from about 0°C to about -78°C. Preferably, the imine is reduced with triethylaluminum and lithium aluminum hydride in THF at about -78°C.

The compound of formula XXXV-a is converted into the corresponding compound of formula XXXVI-a by conversion of the acetal to the oxime and then reduction to give the amine. The acetal is stirred in water and an organic cosolvent such as THF with a mineral acid such as hydrochloric acid, sulfuric acid, or nitric acid, hydroxylamine, and a buffer such as ammonium acetate or ammonium chloride at a temperature from about 0°C to about 100°C. Reduction of the oxime is accomplished in an organic solvent such as methanol, ethanol, ethyl acetate, or acetic acid, using a catalyst such as Raney-Nickel, platinum, or palladium, under a hydrogen atmosphere of about 1 to about 50 psi at a temperature from about room temperature to about 60°C. Preferably, the compound off formula X XXV-a and hydroxylamine hydrochloride are dissolved in water, THF, and concentrated hydrochloric acid, and then ammonium acetate is added at about room temperature. The oxime is then reduced by Raney-Nickel in ethanol under 40 psi of hydrogen at about room temperature.

The final products of formula XXXVII-a are made by reductive amination of the corresponding compounds of formula XXXVI-a using an appropriate aldehyde, acid such as acetic acid, hydrochloric acid, sulfuric acid, camphor sulfonic acid, or toluene sulfonic acid, and hydride source such as sodium borohydride, sodium cyanoborohydride, or sodium triacetoxyborohydride, in an organic solvent such as methanol, ethanol, or THF at a temperature from about 0°C to about 75°C. Preferably, the compound of formula XXXVI-a, an appropriate aromatic aldehyde, acetic acid, and sodium cyanoborohydride are stirred in methanol at about room temperature to give desired product of formula XXXVII-a.

Starting materials of the type of formula XXXVII-b, which possess the opposite stereochemistry at the R² site from those of formula XXXVII-a, can be prepared through a similar reaction sequence whereby the only change is in the set of conditions used in the imine reduction. For example, substitution of NaBH₃CN/MeOH for triethyl aluminum/LiAIH₄ gives the compound XXXV-b. Many other reduction methods are available for this transformation and are commonly known to those skilled in the art. Preferred conditions include reduction with NaBH₄ in alcoholic or ethereal solvents or reduction with NaBHOAc₃ in the presence of an acid such as acetic acid in chlorinated solvents such as CH₂Cl₂ or CHCl₃ or in other inert solvents such as benzene, toluene, ether, THF or glyme. LiAl₄ in ether, THF or glyme in the absence of triethyl aluminum may also be used. These reductions are generally carried out at temperatures from about -78°C to about 100°C, preferably from about 0°C to about 60°C. The subsequent reaction sequences which yield the compound of formula XXXVII-b may take place under the same conditions as the reaction sequences which yield the compound of formula XXXVII-a.

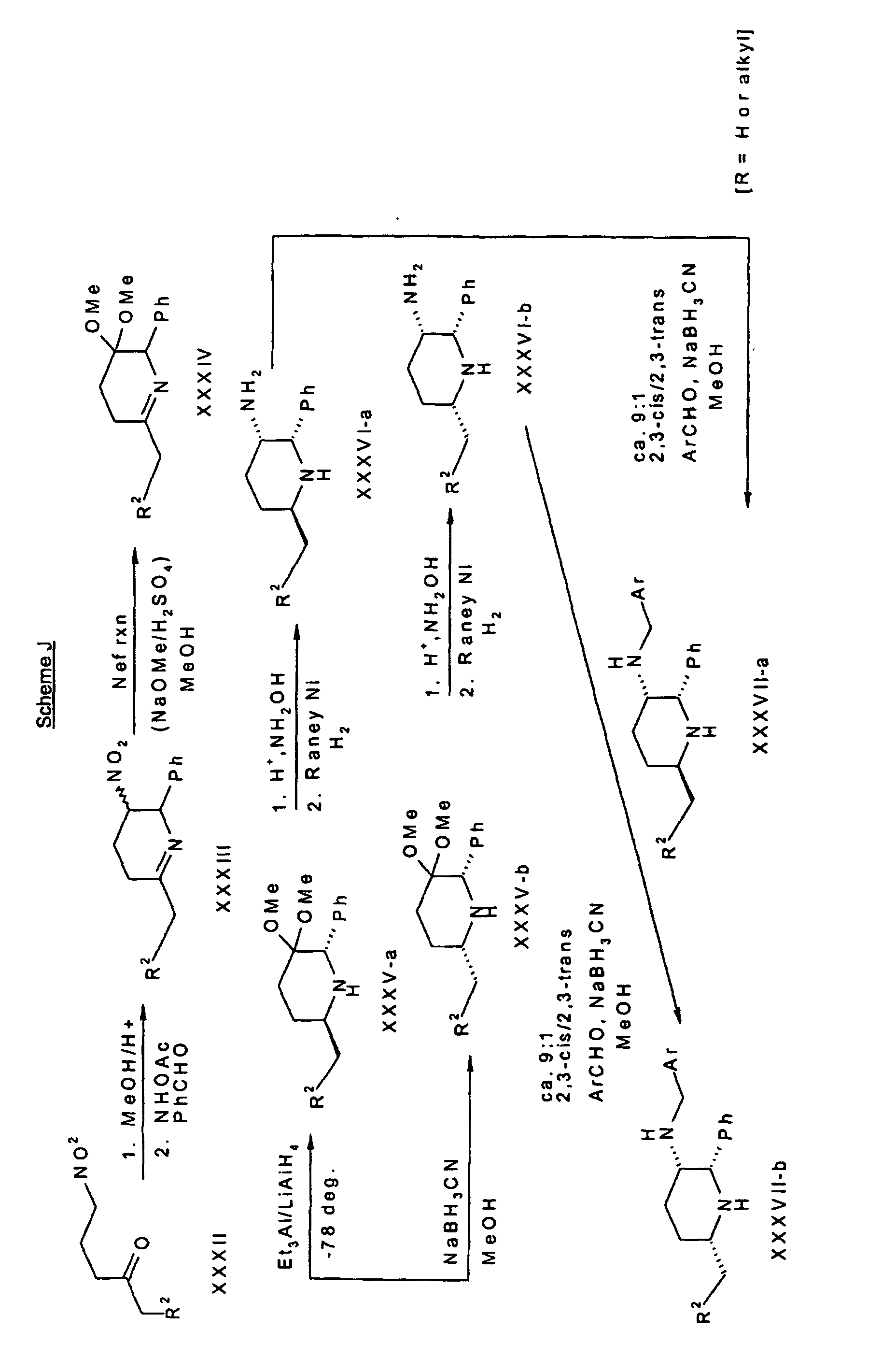

Scheme L illustrates a method of preparing compounds of the formula I wherein R³ is benzyl and wherein R¹² and R¹³, together with the carbon atoms to which they are attached, from a ring.

The starting materials for Scheme L can be prepared from 2-bromo-3-hydroxypyridine, or, in a similar fashion, from 2-iodo-3-hydroxypyridine. The reaction may be conducted using either of these starting materials and a nucleophile such as phenyl (or substituted phenyl) boronic acid or another aryl substituted boronic ester or phenylalkyl borane and/or an aryl (or substituted aryl) stannane such as phenyl tri-n-butylstannane or phenyltrimethylstannane. A palladium catalyst is usually employed. It can be either a palladium (0) source with a variety of phosphine ligands, including but not limited to palladium tetrakis[triphenylphosphine], or a non-phosphine source such as palladium dibenzylidene acetone (dba) or a palladium (II) source such as palladium acetate or palladium dichloride bis triphenylphosphine. The reaction can be carried out in a variety of solvents or mixtures of solvents such as ethyl acetate, methylene chloride, dichloroethane, toluene, benzene, ether, THF, or DMF and water, at a temperature from about ambient temperature to about the reflux point of the solvent in the presence of a suitable base such as sodium or potassium carbonate or bicarbonate. Preferably, 2-bromo-3-hydroxypyridine and phenylboronic acid, with palladium tetrakistriphenyl phosphine as the catalyst, in a mixture of benzene water and sodium carbonate, are used.

The product from this step (compound of formula XLII) is alkylated with benzyl bromide, chloride, mesylate, triflate or iodide or a substituted benzyl bromide in one of a variety of solvents such as a cetonitrile, ethanol, methanol or water, at a temperature from about ambient temperature to about the reflux point of the solvent. The product of this reaction is a pyridine salt (salt of formula XLIII). The most preferred conditions involve the use of acetonitrile at reflux with benzyl bromide to form a pyridinium bromide. The resulting salt of formula XLIII can be converted into a betaine with a suitable base, either as a separate step or in situ via the following reaction. Typically, the pyridium salt is treated with amberlite™ or another type of basic ion exchange resin or triethyl amine, diisopropylethyl amine, sodium hydroxide, sodium carbonate or bicarbonate at about ambient temperature. The most preferred conditions involve using amberlite™, a strongly basic ion exchange resin. The betaine is then reacted with a vinyl sulfone such as phenyl vinyl sulfone or substituted phenyl vinyl sulfone in an inert solvent such as ethyl acetate, methylene chloride, dichloroethane, toluene, benzene, ether, THF, or DMF, at about the reflux point of the solvent. Typically, a radical inhibitor such as hydroquinone or a related inhibitor is added to prevent radical polymerization of the vinyl sulfone. Most preferably, phenyl vinylsulfone in refluxing toluene with hydroquinone is used.

The resulting product, an enone (compound of formula XLIV), contains a carbon-carbon double bond, which can be reduced under 1-110 psi of hydrogen pressure using a suitable catalyst such as palladium on carbon, palladium hydroxide, platinum oxide or platinum on carbon. This reaction may be carried out in a solvent such as methanol, ethanol or water, at a temperature from about ambient temperature to about the reflux point of the solvent. Alternatively, the reaction can be conducted using ammonium formate or another formate salt as the source of hydrogen. Preferably, ammonium formate in methanol at reflux is used with Pd(OH)₂/C. The resulting ketone is converted into an oxime ether or an oxime using hydroxylamine methyl ether or, alternatively, ethyl or benzyl ether as well as hydroxyl amine itself. The reaction is carried out in a solvent or mixture of solvents selected from ethyl acetate, methylene chloride, dichloroethane, toluene, benzene, ether, THF, DMF, methanol, ethanol and water, or mixtures thereof, at a temperature from about ambient temperature to about the reflux point of the solvent, with a suitable buffer such as sodium or potassium acetate. The most preferred conditions involve using a refluxing mixture of methylene chloride, methanol and water with hydroxylamine methyl ether and sodium acetate. The resulting oxime methyl ether (compound of formula XLV) can be selectively reduced with a suitable reducing agent such as sodium cyanoborohydride or sodium triacetoxy borohydride or triethyl silane in a solvent under acidic conditions. Suitable solvents include acetic, formic and trifluoroacetic acid. This reaction is usually run at about ambient temperature. Most preferably, sodium cyanoborohydride in acetic acid is used to afford a compound of formula I(g).

Reduction of the hydroxylamine ether can be combined with removal of the phenylsulfone functionality. The reducing agent is typically sodium, lithium or potassium metal, or sodium amalgam, aluminum amalgam or samarium diiodide. The reaction can be conducted in a solvent or a mixture of solvents selected from liquid ammonia, ethanol, methanol, toluene, THF, t-butanol and similar solvents. The reaction is usually conducted at a temperature from about -33°C to about ambient temperature or about the reflux point of the solvent. The most preferred conditions involve using sodium metal in liquid ammonia/THF at reflux to afford a compound of formula XLVI. Removal of the N-benzyl protecting group, can be accomplished under 1-110 psi of hydrogen pressure using a suitable catalyst such as palladium on carbon or palladium hydroxide. This reaction can be carried out in a solvent such as methanol, ethanol or water, at a temperature from about ambient temperature to about the reflux point of the solvent. Alternatively, this reaction can be conducted using ammonium formate or another formate salt as the source of hydrogen. Preferably, ammonium formate in methanol is used, with palladium hydroxide on carbon as the catalyst and the reaction is conducted at the reflux point of the solvent. In the final step, the aldehyde of formula XLVII is coupled to the diazabicyclo[3.2.1]octane of formula XLVI (wherein the N-benzyl protecting group, Bn, has been removed) under the conditions described above for schemes A-I-A-III.

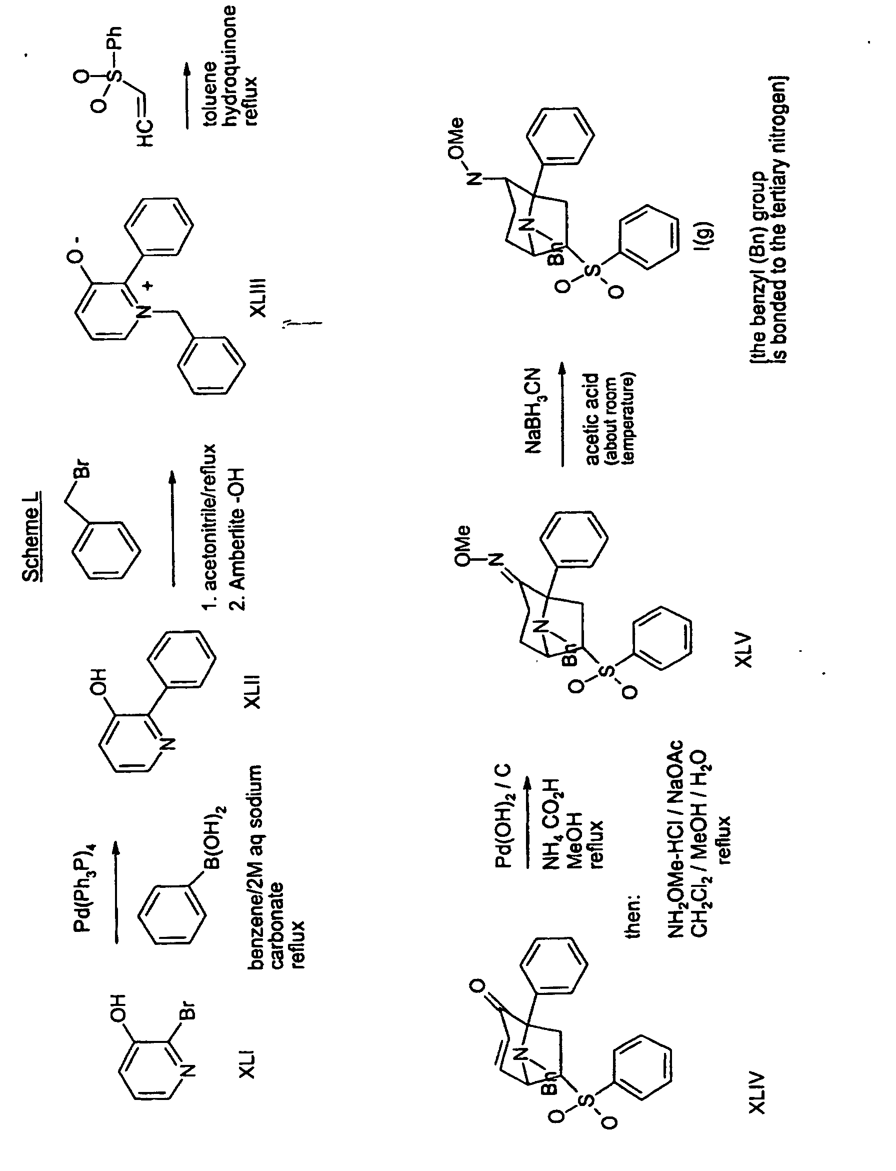

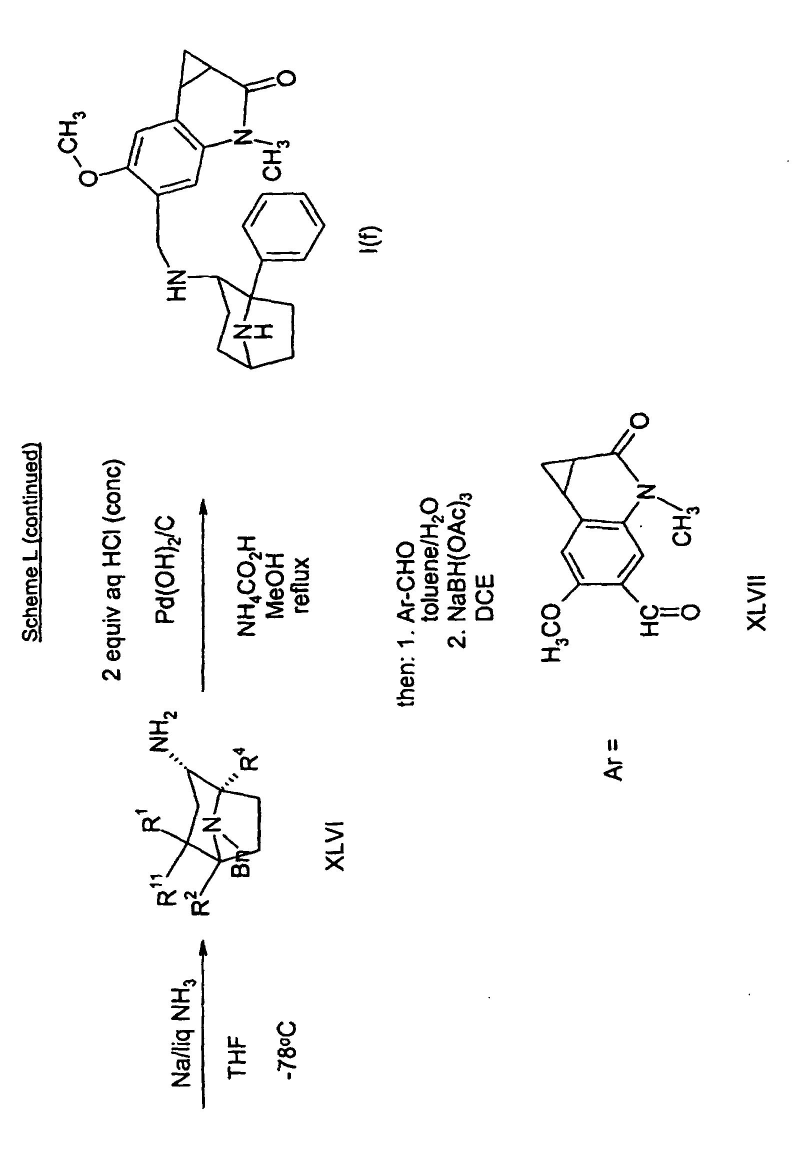

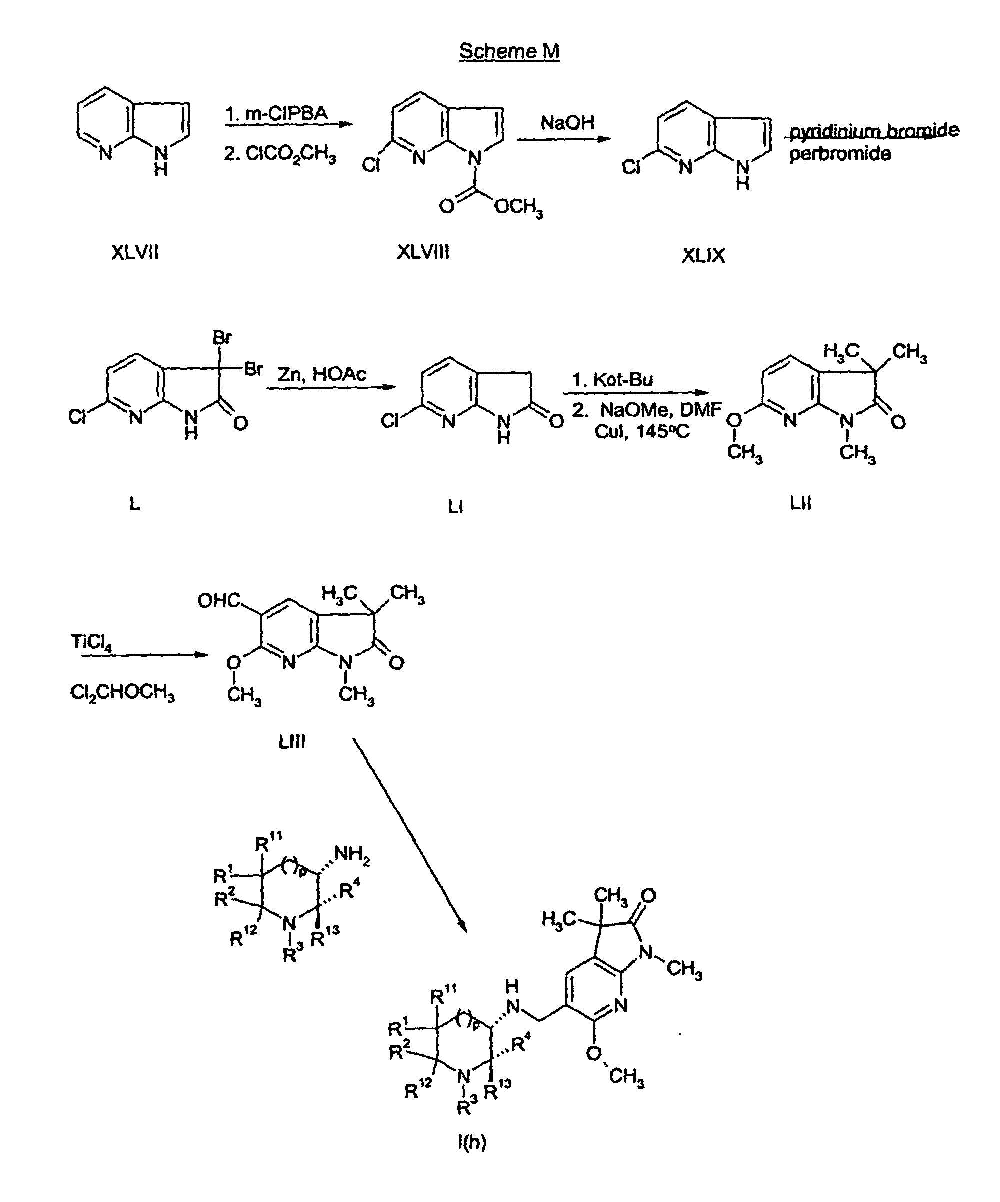

Referring to Scheme M, 7-azaindole (formula XLVII) (Aldrich) is treated with 1.4 equivalents of m-chloroperbenzoic acid in dichloroethane at about ambient temperature for a period of about 4 hours. The product from the foregoing reaction is isolated and dissolved in THF and treated with 1 equivalent of hexamethyldisilane and 2.5 equivalents, of methylchloroformate at about ambient temperature for about 16 hours. This reaction yields the chlorinated compound having formula XLVIII. Sodium hydroxide hydrolysis in methanol/water at about ambient temperature, for about 2 hours yields 6-chloro-7-azaindole (formula XLIX). Sequential treatment of this intermediate with pyridinium bromide perbromide in t-butanol (t-BuOH) at about ambient temperature for about 16 hours, followed by treatment with Zn in acetic acid (HOAc) at about ambient temperature for about 20 minutes affords 6-chloro-7-azaoxindol-2-one (compound LI). The compound of formula LI is then permethytated using methyl iodide/potassium t-butoxide a t about ambient temperature for about 16 h ours and then converted to the 6-methoxy derivative of formula LII using sodium methoxide (NaOMe) in DMF and a copper (I) iodide (Cul) catylist at about 145°C, for about 6 hours. The resulting aldehyde of formula LIII can then be formed by reacting the compound of formula LII with titanium tetrachloride (TiCl₄)/α,α-dichloromethyl methyl ether at about ambient temperture for about 16 hours. Standard reductive amination conditions to couple the compound of formula LIII to the appropriate compound of formula T-NH₂ will yield the desired final product of formula I(h).

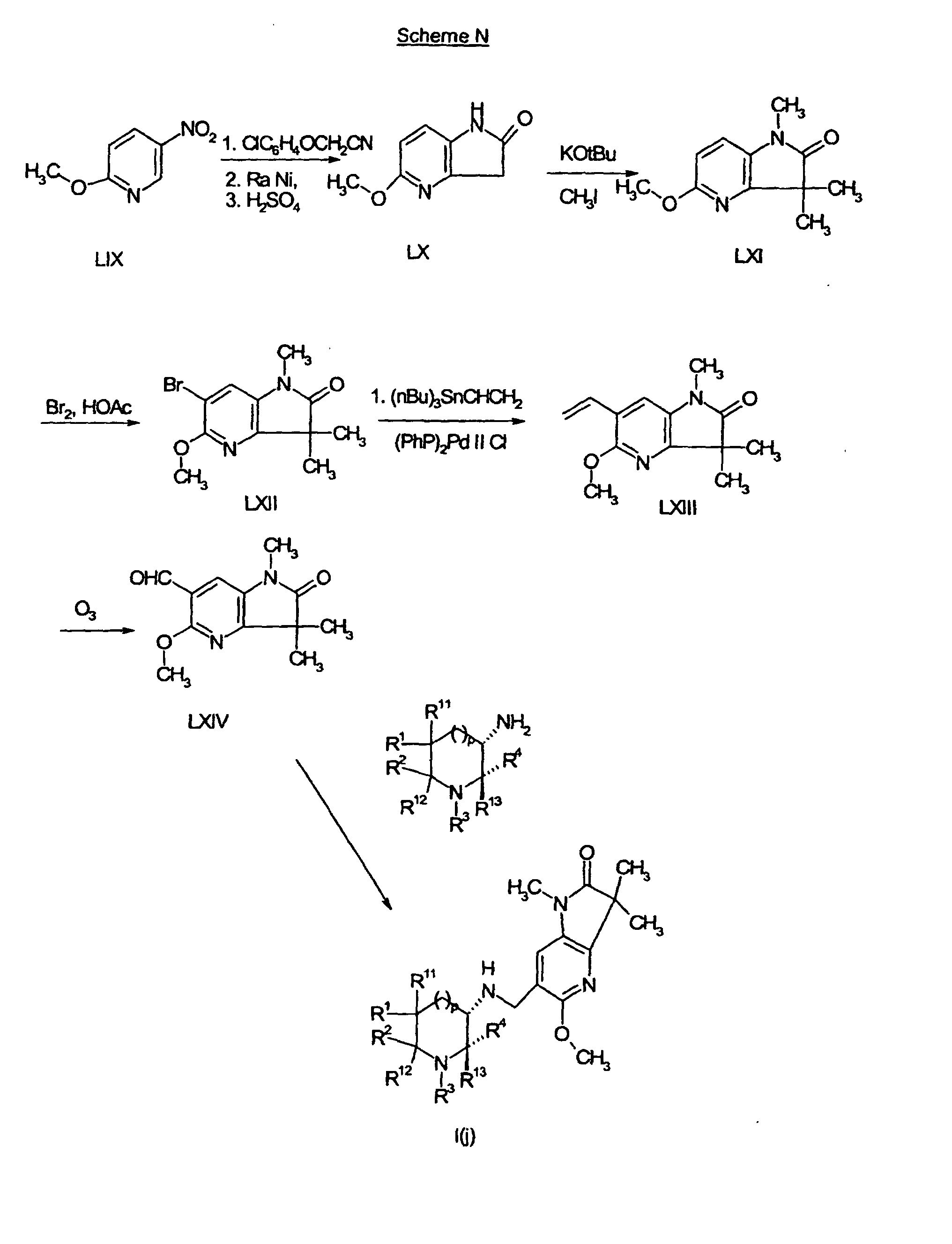

Referring to Scheme N, the 4-aza-5-methoxy-oxindole intermediate (LX) can be prepared according to the literature (See Robinson et al., *J. Het. Chem.,* 1996, *33,* 287-293). It is permethylated using methyl iodide/potassium t-butoxide at about ambient temperature for about 16 hours, and then brominated (using liquid bromine (Br₂) and acetic acid) at about 60°C for about 1 hour). Vinylation of the compound of formula LXII is accomplished using tri-n-butyl-vinyl tin, hexamethylphosphoramide (HMPA), and a (Ph₃P)₂PdCl₂ catalyst at about 65°C for about 14 hours. Ozonolysis of the vinyl group in methylene chloride for about 5 minutes afforded the aldehyde derivative of formula LXIV. The desired final product of formula I(j) is formed using standard reductive amination conditions with the appropriate compound of formula T-NH₂, as described above for Schemes A-I-A-III.

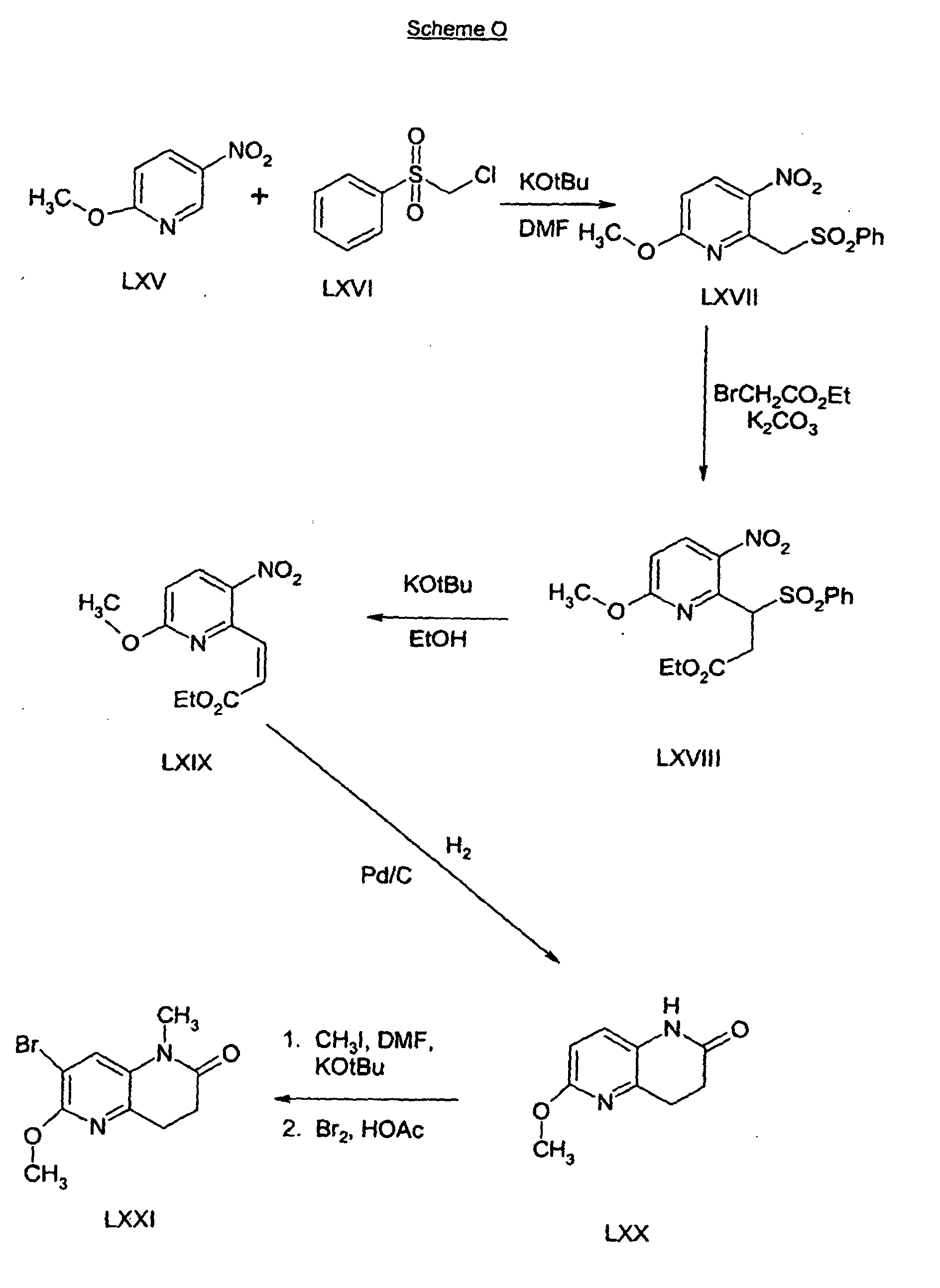

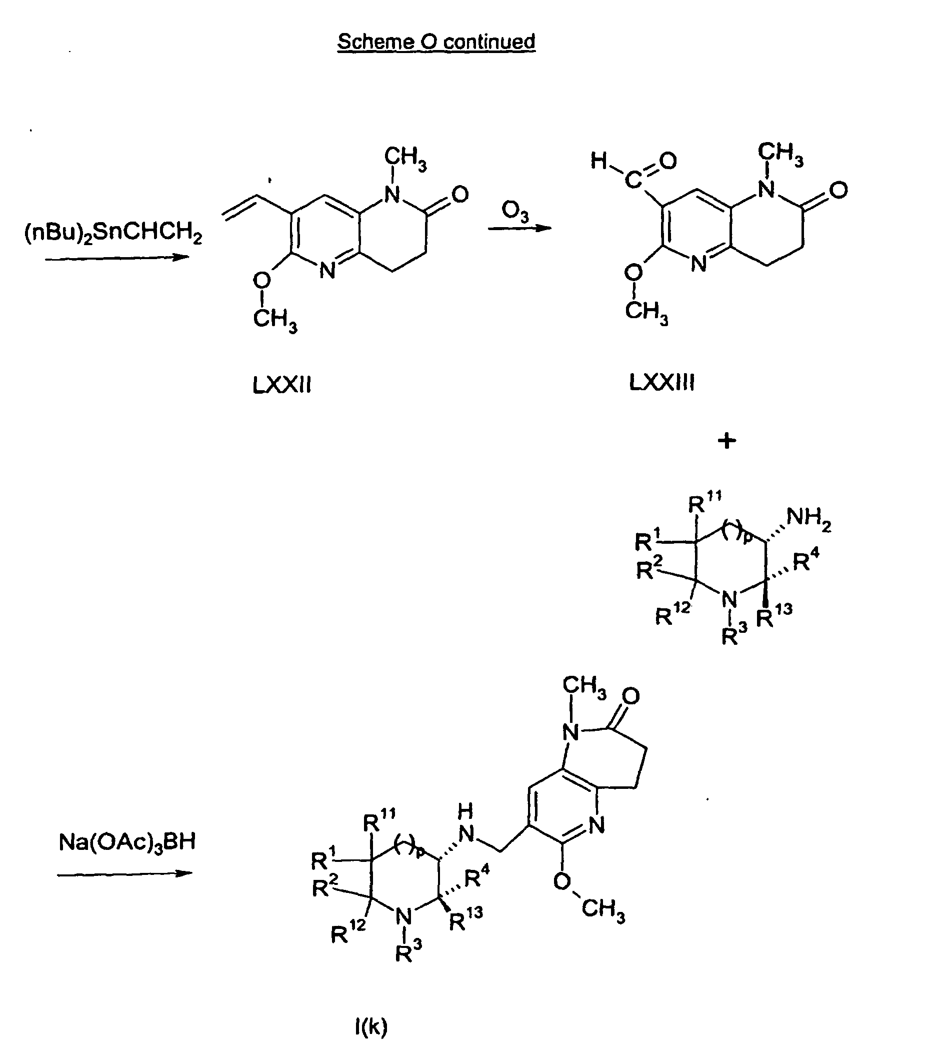

Referring to Scheme O, 2-methoxy-5-nitropyridine (formula LXV) is converted into the phenyl sulfone derivative having formula LXVII by reacting it with potassium t-butoxide in DMF at about ambient temperature for about 16 hours. The latter compound is then alkylated with ethyl bromoacetate in DMF, in the presence of potassium carbonate (K₂CO₃) at about 45°C for about 2 hours. Hydrogenation (using hydrogen gas at about 40 psi, a palladium on carbon catalyst and an ethanol solvent) for about 18 hours affords the ring cyclized product of formula LXX. The compound of formula LXX is then reacted with methyl iodide at about 0°C in DMF for about from 1 to about 4 hours, in the presence of an organic base, preferably potassium t-butoxide. After the reactants are mixed at about 0°C, the reaction mixture is allowed to warm to ambient temperature. Bromination of the resulting intermediate using liquid bromine and acetic acid at about 60°C, for about 1 hour, yields the compound of formula LXXI. Vinylation to produce the compound of formula LXXII can then be accomplished using tri-n-butyl-vinyl tin, HMPA, and a (Ph₃P)₂PdCl₂ catalyst at about 65°C for about 14 hours. Ozonolysis of the vinyl group in methylene chloride for about 5 minutes affords the aldehyde derivative of formula LXXIII. The desired final product of formula I(k) is formed using standard reductive amination conditions with the appropriate compound of formula T-NH₂. as described above for Schemes A-I - A-III.

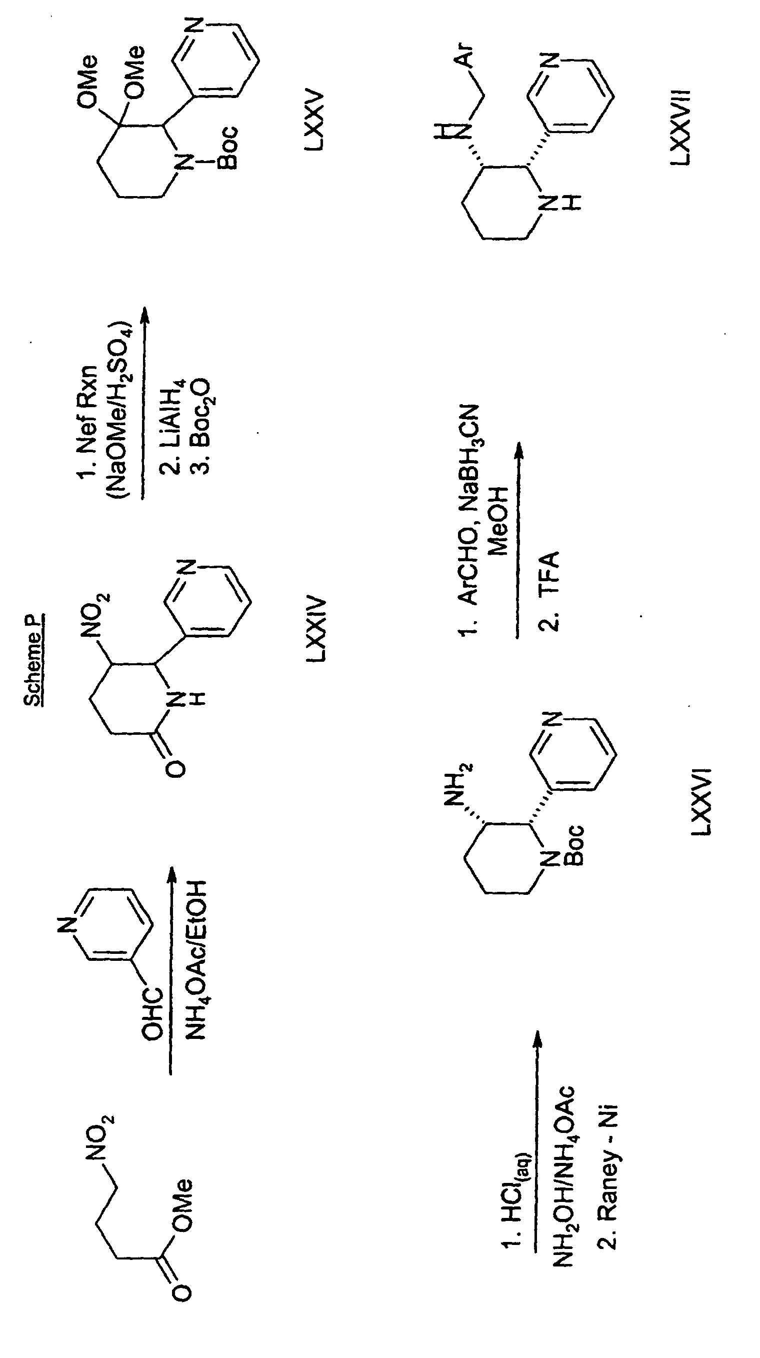

Referring to Scheme P, 4-nitrobutyric acid methyl ester is converted into the nitropyridyl derivative of formula LXXIV by reaction with pyridine-3-carboxaldehyde in the presence of an ammonia source such as ammonium acetate, ammonium chloride or ammonium formate. The reaction can be conducted in alcoholic solvents such as methanol or ethanol at temperatures ranging from about -78°C to about the reflux temperature, with reflux being the preferred temperature. The nitro-group is converted into the dimethyl acetal by subjection to Nef reaction conditions as described earlier. Reduction of the amide group yields a piperidine compound. This reduction can be brought about by a number of conditions known to those skilled in the art. Preferred conditions include LiAIH₄ or borane-dimethylsulfide reduction in inert solvents such as ether or THF. This is followed by N-Boc protection via standard protocols as described earlier which yields the compound of formula LXXV. Conversion of the acetal of formula LXXV into the oxime is carried out under acidic conditions catalyzed by mineral acids such as HCl, HBr, HNO₃ or H₂SO₄, or organic acids such as carboxylic acids or sulfonic acids, at temperatures ranging from about -78°C to about 100°C. Suitable solvents include water or mixtures of water with a variety of organic solvents such as THF, ether, toluene, DMF, ethanol and methanol. Preferred conditions are HCl-catalyzed hydrolysis at about room temperature in water/THF or water/alcohol. The initially formed ketone is converted into the oxime by treatment with hydroxylamine hydrochloride and ammonium acetate at about room temperature. The oxime is reduced by catalytic hydrogenation as described earlier. Preferred conditions for the reduction of the oxime are treatment with Raney nickel in methanol or ethanol under 52 psi of hydrogen. The resultant amine of formula LXXVI is converted into the benzylamine derivatives via reductive amination under standard conditions as described previously. The N-Boc group is cleaved under standard conditions as described earlier to yield the compound of formula LXXVII.

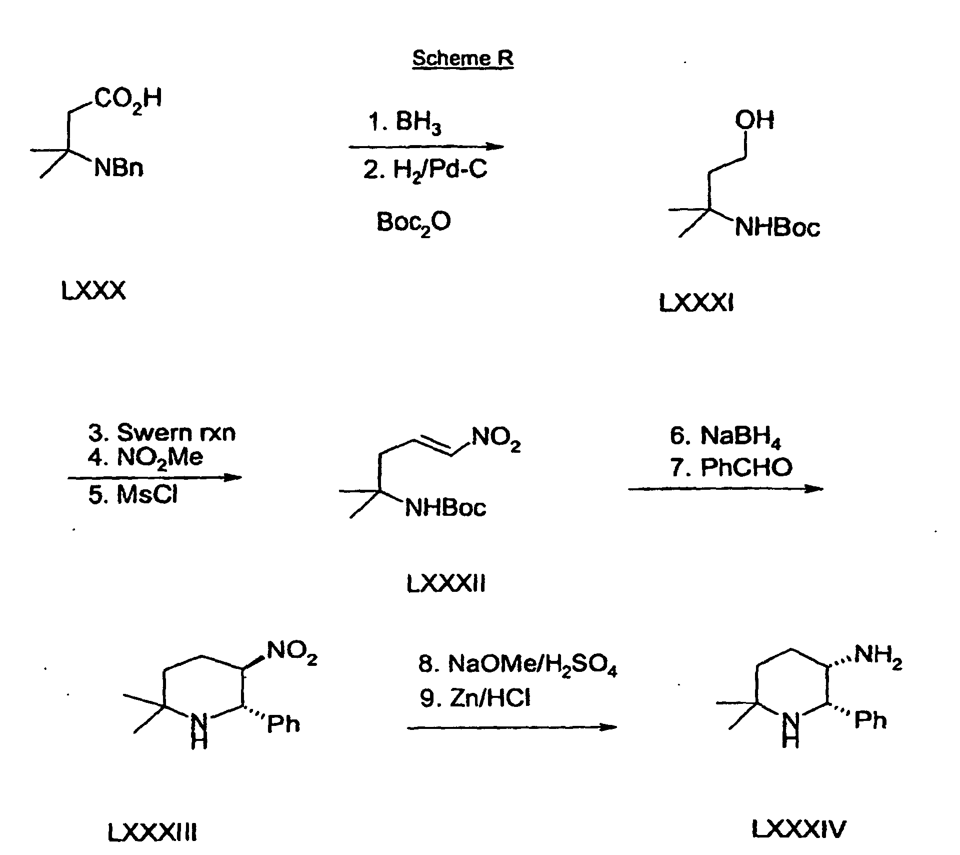

Referring to Scheme R, 3-Benzylamino-3-methyl-butyric acid is reduced to the corresponding N-Boc amino alcohol under standard conditions which are known to those skilled in the art. Preferably, this series of reactions is brought about by reduction of the carboxylic acid with borane or borane-dimethylsulfide complex in THF or ether. This is followed by hydrogenolysls of the N-benzyl group with palladium on carbon catalysis in ethanol or methanol at about room temperature under an atmosphere of hydrogen. Alternative hydrogen sources include ammonium formate or cyclohexene. The Boc group is installed under standard conditions using di-tert-butyldicarbonante in THF/water or dioxane/water in the presence of a carbonate base such as NaHCO₃ or K₂CO₃. Alternatively, this group may be installed in CH₂Cl₂, CHCl₃, THF, ether, toluene or related solvents in the presence of triethylamine or an alternative amine base. Oxidation of the alcohol to the aldehyde is accomplished under standard conditions associated with the use of Dess-Martin periodinane, Swern oxidation or MnO₂ oxidation. These methods are well known to those skilled in the art. A Henry reaction sequence yielding the nitroalkene of formula LXXXII is brought about by treatment of the aldehyde with nitromethane and a suitable base. Typical conditions include an alcoholic solvent such as methanol or ethanol and an alkoxide base such as sodium methoxide or sodium ethoxide. Alternative conditions include CH₂Cl₂, CHCl₃, THF or ether solvents and an amine base such as triethylamine. These conditions provide an intermediate nitroalcohol which can be converted into the nitroalkene by activation of the alcohol in the presence of base. This dehydration reaction is commonly known to those skilled in the art. The preferred method for this transformation involves activation of the alcohol with methanesulfonyl chloride or toluenesulfonyl chloride in the presence of triethylamine. The resultant nitroalkene is reduced to the nitroalkane under standard conditions. Preferred conditions for this transformation include treatment with NaBH₄ or LiBH₄ in alcoholic solvents such as methanol or ethanol. THF may also be used. The reaction may be conducted at temperatures between about -78°C and about 88°C, with about 0°C to about ambient temperature being preferred. Cyclization to the 2,3-trans piperidine ring is brought about by condensation with an aryl aldehyde such as benzaldehyde in the presence of a catalyst such as ammonium acetate or amonium chloride. The preferred solvent for this transformation is an alcoholic solvent such as methanol or ethanol and the typical temperature for this transformation lies between about -20°C and about 100 °C. Alternatively, the transformation may be brought about in solvents such as CH₂Cl₂, CHCl₃, THF, ether, toluene, ethyl acetate or related solvents, with or without acid or base catalysis. The 2,3-cis piperidine ring is obtained by treatment of the trans-piperidine with base followed by quenching in a kinetic fashion via addition of the nitronate solution to an excess of an acid in solution. Typical bases range from metal alkoxides or hydroxides in alcoholic solution to amine bases such as triethylamine or Hunig's base to LHMDS, KHMDS, NaHMDS in organic solvents such as CH₂Cl₂, CHCl₃, THF, ether, toluene, or ethylacetate. Alternative bases include the butyllithiums in THF or ether solution. Temperatures for this transformation can range between about -78°C and about 100°C. The nitropiperidine is transformed into the aminopiperidine by reduction according to a variety of possible methods known to those skilled in the art. Preferred methods include zinc/HCl, zinc/acetic acid, or iron/HCl reduction in suitable solvents including water, THF/water, or water/alcohol mixtures. Suitable temperatures range from about -20°C to about 118°C. The reduction may also be brought about by Raney nickel reduction in alcoholic solvents of in water/alcohol mixtures under an atmosphere of hydrogen. Typical reation temperatures range from about 0°C toabout 100°C. The resultant amine is subjected to reductive amination conditions as previously described to provide the compounds of interest.

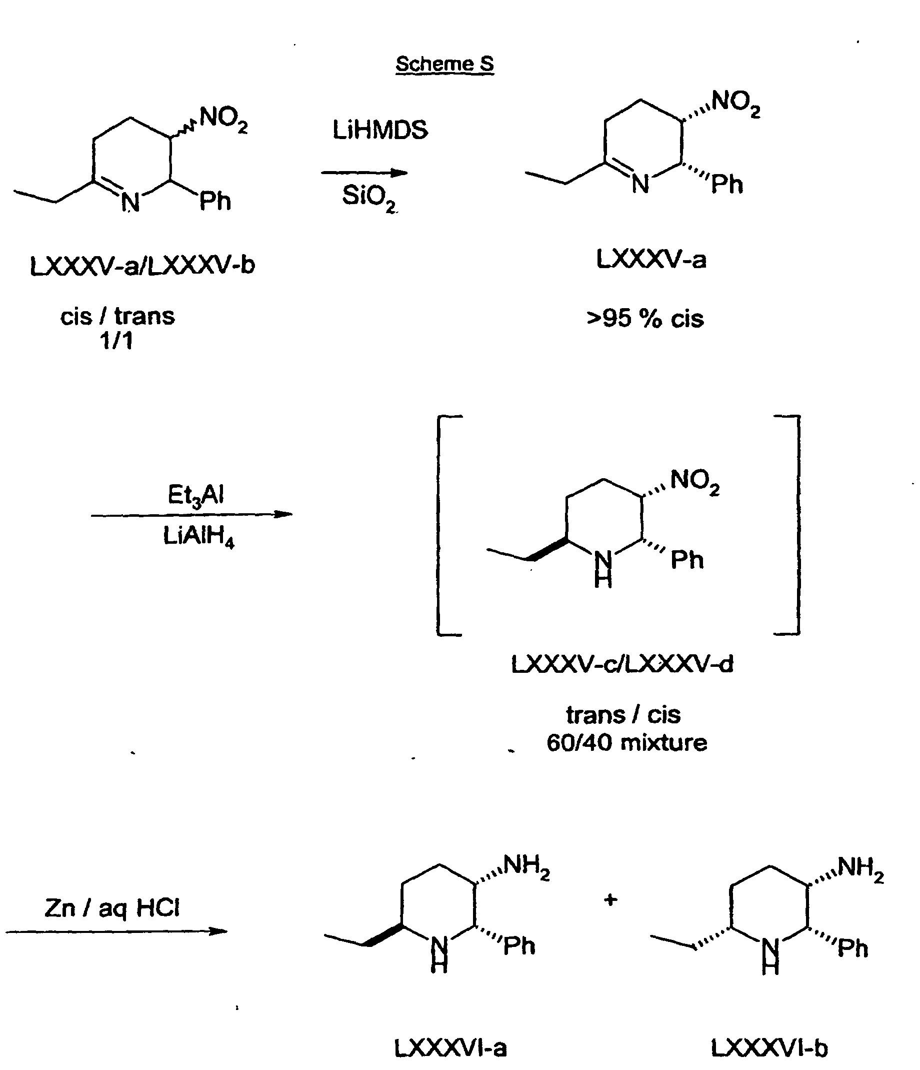

The mixture of cis and trans nitroimine of the formula LXXXV-a/LXXXV-b (compound of formula XXXIII prepared in Scheme J) may be transformed by the process of base induced epimerization to a single diastereomer LXXXV-a. A variety of bases may be used for this process including but not limited to lithium diisopropyl amide, sodium, lithium or potassium hexamethyldisilazane or potassium t-butoxide, potassium or sodium hydride, diazabicycloundecene (DBU), diazabicydononane (DBN), tetramethylguanidine, triethyl amine. diisopropylethylamine, sodium or potassium or lithium carbonate with or without accompanying 18-crown-6 or 15 crown-5, 12-crown-4, lithium sec-butylborohydride (L-selectride) in a solvent such as THF, ether or dimethoxyethane, at a temperature from about -100°C to about -25°C, for a period of about 15 minutes to about 5 hours. The reaction mixture is quenched into a suitable acidic reagent which is either suspended or dissolved in an inert solvent to afford the desired diastereomer LXXXV-a. Suitable quenching agents include silica gel, alumina, hydrated sodium sulfate, paratoluenesulfonic acid pyridinium salt (PPTS), boric acid, hydrogen chloride, aqueous hydrogen chloride or aqueous sulfuric acid, nafion resin, molecular sieves, acidic ion exchange resins and the like. Preferred conditions utilize lithium hexamethyldisilazane in THF at about -78°C followed by quench into a suspension of silica gel in THF.

Reduction of the imine of formula LXXXV-a to afford a mixture favoring either diastereomer of formula LXXXV-c/ LXXXV-d may be accomplished through the use of a reducing agent and optionally a Lewis acid activating agent. Thus the imine may be reduced with lithium aluminum hydride with or without added triethyl or trimethyl aluminum. Other aluminum reagents may be used such as diethyl aluminum chloride or ethylaluminum dichloride. Other reducing agents include Vitride™ or Red-Al or diisobutylaluminum hydride and also sodium borohydride, sodium cyanoborohydride and sodium triacetoxy borohydride with or without borontrifluoride etherate, lithium sec-butylborohydride (L-selectride) and/or lithium borohydride in the presence of borontrifluoride etherate. A suitable inert solvent is used during this transformation and may be selected from THF, ether, dimethoxyethane, toluene, hexane, methylene chloride or other suitable solvents. The reaction is generally run below room temperature from about -78°C to about 0°C. The most preferred conditions use lithium aluminum hydride in THF at about -78°C with triethyl aluminum.

The resulting imine of formula LXXXV-c or LXXXV-d or the mixture of compounds of formulas LXXXV-c/LXXXV-d may be reduced to either a compound of formula LXXXVI-a or a compound of formula LXXXVI-b or a mixture of compounds of formulas LXXXVI-a/LXXXVI-b with a suitable reducing agent. Agents capable of reducing compounds of formulas LXXXV-c/LXXXV-d include, but are not limited to, zinc, tin, stannous chloride, zinc amalgam, palladium on carbon, palladium hydroxide, platinum oxide, platinum on carbon and Raney nickel, with or with out the presence of from 1-1000 psi hydrogen pressure, in a suitable solvent such as hydrochloric acid (aq), water, methanol, ethanol, isopropanol, ethyl acetate or similar inert solvents. The most preferred conditions include zinc metal in aqueous hydrochloric acid at a temperature from about room temperature to about the reflux temperature of the solvent.

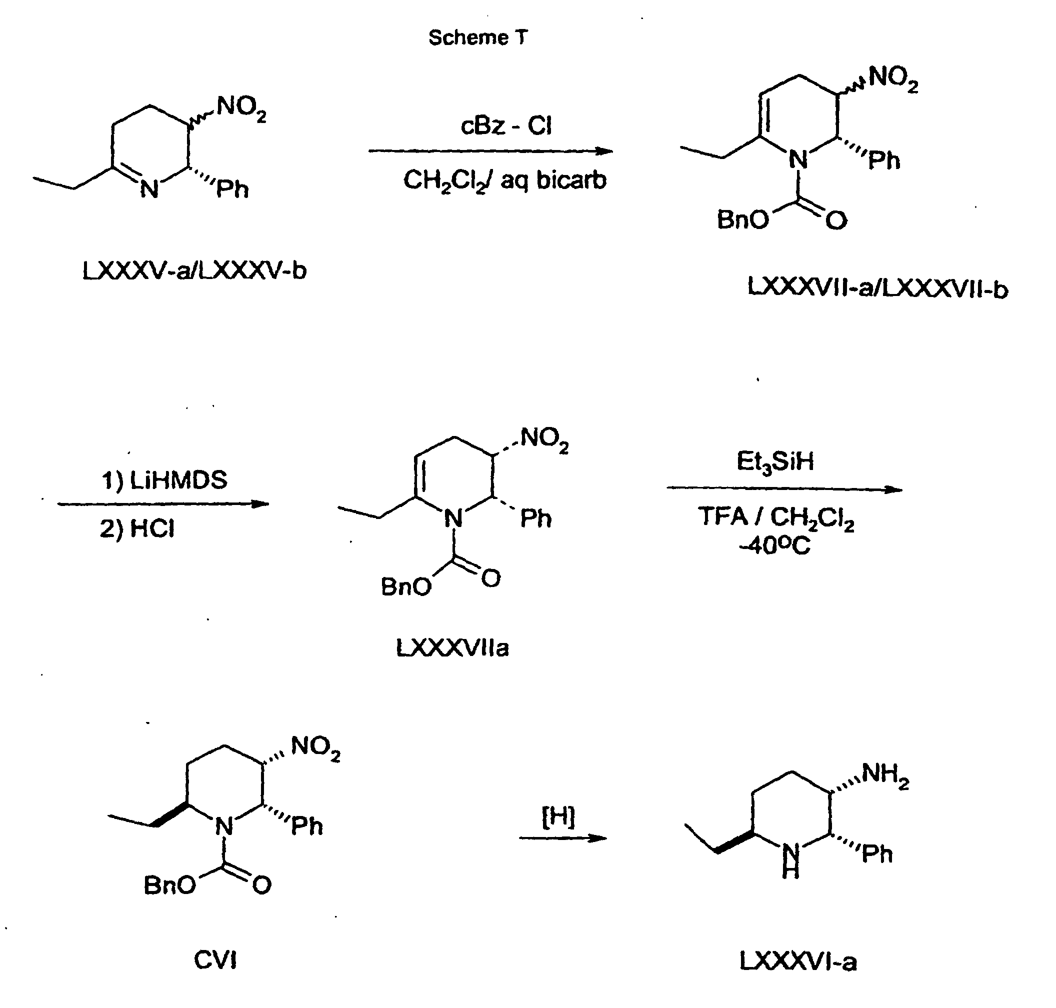

Alternatively, according to Scheme T, a compound of the formula LXXXVI-a may also be prepared from compounds of the formulas LXXXV-a/LXXXV-b in a straightforward manner. The imine of formula LXXXV-a/LXXXV-b prepared above can be converted to an enamine of the formula LXXXVII-a/LXXXVII-b through reaction with an activated acyl compound such as, but not limited to, carbobenzyloxychloroformate (cBz-Cl), benzoyl chloride, 9-fluorenylmethyl choroformate(FMOC-Cl), t-butoxychloroformate, phenyl chloroformate, nitro and dinitrophenylchloroformate, methyl, ethyl and isopropyl chloroformate in an inert solvent and in the presence of a suitable base. Suitable solvents include methylene chloride, chloroform, dichloroethane, benzene, toluene, water, ethyl acetate, dioxane and other suitable organic solvents. Typical bases include aqueous sodium, lithium and potassium carbonate or bicarbonate solutions, pyridine, triethylamine, diisopropylethylamine, lutidine, collidine and other suitable bases. The reaction may be run at about room temperature up to about the reflux temperature of the solvent. The most preferred conditions involve reaction of a compound of formula LXXXV-a/LXXXV-b with cBz-Cl in methylene chloride and aqueous sodium bicarbonate solution at about the reflux temperature of the solvent mixture.

The desired cis isomer of formula LXXXVII-a may be obtained directly from the foregoing reaction by direct crystallization of the crude product from a suitable solvent such as diethyl ether or diisopropyl ether. Alternatively, the mixture of cis and trans isomers of formula LXXXVII-a/LXXXVII-b may be converted to mainly the cis isomer of formula LXXXVII-a by treatment with a suitable base such as lithium or sodium bis (trimethylsilyl)amide, lithium diisopropylamide, DBU, sodium or potassium carbonate in a suitable solvent such as THF or DME at a temperature of about -78°C to about ambient temperature followed by quenching in a suitable aqueous acid such as dilute aqueous hydrochloric acid or dilute acetic acid. The most preferred conditions involve reaction of the compound of formula LXXXVII-a/LXXXVII-b with lithium bis (trimethylsilyl)amide in THF at about -78°C followed by quenching in 1N aqueous HCl.

The reduction of the compound of formula LXXXVII-a to form the compound of formula CVI may be carried out in several ways. The reduction may be conducted under acidic or neutral conditions in an inert solvent at a temperature between a bout -78°C a nd about room temperature. Suitable reducing agents include 1-1000 psi hydrogen gas, ammonium formate, sodium cyanoborohydride, sodium triacetoxyborohydride, tetrabutylammonium triacetoxyborohydride, triethylsilane, polyhydroxysilane, and sodium and lithium borohydride. If the reaction requires a suitable acid, then acetic or trifluoroacetic acid may be added. Additionally, hydrogen chloride triflic acid or sulfuric acid may be used. Typical solvents include methylene chloride, dichloroethane, chloroform, methanol, ethanol, toluene, dioxane and water. The most preferred conditions involve reaction of the compound of formula LXXXVII-a with sodium cyanoborohydride in methylene chloride with trifluoroacetic acid at about -40°C: Equally preferred are conditions involving reaction of the compound of formula LXXXVII-a with triethylsilane in methylene chloride with trifluoroacetic acid at about -40°C.

Deprotection of the acyl group and reduction of the nitro to an amine as in the compound of formula LXXXVI-a may be done in either order. For instance, reduction of the nitrogroup may be carried out with agents such as zinc, tin, stannous chloride, zinc amalgam, palladium on carbon, palladium hydroxide, platinum oxide, platinum on carbon or Raney nickel, with or with out the presence of from 1-1000 psi hydrogen pressure, in a suitable solvent such as hydrochloric acid (aq), water, methanol, ethanol, isopropanol, ethyl acetate or similar inert solvents. The most preferred conditions include zinc metal in aqueous hydrochloric acid at a temperature from about room temperature to about the reflux temperature of the solvent. Removal of the acyl group may be carried out in a variety of ways. If the acyl group is cBz- then catalytic hydrogenation is successful and may or may not be coupled with reduction of the nitro group. General conditions indude hydrogenation with 1-1000 psi hydrogen over palladium on carbon or palladium hydroxide or oxide in methanol or ethanol. Alternatively, hydrogenolysis may be carried out with ammonium formate or cyclohexene under reflux in methanol or ethanol over a catalyst such as palladium on carbon or palladium hydroxide or oxide. An alternative method for cBz cleavage is treatment with HBr in acetic acid or propionic acid solution. For other acyl groups such as tertiary butoxy- (t-BOC), simple treatment with strong acid is sufficient, whereas with other acyl groups, treatment with aqueous sodium hydroxide solution at a temperature from ambient temperature to about the reflux temperature of the solvent is necessary. Alternatively, cBz or t-BOC groups may be removed by treatment with HBr/acetic acid.

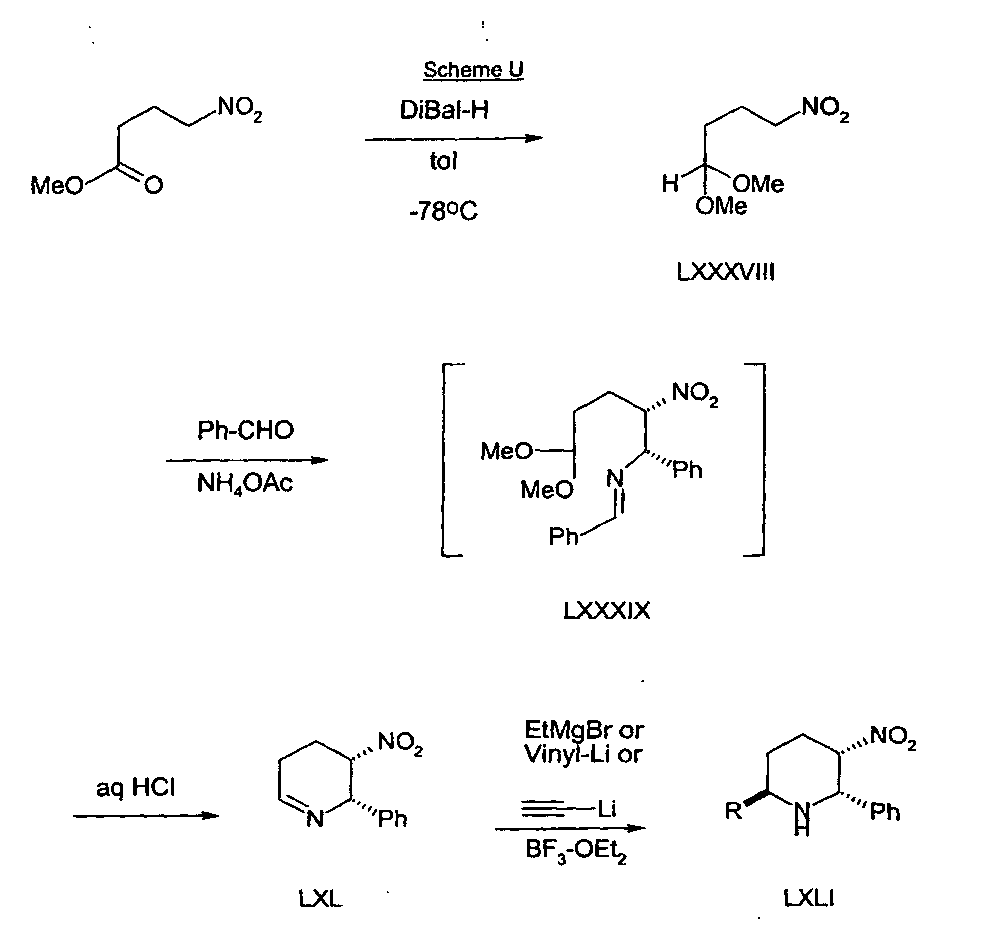

Alternatively, compounds of formula LXLI may be prepared by the sequence of reactions illustrated in Scheme U. 4-nitrobutyric acid methyl ester or 4-nitrobutyric acid ethyl ester may be reduced to an aldehyde and said aldehyde be protected as a nitro acetal of formula LXXXVIII. Reduction of the 4-nitrobutyric acid methyl ester may be completed in one step through reaction in toluene, hexane or methylene chloride at about -78°C with diisobutylaluminum hydride. Alternatively, this reduction may be carried out over two steps via reduction of the 4 -nitrobutyric acid methyl ester in THF or ether at about 0°C to about ambient temperature through the use of lithium aluminum hydride, lithium borohydride or borane in THF. The resulting nitroalcohol may then be oxidized to the aldehyde through a Swern oxidation in methylene chloride or a Dess-Martin oxidation. The aldehyde is protected as a nitroacetal of formula LXXXVIII by use of a mineral acid such as hydrochloric acid, sulfuric acid, and nitric acid or a catalytic organic acid such as camphor sulfonic acid or toluene sulfonic acid, in an alcoholic solvent such as methanol, ethanol, propanol, or ethylene glycol, with a water scavenger such as trimethyl orthoformate, triethyl orthoformate, magnesium sulfate, or molecular sieves, at a temperature from about 0°C to about 75°C.

Compounds of formula LXL can be prepared by using a modified Henry reaction that condenses to give substituted cyclic imines. The nitroacetal of formula LXXXVIII is condensed *in situ* with an imine created by an a mine source such as ammonium acetate, ammonium chloride, or ammonium formate, and an aldehyde such as one of variously substituted aromatic aldehydes, at a temperature from about 0°C to about 75°C. The acetal of formula LXXXIX is then converted into a nitroaldehyde by addition of a mineral acid such as hydrochloric acid, sulfuric acid, or nitric acid in water, at a temperature from a bout 0°C to about 75°C, which causes cyclization to occur, giving the compound of formula LXL. Preferably, the nitroaldehyde is dissolved in methanol at about room temperature with a catalytic amount of camphor sulfonic acid and trimethyl orthoformate. Ammonium formate is then added, followed by benzaldehyde. Aqueous hydrochloric acid is added and stirred to give the cyclic imine of formula LXL.

The imine of formula LXL may be transformed to the compound of formula LXLI through the use of organometallic reagents. For instance, addition of ethyl lithium, ethylmagnesium bromide, diethyl magnesium, diethylzinc, ethylzinc chloride, diethyl cuprate or other cuprate reagents such as higher order cuprates in a solvent such as THF or ether, toluene or dimethoxyethane at a temperature from about - 78°C to about 0°C. Alternatively, in a two step process, one may add vinyl lithium, vinylmagnesium bromide, divinyl magnesium, divinylzinc, vinylzinc chloride, divinyl cuprate or other cuprate reagents such as higher order cuprates in a solvent such as THF or ether, toluene or dimethoxyethane at a temperature from about -78°C to about 0°C. Once addition is complete the resulting product is hydrogenated under 1-1000 psi hydrogen pressure over palladium on carbon, palladium hydroxide, platinum oxide, platinum on carbon or Raney nickel. Alternatively, in another two step process, one may add an acetylenic lithium under similar conditions to those listed above for vinyl. This process is also followed by hydrogenation. Optionally, one may include a Lewis acid in the above additions to the imine. Typical Lewis acids indude boron trifluoride etherate, zinc chloride and trimethyl or triethylaluminum.

The resulting amine of formula LXLI may be reduced to the compound of formula LXXXVI-a (Scheme T) with a suitable reducing agent. Agents capable of reducing LXLI include, but are not limited to, zinc, tin, stannous chloride, zinc amalgam, palladium on carbon, palladium hydroxide, platinum oxide, platinum on carbon and Raney nickel, with or with out the presence of from 1-1000 psi hydrogen pressure, in a suitable solvent such as hydrochloric acid (aq), water, methanol, ethanol, isopropanol, ethyl acetate or similar inert solvents. The most preferred conditions include zinc metal in aqueous hydrochloric acid at a temperature from about room temperature to about the reflux temperature of the solvent.

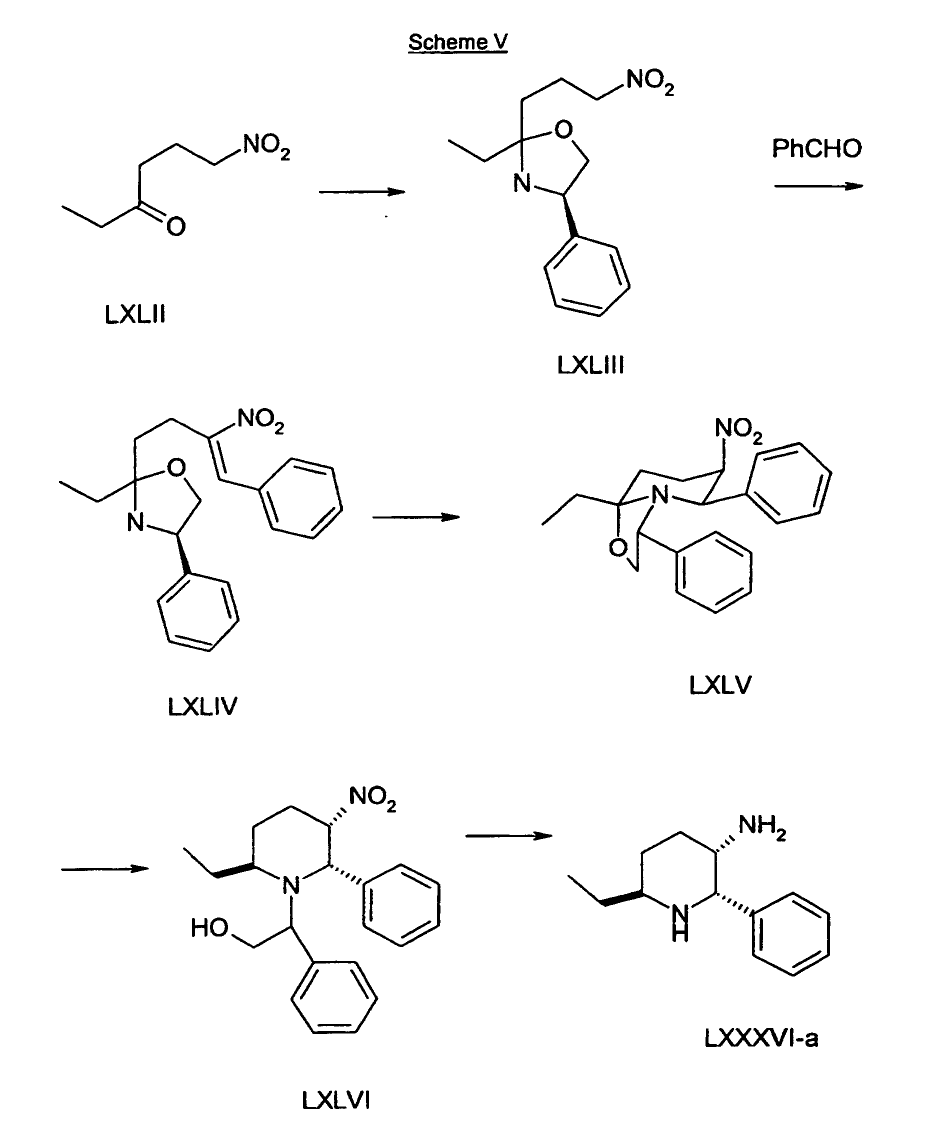

Alternatively, a compound of the formula LXXXVI-a may be prepared by the sequence of reactions shown in Scheme V. 1-nitrohex-4-one (the compound of formula LXLII) prepared above, may be reacted to form a hemiacetal of formula LXLIII through reaction of phenylglycinol with either configuration in a suitable solvent such as benzene, toluene, dichloroethane, methanol or ethanol with optional removal of water by molecular sieves or through the use of a Dean Stark water separator while under reflux. Compounds of the formula LXLIV can be prepared by using a modified Henry reaction which then condenses to give the substituted cyclic acetal of formula LXLV. The cyclic acetal of formula LXLV is condensed *in situ* with an imine created by an amine source such as ammonium acetate, ammonium chloride, or ammonium formate, and an aldehyde such as benzaldehyde or one of variously substituted aromatic aldehydes, at a temperature from about 0°C to about 75°C. In most cases the compound of formula LXLIV is not isolated but directly cyclizes to the compound of formula LXLV.

The reduction of the compound of formula LXLV to form the compound of formula LXLVI may be carried out in several ways. The reduction may be conducted under acidic or neutral conditions in an inert solvent at a temperature between about -78°C and about room temperature. Suitable reducing agents include 1-1000 psi hydrogen gas, ammonium formate, sodium cyanoborohydride, sodium triacetoxyborohydride, tetrabutylammonium triacetoxyborohydride, triethylsilane, polyhydroxysilane, and sodium and lithium borohydride. If the reaction requires a suitable acid, then acetic or trifluoroacetic acid and boron trifluoride etherate or trimethyl aluminum may be added. Additionally, hydrogen chloride, triflic acid or sulfuric acid may be used. Typical solvents include methylene chloride, dichloroethane, chloroform, methanol, ethanol, toluene, dioxane and water.

The compound of formula LXLVI may be deprotected through hydrogenolysis. Typically, the nitro group is also reduced to the amine of formula LXXXVI-a during this process. General conditions indude hydrogenation with 1-1000 psi hydrogen over palladium on carbon or palladium hydroxide or oxide in methanol or ethanol. Alternatively, hydrogenolysis may be carried out with ammonium formate or cyclohexene under reflux in methanol or ethanol over a catalyst such as palladium on carbon or palladium hydroxide or oxide. If the nitro group is to be removed prior to hydrgenolysis, then suitable reaction conditions indude the following. The nitro functionality may be reduced by agents which include, but are not limited to, zinc, tin, stannous chloride, zinc amalgam, palladium on carbon, palladium hydroxide, platinum oxide, platinum on carbon and Raney nickel, with or with out the presence of from 1-1000 psi hydrogen pressure, in a suitable solvent such as hydrochloric acid (aq), water, methanol, ethanol, isopropanol, ethyl acetate or similar inert solvents. The most preferred conditions indude zinc metal in aqueous hydrochloric acid at a temperature from about room temperature to the about reflux temperature of the solvent.

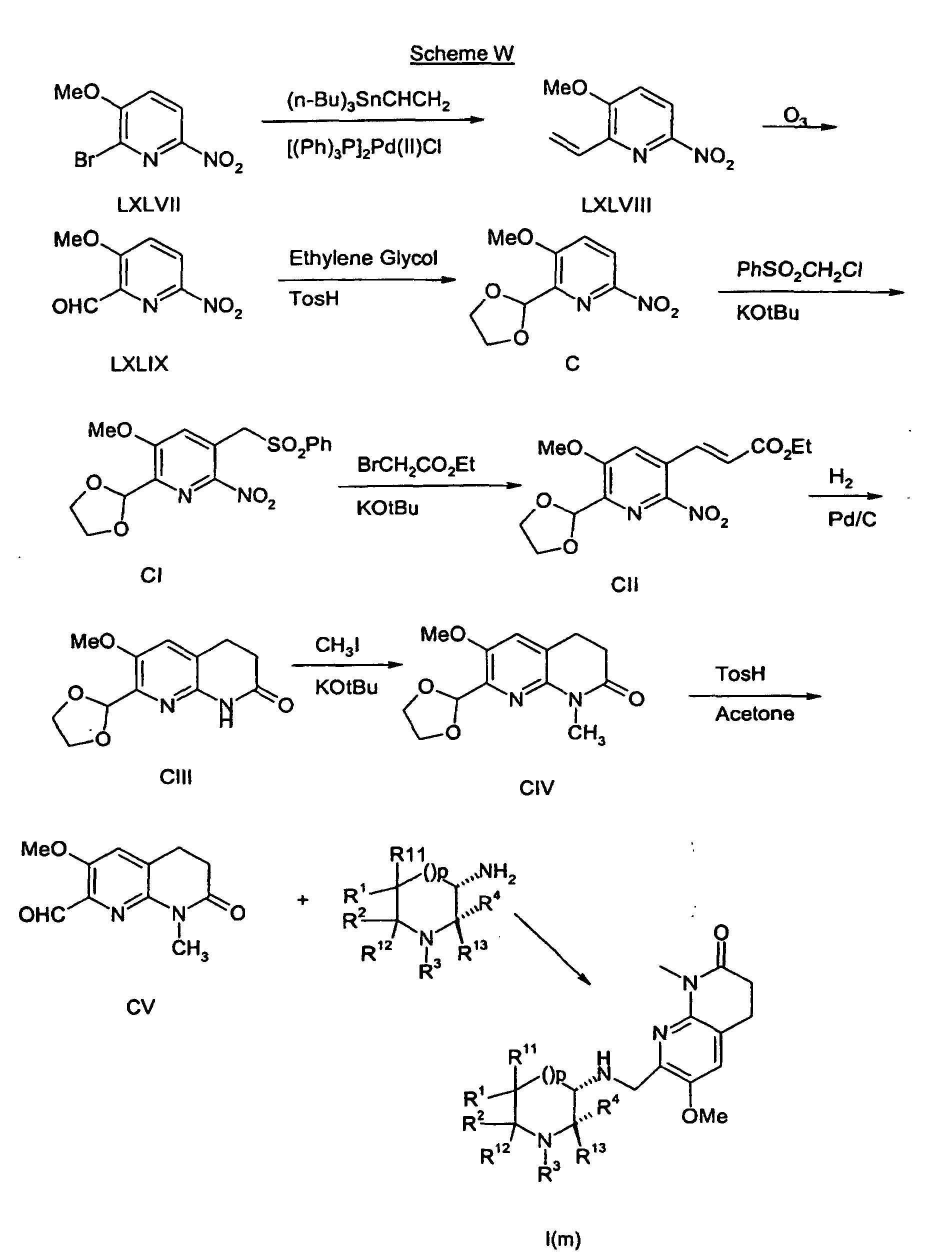

2-Bromo-3-methoxy-5-nitropyridine (J. Lombardino, *J. Med. Chem*. **1981**, *24*, 39-42) was converted to the 2-aldehyde derivative of formula LXLIX using a two step sequence. The compound of formula LXLVII was treated with tri-n-butyl-vinyltin using a catalytic amount of bis(triphenylphosphine)-palladium(II) chloride in a non-protic solvent (preferably toluene) at about room temperature to about 150° C (preferably 111°C) for about 1 hour to about 24 hours (preferably 2 hours). Chromatography of the reaction mixture yields the vinyl compound of formula LXLVIII: The vinyl compound of formula LXLVIII was treated with ozone at about -100° C to about 0°C (preferably -78°C) in methylene chloride and then quenched with dimethyl sulfide to afford the aldehyde of formula LXLIX. The aldehyde of formula LXLIX was protected using methods well known to those of skill in the art, *e.g*., reaction with ethylene glycol in benzene in the presence of a catalytic amount of acid (preferably p-toluenesulfonic acid) to afford the acetal of formula C. The pyridine derivative of formual C was reacted with benzylsulfonyl chloride using a strong base (preferably potassium t-butoxide) in a non-protic solvent (preferably a mixture of THF and DMF) to afford the sulfone derivative of formula CI. The methylene group of the sulfone of formula CI can be alkylated with ethyl bromoacetate in a protic solvent (preferably ethanol) using a strong base (preferably potassium t-butoxide). During these reaction conditions benzenesulfinic acid is eliminated to form the ester of formula CII. The ester of formula CII is hydrogenated in a protic solvent (preferably ethanol) using a metal catalyst (preferably 10% palladium on carbon) at about atmospheric pressure to about 50 PSI (preferably 50 PSI). During this reaction the nitro group is reduced to an amine, the double bond is reduced, and cyclization to the 6-membered amide is accomplished to yield the amide of formula CIII. Alkylation of the amide of formula CIII is accomplished with methyl iodide using a strong base (preferably potassium-t-butoxide) in a non-protic solvent (preferably THF) to afford the amide of formula CIV. The aldehyde in the amide of formula CIV is unmasked using acid (preferably p-toluenesulfonic acid) in a non-protic solvent (preferably acetone) to afford the aldehyde of formula CV. Reductive amination using aminopiperidine T-NH₂ as described in Scheme A-1 affords the product of formula I(m).

The preparation of other compounds of the formula I not specifically described in the foregoing experimental section can be accomplished using combinations of the reactions described above that will be apparent to those skilled in the art.

In each of the reactions discussed or illustrated above, pressure is not critical unless otherwise indicated. Pressures from about 0.5 atmospheres to about 5 atmospheres are generally acceptable, and ambient pressure, i.e., about 1 atmosphere, is preferred as a matter of convenience.

The compounds of formula I, and the intermediates shown in the above reaction schemes can be isolated and purified by conventional procedures, such as recrystallization or chromatographic separation.

The compounds of the formula I and their pharmaceutically acceptable salts can be administered to mammals via either the oral, parenteral (such as subcutaneous; intraveneous, intramuscular, intrastemal and infusion techniques), rectal, intranasal or topical routes. In general, these compounds are most desirably administered in doses ranging from about 0.01 to about 1500 mg per day, in single or divided doses (i.e., from 1 to 4 doses per day), although variations will necessarily occur depending upon the species, weight and condition of the subject being treated and the particular route of administration chosen. However, a dosage level that is in the range of about 0.5 mg to about 500 mg per kg of body weight per day is most desirably employed. Nevertheless, variations may occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effects, provided that such higher dose levels are first divided into several small doses for administration throughout the day.

The compounds of the present invention may be administered alone or in combination with pharmaceutically acceptable carriers or diluents by either of the routes previously indicated, and such administration may be carried out in single or multiple doses. More particularly, the novel therapeutic agents of this invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutically-effective compounds of this invention are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a compound of the present invention in either sesame or peanut oil or in aqueous propylene glycol may be employed. The aqueous solutions should be suitably buffered (preferably pH greater than 8) if necessary and the liquid diluent first rendered isotonic. These aqueous solutions are suitable for intravenous injection purposes. The oily solutions are suitable for intra-articular, intra-muscular and subcutaneous injection purposes. The preparation of all these solutions under sterile conditions is readily accomplished by standard pharmaceutical techniques well known to those skilled in the art.

Additionally, it is also possible to administer the compounds of the present invention topically when treating inflammatory conditions of the skin and this may be done by way of creams, jellies, gels, pastes, patches, ointments and the like, in accordance with standard pharmaceutical practice.

The activity of the compounds of the present invention as substance P antagonists is determined by their ability to inhibit the binding of substance P at its receptor sites in IM-9 cells employing radioactive ligands. The substance P antagonist activity of the compounds described herein is evaluated by using the standard assay procedure described by D. G. Payan et al., as reported in the The Joumal of Immunology. 133, 3260 (1984). This method essentially involves determining the concentration of the individual compound required to reduce by 50% the amount of radiolabelled substance P ligands at their receptor sites in said isolated cow tissues or IM-9 cells, thereby affording characteristic IC₅₀ values for each compound tested. More specifically, inhibition of [³H]SP binding to human IM-9 cells by compounds are determined in assay buffer (50 mM Tris-HCl (pH 7.4), 1 mM MnCl₂, 0.02 % bovine serum albumin, bacitracin (40 µg/ml), leupeptin (4 µg/ml), chymostatin (2 µg/ml) and phosphoramidon (30 µg/ml)). The reaction is initiated by the addition of cells to assay buffer containing 0.56 nM [³H]SP and various concentrations of compounds (total volume 0.5 ml) and allowed to incubate for 120 min at 4 °C. Incubation is terminated by filtration onto GF/B filters (presoaked in 0.1 % polyethylenamine for 2 hours). Nonspecific binding is defined as the radioactivity remaining in the presence of 1 µM SP. The filters are placed into tubes and counted using liquid scintillation counter.

All of the title compounds of the examples were tested and at least one stereoisomer of each such compound exhibited a binding affinity, measured as Kᵢ, of at least 600 nM.

The activity of the compounds of this invention against generalized anxiety disorder can be determined by inhibition of GR73632-induced tapping test in gerbils. More specifically, gerbils are lightly anesthetized with ether and the skull surface is exposed. GR73632 or vehicle (PBS, 5 µl) are administered directly into the lateral ventricles via a 25 gauge needle inserted 4.5 mm below bregma (preceded by pretreatment with an antagonist, 0.1-32.0 mg/kg, s.c. or p.o.). Following injection, gerbils are placed in 1 L beaker individually and monitored for repetitive hind paw tapping. Some compounds prepared in the following Examples were tested in accordance with these testing methods. As a result, it was found that the compounds of the present inventions have good antagonist activity toward substance P, particutarly good activity against CNS disorders with decreased side effects.

The present invention is illustrated by the following examples. It will be understood, however, that the invention is not limited to the specific details of these examples. Melting points are uncorrected. Proton nuclear magnetic resonance spectra (¹H NMR) and ¹³C nuclear magnetic resonance spectra were measured for solutions in deuterochloroform (CDCl₃) or in CD₃OD or CD₃SOCD₃ a nd peak positions are expressed in parts per million (ppm) downfield from tetramethylsilane (TMS). The peak shapes are denoted as follows: s, singlet; d, doublet; t, triplet; q, quartet; m, multiplet; b, broad.

### EXPERIMENTAL PROCEDURES

### Preparation 1 (reference)

### 6-Hydroxy-2-oxo-1,2,3,4-tetrahydro-quinoline

P-aminophenol [0.5 gm (4.58 mmol)] was dissolved in 30 ml of each methylene chloride and saturated aqueous bicarbonate solution and it was stirred for 5 min at ambient temperature. 3-chloropropionyl chloride [0.49 ml (5.04 mmol.)] was added over 10 min. and the reaction mixture was stirred at ambient temperature for 4 hours. A large amount of precipitate was observed. The solids were filtered and dried to afford 0.82 gm (90 %) of an off white solid. MS APCI m/e 200 (p+1)

Combine 0.82 gm (4.1 mmol.) and 1.6 gm (12.3 mmol.) aluminum chloride as a mixture of solids. The mixture was then heated in an oil bath at 210°C for 10 min or until the gas evolution ceases. The reaction mixture was then allowed to cool to ambient temperature and then quenched in ice/water mixture. The aqueous phase was extracted with ethyl acetate which was separated, dried over sodium sulfate and evaporated in vacuo to a light brown solid 0.58 gm (87 %) MS APCI m/e 164 (p+1)

### Preparation 2 (reference)

### 6-Methoxy-2-oxo-1,2,3,4-tetrahydro-quinoline

A solution of 0.58 gm (3.56 mmol.) 6-hydroxy-2-oxo-1,2,3,4-tetrahydro-quinoline was prepared in 10 ml acetone followed by the addition of 1.46 gm (10.58 mmol.) potassium carbonate and 0.51 ml (5.36 m mol.) dimethylsulfate. The reaction mixture was stirred at ambient temperature for 16 hours and then evaporated in vacuo. The residue was partitioned between saturated aqueous bicarbonate solution and methylene chloride. The organic phase was dried over sodium sulfate and evaporated. The residue was purified by silica gel chromatography eluting with 96/4 methylene chloride/methanol to afford 0.53 gm (85 %) of the desired product as a white solid. MS APCl m/e 178 (p+1)

### Preparation 3 (reference)

### 6-Methoxy-2-oxo-1,2,3,4-tetrahydro-quinoline-7-carbaldehyde

A mixture of 0.29 gm (2.19 mmol) aluminum chloride in 5 ml of methylene chloride was prepared under nitrogen gas N₂ and stirred for 15 minutes and then cooled to 0°C. The mixture was treated with 0.2 gm (1.13 mmol) 6-Methoxy-3,4-dihydro-1H-quinolin-2-one in 5 ml of methylene chloride. The reaction mixture was stirred for 10 minutes at this temperature and was then cooled to - 5°C. α,α-dichloromethyl m ethyl ether 0.28 ml (3.07 mmol) was added over a 5 minutes, period and the green reaction mixture was slowly warmed to room temperature and stirred for 6 hours. The reaction mixture was diluted with 2N HCl and extracted with methylene chloride (4 X 10 ml). The combined organics were dried over sodium sulfate, filtered and concentrated to yield an off white solid. The crude product was chromatographically purified on silica gel, eluting with 7/3 ethyl acetate / hexane. There was obtained 125 mg (54 %) of an off white solid. MS APCI m/e 206 (p+1)

### Reference Example 1

### 6-Methoxy-7-[(2-phenyl-piperidin-3-ylamino)-methyl]-3,4-dihydro-1H-quinolin-2-one

To a flame dried round bottom flask fitted with Dean-Stark trap, condenser and nitrogen atmosphere was placed: 66 mg (0.37 mmole) cis-(2S,3S)-3-amino, 2-phenylpiperidine and 77 mg (0.37 mmole) 6-Methoxy-2-oxo-1,2,3,4-tetrahydro-quinoline-7-carbaldehyde in 15 ml of toluene containing 3A molecular sieves. The reaction mixture was heated under reflux for 6 hours and monitored by mass spectrum analysis for the presence of the imine intermediate. The reaction mixture was allowed to cool to ambient temperature and then evaporated in vacuo. The residue was taken up in approximately 15 ml of dichloroethane and was treated with 102 mg (0.48 mmol.) sodium triacetoxybrohydride and then stirred for 16 hours under nitrogen at ambient temperature. The reaction mixture was then washed with aqueous saturated sodium bicarbonate solution, washed with brine and then dried and evaporated in vacuo. The residue was purified by column chromatography on silica gel eluting with 95/5 CHCl₃/MeOH containing 3 drops of concentrated NH₄OH solution. There was obtained 100 mg of the free base (75 %) which was converted to the above named product as the hydrochloride in the following manner. Treatment of methanol with 3 equivalents (53 µl, 0.82 mmol) acetyl chloride afforded a methanolic hydrochloric acid (HCl) solution, which was stirred for 10 minutes. The free base was added in methanol and the mixture was again stirred for 10 minutes and then evaporated in vacuo. The residue was taken up in the minimum amount of methanol and treated with ether until a cloudy precipitate form. Upon standing the di-HCl salt was obtained in 46 % overall yield (75 mg). Mp 233-235°C MS, APCI m/e 366 (p+1).

### Preparation 4 (reference)

### 1-Ethyl-6-methoxy-2-oxo-1,2,3,4-tetrahydro-quinoline-7-carbaldehyde

6-Methoxy-2-oxo-1,2,3,4-tetrahydro-quinoline-7-carbaldehyde (preparation 3) 60 mg (0.293 mmol) was dissolved in 5 ml of anhydrous THF and the solution was cooled to 0°C. The reaction mixture was treated with 33 mg (0.293 mmol) of potassium t-butoxide whereupon it became yellow in color. After stirring for 30 minutes, the mixture was treated with 23 µl (0.293 mmol) ethyl iodide and slowly warmed to room temperature while stirring for 16 hours (h). The reaction mixture was diluted with saturated aqueous sodium bicarbonate solution and extracted with ethyl acetate. The organic phase was dried over sodium sulfate and concentrated to an oil. The residue was chromatographed on silica gel eluting with 6/4 ethyl acetate / hexane to afford 34 mg (40 %) as a white solid. MS APCI m/e 234 (p+1)

### Preparation 5 (reference)

### 1-Methyl-6-methoxy-2-oxo-1,2,3,4-tetrahydro-quinoline-7-carbaldehyde

6-Methoxy-2-oxo-1,2,3,4-tetrahydro-quinoline-7-carbaldehyde 30 mg (0.146 mmol) was dissolved in 5 ml of anhydrous THF and the solution was cooled to 0°C. The reaction mixture was treated with 16 mg (0.146 mmol) of potassium t-butoxide whereupon it became yellow in color. After stirring for 30 minutes, the mixture was treated with 14 µl (0.146 mmol) dimethylsulfate and slowly warmed to room temperature while stirring for 16 hours. The reaction mixture was diluted with saturated aqueous sodium bicarbonate solution and extracted with ethyl acetate. The organic phase was dried over sodium sulfate and concentrated to an oil. The residue was chromatographed on silica gel eluting with 6/4 ethyl acetate/hexane to afford 16 mg (50 %) as a white solid. MS APCl m/e 220 (p+1)

### Reference Example 2

### 1-Ethyl-6-methoxy-7-[(2-phenyl-piperidin-3-ylamino)-methyl]-3,4-dihydro-1H-quinolin-2-one

To a 50 ml round bottom flask fitted with a Dean Stark water separator, condenser and nitrogen cap was placed 0.027 gm (0.151 mmole) cis-(2S,3S)-3-amino, 2-phenylpiperidine and 0.033 gm (0.151 mmole) 1-Ethyl-6-methoxy-2-oxo-1,2,3,4-tetrahydroquinoline-7-carbaldehyde (preparation 5) in 10 ml of toluene containing 3A molecular sieves. The reaction mixture was heated for 18 hours under reflux. The solution was then allowed to cool to room temperature and then evaporated in vacuo. The residue was taken up in 25 ml of dichloroethane and treated with 0.048 gm (0.229 mmol) sodium triacetoxyborohydride and the solution was stirred at ambient temperature for 16 hours. The reaction was then partitioned between saturated aqueous bicarbonate solution and dichloromethane. The organic phase was washed with saturated brine and then dried over sodium sulfate before evaporation in vacuo. The crude product was chromatographed on silica gel eluting with 95 CHCl₃, 4 MeOH, 1 NH₄OH. The resulting oil was taken up in methanol which had previously been treated with 17.5 µl of acetyl chloride. The solution was evaporated in vacuo and the dihydrochloride salt of the desired product was obtained by recrystallization from methanol - ether; 33 mg (47 %). Mp 253 - 255°C; MS, APCI m/e 394 (p+1).

### Preparation 6 (reference)

### 6-Methoxy-1-methyl-1H-quinolin-2-one

A solution of 10 gm (63 mmol) 6-methoxyquinoline in 150 ml of acetone was treated with 4.4 ml (70 m mol) o f methyl iodide and heated under reflux for 4.5 hours followed by stirring at ambient temperature for 16 hours. The desired product was obtained by filtration of the solution. [14 gm (74%)]. This material was suspended in 360 ml of water and was cooled to 5°C. The mixture was treated with 90 gm (0.273 mol) potassium ferricyanide in a portionwise fashion over 1 hour. This mixture was kept at 5°C for 30 minutes and was then treated over 30 minutes with a solution of 31 gm (0.56 mol) KOH in 65 ml of water which had been previously cooled in an ice bath. The mixture becomes very very thick. Added 250 ml of toluene and heated to 40°C in a water bath for 30 minutes. The organic layer was separated and the aqueous phase was extracted in the same fashion twice more. The combined organic phase was washed with brine, dried over sodium sulfate and evaporated in vacuo to afford 8.2 gm of the desired product . Mass Spectrum APCI m/e 190 (p+1).

### Preparation 6A (reference)

### 6-Methoxy-1-methyl-1H-quinolin-2-one

A solution of 50 gm (314.1 mmol) 6-methoxyquinoline in 650 ml of acetone was treated with 21.5 ml (345.5 mmol) of methyl iodide and heated under reflux for 6 hours followed by stirring at ambient temperature for 16 hours. The desired product was obtained by filtration of the solution. [81.4 gm (91%)]. This material (285.4 mmol) was suspended in 2000 ml of water and was cooled to 5°C. The mixture was treated with 552.6 gm (1.68 mol) potassium ferricyanide in a portionwise fashion over 1 hour. This mixture was kept at 5°C for 30 minutes and was then treated over 30 minutes with a solution of 191.8 gm (3.43 mol) KOH in 400 ml of water which had been previously cooled in an ice bath. The mixture becomes very very thick. Added 250 ml of toluene and heated to 45°C in a water bath for 45 minutes. The reaction mixture was treated with 100 ml of saturated aqueous sodium thiosulfate solution. The organic layer was separated and the aqueous phase was extracted in the same fashion with ethyl acetate twice more. The combined organic phase was washed with brine, dried over sodium sulfate and evaporated in vacuo to afford 48.9 gm of the desired product (91 %). Mass Spectrum APCI m/e 190 (p+1).

### Preparation 7 (reference)

### 6-Methoxy-3-methyl-1,1a,3,7b-tetrahydro-3-aza-cyclopropa[a]naphthalen-2-one

A suspension of 7.0 gm (42.0 mmol) potassium iodide and 4.89 gm (38.0 mmol) trimethylsulfoxonium chloride in 30 ml of DMSO under nitrogen was treated portionwise with 1.69 gm (42.0 mmol) of 60% sodium hydride in mineral oil. The mixture was stirred for one hour and then treated with 2.0 gm (11.0 mmol) 6-Methoxy-1-methyl-1H-quinolin-2-one followed by stirring for 0.5 hours at ambient temperature and 4 days at 90°C. The reaction mixture was allowed to cool to ambient temperature before quenching into ice followed by extraction into ether. The ether layer was washed with brine, dried over sodium sulfate and evaporated. The residue was partitioned between acetonitrile and pentane. The acetonitrile layer was concentrated. The residue was partitioned between 1:1 hexane/ethyl acetate and 50% saturated (sat'd) brine solution. The organic layer was washed with saturated brine, dried over sodium sulfate and evaporated in vacuo to afford 1.6 gm of a mixture of desired product and starting material. The residue was purified by silica gel chromatography eluting with 7/3 hexane/ethyl acetate to afford 972 mg (44%). Mass spectrum APCl m/e 204 (p+1).

### Preparation 7A (reference)

### 6-Methoxy-3-methyl-1,1a,3,7b-tetrahydro-3-aza-cyclopropa[a]naphthalen-2-one

A suspension of 3.35 gm (15.1 mmol) trimethylsulfoxonium iodide in 10 ml of THF at 0° C under nitrogen was treated portionwise with 5.8 ml (14.6 mmol) of 2.5 M n-hexyllithium in heptane over 30 minutes. The mixture was stirred for 30 minutes at 0° C and then treated with 1.0 gm (5.02 mmol) 6-Methoxy-1-methyl-1H-quinolin-2-one followed by heating under reflux for 1.5 hours. The reaction mixture was allowed to cool to ambient temperature before quenching with 35 ml of water followed by extraction into ethyl acetate. The organic layer was washed with brine, dried over sodium sulfate and evaporated to afford 1.1 gm of desired product. Mass spectrum APCl m/e 204 (p+1).

### Preparation 8 (reference)

### 6-Methoxy-3-methyl-2-oxo-1a,2,3,7b-tetrahydro-1H-3-aza-cyclopropa[a]naphthalene-5-carbaldehyde

To a flame dried round bottom flask equipped with a nitrogen cap was added 972 mg (4.8 mmol) 6-Methoxy-3-methyl-1,1a,3,7b-tetrahydro-3-aza-cyclopropa[a]naphthalen-2-one, 40 ml of trifluoroacetic acid and 806 mg (5.7 mmol) of hexamethylene tetraamine. The mixture was stirred under reflux for 1.5 hours and then cooled to ambient temperature. The reaction mixture was quenched in 200 ml ice and then treated with solid sodium carbonate until pH 9 was reached. The aqueous phase was extracted with chloroform (3x30ml). The combined organics were then washed with brine followed by drying over sodium sulfate and evaporation in vacuo. The crude residue was taken up in ethyl acetate whereupon crystals formed. Four crops totaling 373 mg were obtained. The mother liquors were purified by silica gel column chromatography eluting with 1/1 ethyl acetate / hexane to afford another 98 mg (total yield 42 %) of a white solid which was used directly in the following step. Mass spectrum APCI m/e 232 (p+1).

### Preparation 8A (reference)

### 6-Methoxy-3-methyl-2-oxo-1a,2,3,7b-tetrahydro-1H-3-aza-cyclopropa[a]naphthalene-5-carbaldehyde

To a flame dried round bottom flask equipped with a nitrogen cap was added 935 mg (4.6 mmol) 6-Methoxy-3-methyl-1,1a,3,7b-tetrahydro-3-aza-cyclopropa[a]naphthalen-2-one, and 40 ml of methylene chloride. The solution was cooled to 0°C and 46.1 ml (46.1 mmol) of 1.0 M titanium tetrachloride was added. Dichloromethytmethylether (2.09 ml (23.05 mmol) was added and the reaction mixture was stirred at room temperature overnight. The reaction mixture was quenched in 100 ml ice / 1N HCl. The aqueous phase was extracted with chloroform (3x30ml). The combined organics were then washed with saturated bicarbonate solution and then brine followed by drying over sodium sulfate and evaporation in vacuo. The crude residue was taken up in ethyl acetate whereupon crystals formed. Four crops totaling 902 mg were obtained. Mass spectrum APCI m/e 232 (p+1).

### Preparation 8B (reference)

### Separation of enantiomers: 6-Methoxy-3-methyl-2-oxo-1a,2,3,7b-tetrahydro-1H-3-aza-cyclopropa[a]naphthalene-5-carbaldehyde

To a flame dried round bottomed flask under nitrogen atmosphere was introduced 7.5 gm (32.5 mmol) 6-Methoxy-3-methyl-2-oxo-1a,2,3,7b-tetrahydro-1H-3-aza-cyclopropa[a]-naphthalene-5-carbaldehyde and 150 ml methanol. The solution was treated with a catalytic amount of toluenesulfonic acid and 4.26 ml (38.9 mmol) trimetylorthoformate. The reaction was stirred for 1.5 hours and was then treated with a small amount of solid sodium bicarbonate. The reaction mixture was diluted with chloroform that had been passed through a plug of neutral or basic alumina and filtered to clarify the solution. The filtrate was concentrated in vacuo to afford 8.0 gm. The acetal was separated by chiral HPLC chromatography on a Chiralpak AS 10cm X 50cm column eluting with 60:40 / heptane: ethanol at a flow rate of 200 ml/min. The desired isomer (4.13 gm) had a retention time of 4.5 min and was estimated to be 100% ee. This material was taken up in 100 chloroform and 5 drops of 1N HCl. The mixture was stirred at room temperature for 16 hours and then dried over sodium sulfate. The solvent was removed in vacuo to afford 3.6 gm of the aldehyde as a single enantiomer.

### Reference Example 3

### 6-Methoxy-3-methyl-5-[(-2-phenyl-piperidin-3-ylamino)-methyl]-1,1a,3,7b-tetrahydro-3-aza-cyclopropa[a]naphthalen-2-one

By a procedure similar to previous reference examples 1 and 2: 331 mg (1.88 mmole) cis-(2S,3S)-3-amino, 2-phenylpiperidine and 443 mg (1.92 mmole) 6-Methoxy-3-methyl-2-oxo-1a,2,3,7b-tetrahydro-1H-3-aza-cyclopropa[a]naphthalene-5-carbaldehyde were combined in 100 ml of toluene and heated under reflux for 4 hours over a Dean -Stark trap. The crude imine (MS APCI m/e = 390 p+1) solution was evaporated in vacuo and redissolved in 100 ml dichloroethane. The solution was treated with 528 mg (2.49 mmol) sodium triacetoxyborohydride and stirred at room temperature for 16 hours. The reaction mixture was washed with saturated aqueous bicarbonate solution followed by brine and then dried over sodium sulfate. The product dissolved in dichloroethane (MS, APCI m/e 392 (p+1) was treated with 392 µl (2.8 mmol) triethylamine and 541 mg (2.48 mmol) t-boc carbonate and the reaction mixture was stirred for 24 hours at ambient temperature. The reaction mixture was washed with saturated aqueous bicarbonate solution followed by brine and then dried over sodium sulfate. After evaporation, the crude residue was chromatographed on silica gel eluting with 98/2/1 methylene chloride, methanol, (CH₃OH), ammonium hydroxide (NH₄OH) to afford 780 mg of the desired product with protection of the N-1 nitrogen with the tertiary butoxycarbonyl group (t-BOC) in 84 % yield (MS, APCI m/e 492 (p+1). Diastereomeric separation was completed on a Chirlpak AD column eluting with 8/2 hex/IPA. The retention times were 7.0 minutes and 10.8 minutes respectively. Removal of the t-BOC protecting group was achieved by exposure of each substrate to ten molar equivalents of trifluoroacetic acid in dichloroethane at 65°C for 5 hours followed by washing with saturated carbonate solution and brine. Chromatography on silica gel eluting with 95:5 methylene chloride : methanol containing ammonium hydroxide afforded the above titled products in 62 and 86 % yields. (MS, APCI m/e 392 (p+1).

### Reference Example 3A

### 6-Methoxy-3-methyl-5-[(-2-phenyl-piperidin-3-ylamino)-methyl]-1,1a,3,7b-tetrahydro-3-aza-cyclopropa[a]naphthalen-2-one

By a procedure similar to previous examples 1 and 2: 331 mg (1.88 mmole) cis-(2S,3S)-3-amino, 2-phenylpiperidine and 443 mg (1.92 mmole) enantiomerically pure 6-Methoxy-3-methyl-2-oxo-1a,2,3,7b-tetrahydro-1H-3-aza-cyclopropa[a]naphthalene-5-carbaldehyde were combined in 100 ml of toluene and heated under reflux for 4 hours over a Dean -Stark trap. The crude imine (MS APCI m/e = 390 p+1) solution was evaporated in vacuo and redissolved in 100 ml dichloroethane. The solution was treated with 528 mg (2.49 mmol) sodium triacetoxyborohydride and stirred at room temperature for 16 hours. The reaction mixture was washed with saturated aqueous bicarbonate solution followed by brine and then dried over sodium sulfate. Chromatography on silica gel eluting with 95:5 methylene chloride : methanol containing ammonium hydroxide afforded the above titled product (550 mg) in 75 % yield. (MS, APCl m/e 392 (p+1).

### Example 1

### 6-Methoxy-1-methyl-7-[(2(S)-phenyl-piperidin-3(S)-ylamino)-methyl]-3,4-dihydro-1H-[1,8]naphthyridin-2-one:

### Step 1

### 3-Methoxy-6-nitro-2-vinyl-pyridine.

To a solution of 1.0 g (4.3 mmol) 2-bromo-3-methoxy-6-nitropyridine (J. Lombardino, *J. Med. Chem.* **1981**, *24*, 39-42) in 25 ml of toluene was added 1.9 ml (6.4 mmol) of tri-n-butyl-vinyltin and a catalytic amount of bis(triphenylphosphine-palladium(II) chloride, and the reaction was refluxed under a nitrogen atmosphere for 2 hours. The reaction mixture was cooled to room temperature and the solvent evaporated. The residue was chromatographed on silica using chloroform as the elutant. Appropriate fractions were combined to yield 0.63 g of 3-methoxy-6-nitro-2-vinyl-pyridine as an oil. TLC: Rf = 0.5 (5:1 hexane/ethyl acetate). ¹H NMR (CDCl₃) δ 8.15 (d, 1H), 7.35 (d, 1H), 7.10 (m, 1H), 6.55 (d, 1H), 5.65 (d, 1H), 4.0 (s, 3H). Mass spectrum: m/e =181.1 (p+1).

### Step 2

### 3-Methoxy-6-nitro-pyridine-2-carbaldehyde.

A solution of 0.63 g (3.5 mmol) 3-methoxy-6-nitro-2-vinyl-pyridine in 50 ml of CH₂Cl₂ and 10 ml of methanol was cooled to -70° C. Ozone was bubbled into the solution until a blue color persisted. The mixture was stirred for 60 minutes at -70°C and then quenched with excess dimethyl sulfide. The reaction was warmed to room temperature and the solvent evaporated. The residue was dissolved in ethyl acetate and water. The pH of the solution was adjusted to 2.0 with 1N HCl and the mixture stirred for 30 minutes. The pH of the solution was then adjusted to 8.0 with 1 N NaOH. The mixture was extracted with excess ethyl acetate. The ethyl acetate extracts were combined, dried (Na₂SO₄) and evaporated to yield 0.6 g of 3-methoxy-6-nitro-pyridine-2-carbaldehyde as a tan solid. TLC: Rf = 0.2 (1:1 ethyl acetate/hexane). ¹H NMR (CDCl₃) δ 10.1 (s, 1H), 8.5 (d, 1H), 7.85 (d, 1H), 4.15 (s, 3H).

### Step 3

### 2-[1,3]Dioxolan-2-yl-3-methoxy-6-nitro-pyridine.

A mixture of 0.55g (0.3 mmol) 3-methoxy-6-nitro-pyridine-2-carbaldehyde, 0.8 ml (15 mmol) of ethylene glycol, and 10 mg (catalytic amount) of p-toluene sulfonic acid was refluxed in 50 ml of toluene using a Dean-Stark trap to trap water. After 90 minutes, the reaction mixture was cooled to room temperature and the solvent evaporated. The residue was chromatographed on silica using 5:1 (chloroform/ethyl acetate) as the elutant. Appropriate fractions were combined and evaporated to yield 0.6 g of 2-[1,3]dioxolan-2-yl-3-methoxy-6-nitro-pyridine as an oil. TLC: Rf = 0.9 (1:1 chloroform/ethyl acetate). ¹H NMR (CDCl₃) δ 8.25 (d, 1H), 7.40 (d, 1H), 6.30 (s, 1H), 4.30 (m, 2H), 4.10 (m, 2H), 4.0 (s, 3H). Mass Spectrum: m/e = 227.2 (p+1).

### Step 4

### 3-Benzenesulfonylmethyl-6-[1,3]dioxolan-2-yl-5-methoxy-2-nitro-pyridine.

A mixture of 0.6 g (2.6 mmol) 2-[1,3]dioxolan-2-yl-3-methoxy-6-nitro-pyridine, 0.55g (2.9 mmol) of chloromethylphenylsulfone and 2.9 ml (2.9 mmol) of potassium t-butoxide (1M solution in THF) in 5 ml of DMF were combined at 5° C and stirred at ambient temperature for 18 hours. The reaction mixture was quenched with 25 ml of water and extracted with ethyl acetate. The ethyl acetate extracts were combined, dried (Na₂SO₄), and evaporated. The residue was chromatographed on silica using 5:1 chloroform/ethyl acetate as the elutant. Appropriate fractions were combined and evaporated to yield 0.28 g of 3-benzenesulfonylmethyl-6-[1,3]dioxolan-2-yl-5-methoxy-2-nitro-pyridine as an oil. TLC: Rf = 0.4 (1:1 chloroform/ ethyl acetate). ¹H NMR (CDCl₃) δ 7.7 (m, 3H), 7.55 (m, 2H), 7.42 (s, 1H), 6.30 (s, 1H), 4.90 (s, 2H), 4.30 (m, 2H), 4.10 (m, 2H), 4.02 (s, 3H).). Mass Spectrum: m/e = 381.0 (p + 1).

### Step 5

### 3-(6-[1,3]Dioxolan-2-yl-5-methoxy-2-nitro-pyridin-3-yl)-acrylic acid ethyl ester.

A mixture of 0.28 g (0.73 mmol) 3-benzenesulfonylmethyl-6-[1,3]dioxolan-2-yl-5-methoxy-2-nitro-pyridine and 0.8 ml (0.73 mmol) of ethyl bromoacetate was dissolved in 10 ml of ethanol. To this solution was added 0.5 ml of potassium t-butoxide (1M in t-butyl alcohol) and the reaction mixture was stirred at room temperature for 1 hour. The solvent was evaporated and the residue was added to 5 ml of water. The suspension was extracted with ethyl acetate. The ethyl acetate extract was dried (Na₂SO₄) and evaporated to yield approximately 0.3 g of an oil. Mass spectrum: m/e = 325.1 (p + 1). This material was used directly in step 6.

### Step 6

### 7-[1,3]Dioxolan-2-yl-6-methoxy-3,4-dihydro-1H-[1,8]naphthyridin-2-one.

The oil isolated in step 5 was dissolved in 30 ml of ethanol and hydrogenated for 90 minutes at 50 PSI using 10% Pd/C as the catalyst. The reaction mixture was filtered and evaporated to yield the intermediate 3-(2-amino-6-[1,3]dioxolan-2-yl-5-methoxy-pyridin-3-yl)-propionic acid ethyl ester as a dark oil. Mass Spectrum: m/e = 297.1 (p + 1). This material was dissolved in 10 ml of toluene and heated to reflux for 4 hours. The solution was cooled to room temperature and evaporated to yield 0.1g of 7-[1,3]dioxolan-2-yl-6-methoxy-3,4-dihydro-1H-[1,8]naphthyridin-2-one as a dark oil. Mass spectrum: m/e = 251 (p + 1). ¹H NMR (CDCl₃) δ 7.10 (s, 1H), 6.10 (s, 1H), 4.30 (m, 2H), 4.10 (m, 2H), 3.90 (s, 3H), 2.90 (m, 2H), 2.75 (m, 2H). This material was used without further purification in step 7.

### Step 7

### 3-Methoxy-8-methyl-7-oxo-5,6,7,8-tetrahydro-[1,8]naphthyridine-2-carbaldehyde.

To a solution of 0.1 g (0.4 mmol) 7-[1,3]dioxolan-2-yl-6-methoxy-3,4-dihydro-1H-[1,8]naphthyridin-2-one in 1 ml of DMF cooled to 5°C was added 0.5 ml (0.5 mmol) of potassium t-butoxide (1M solution in THF). The reaction mixture was stirred fo 30 minutes. To this mixture was added 0.06 ml (1 mmol) of methyl iodide and the reaction stirred at ambient temperature for 2 hours. The reaction mixture was diluted with 5 ml of water and extracted with ethyl acetate. The ethyl acetate extract was dried (Na₂SO₄) and evaporated to yield intermediate 7-[1,3]dioxolan-2-yl-6-methoxy-1-methyl-3,4-dihydro-1H-[1,8]naphthyridin-2-one as a dark oil. Mass spectrum: m/e = 205.1 (p + 1). This material was dissolved in 5 ml of acetone containing 0.1 g of para-toluenesulfonic acid and refluxed for 2 hours. The reaction mixture was evaporated and the residue triturated with saturated NaHCO₃. This mixture was extracted with ethyl acetate. The ethyl acetate extract was dried (Na₂SO₄) and evaporated and the residue chromatographed on silica using 3:1 chloroform/ethyl acetate as the elutant. Appropriate fractions were combined to yield 12 mg of 3-methoxy-8-methyl-7-oxo-5,6,7,8-tetrahydro-[1,8]naphthyridine-2-carbaidehyde as an oil. Mass spectrum: m/e = 221.1 (p + 1). ¹H NMR (CDCl₃) δ 10.1 (s, 1H), 7.25 (s, 1H), 3.95 (s, 3H), 3.50 (s, 3H), 3.0 (m, 2H), 2.90 (m, 2H).

### Step 8

### 6-Methoxy-1-methyl-7-[(2(S)-phenyl-piperidin-3(S)-ylamino)-methyl]-3,4-dihydro-1H-[1,8]naphthyridin-2-one.

The 3-methoxy-8-methyl-7-oxo-5,6,7,8-tetrahydro-[1,8]naphthyridine-2-carbaldehyde prepared in step 7 (12 mg; 0.05 mmol) was dissolved in 3 ml of dichloroethane. To this was added 25 mg (0.1 mmol) of 2-(S)-phenyl-piperidin-3(S)-ylamine and 0.014 ml (0.1 mmol) of triethylamine, and the mixture was stirred for 60 minutes at room temperature. To this mixture was added 32 mg (0.15 mmol) of sodium triacetoxyborohydride, and the reaction mixture was stirred at room temperature for 18 hours. The reaction was quenched with water and stirred at room temperature for 30 minutes. The pH of the mixture was adjusted to 2 with 1 N HCl and extracted with ethyl acetate. The pH of the water layer was adjusted to 7.0 with sodium bicarbonate and extracted with ethyl acetate. The pH = 7 ethyl acetate extracts were combined, dried(Na₂SO₄) and evaporated to yield 7 mg of a yellow amorphous solid. ¹H NMR (CDCl₃) δ 7.2-7.4 (m, 5H), 6.85 (s, 1H), 3.95 (s, 1H), 3.85 (s, 3H), 3.62 (d,d 2H), 3.30 (d. 1H), 3.10 (s, 3H), 3.0 (s, 1H), 2.80 (m, 2H), 2.60 (m, 2H), 2.2-2.4 (m, 3H), 1.9 (m, 2H), 1.7 (m, 2H), 1.4 (m, 1H). Mass spectrum: m/e = 381.1 (p + 1). TLC: Rf = 0.4; (5:1 chloroform/methanol).

### Example 2

### 6-Methoxy-1-methyl-7-[(2(S)-phenyl-piperidin-3(S)-ylamino)-methyl]-3,4-dihydro-1H-[1,5]naphthyridin-2-one:

### Step 1

### 6-Methoxy-3,4-dihydro-1H-[1,5]naphthyridin-2-one.

A 2.2g (8.7 mmol) solution of 3-(6-methoxy-3-nitro-pyridin-2-yl)-acrylic acid ethyl ester (M.Makosza, A. Tryula *Synthesis,* **1987,** 1142-1144) in 50 ml of ethanol containing a catalytic amount of 10% Pd/C was hydrogenated at 50 PSI for 2 hours. The reaction was filtered and the solvent evaporated to afford crude intermediate 3-(3-amino-6-methoxypyridin-2-yl)-propionic acid ethyl ester. This material was disolved in 5 ml of acetic acid and heated on a steam bath for 30 minutes. The solution was cooled to room temperature and the solvent evaporated. The residue was dissolved in 25 ml of ethyl acetate and washed with saturated NaHCO₃. The ethyl acetate solution was dried (Na₂SO₄) and evaporated to yield a brown solid residue. This material was chromatographed on silica using 5:1 chloroform/ethyl acetate as the elutant. Appropriate fractions were combined and evaporated to yield 1.2 g of 6-methoxy-3,4-dihydro-1H-[1,5]naphthyridin-2-one. TLC: Rf = 0.55 (5:1 ethyl acetate: hexane). ¹H NMR (CDCl₃) δ 8.6 (s, 1H), 7.1 (d, 1H), 6.6 (d, 1H), 3.9 (s, 3H), 3.05 (m, 2H), 2.7 (m, 2H). Mass Spectrum: m/e = 179.2 (p +1).

### Step 2

### 6-Methoxy-1-methyl-3,4-dihydro-1H-[1,5]naphthyridin-2-one.

A solution of 1.2 g (6.7 m mol) 6-methoxy-3,4-dihydro-1H-[1,5]naphthyridin-2-one in 15 ml of DMF was cooled to 5°C and to this solution was slowly added 6.7 ml (6.7 mmol) of potassium t-butoxide (1M solution in THF). The mixture was stirred for 10 minutes followed by addition of 0.46 ml (7.4 mmol) methyl iodide. After addition was complete, the reaction mixture was stirred for 1 hour at 5°C. The reaction mixture was poured into saturated NaCl. The mixture was extracted with ethyl acetate. The ethyl acetate extracts were dried (Na₂SO₄) and evaporated. The residue was chromatographed on silica using 5:1 chloroform/ethyl acetate as the elutant. Appropriate fractions were combined and evaporated to yield 0.83 g of 6-methoxy-1-methyl-3,4-dihydro-1H-[1,5]naphthyridin-2-one. TLC Rf = 0.6 (1:10 ethyl acetate/chloroform). ¹H NMR (CDCl₃) δ 7.2 (d, 1H), 6.6 (d, 1H), 3.9 (s, 3H), 3.3 (s, 3H), 3.0 (m, 2H), 2.7 (m, 2H). Mass Spectrum: m/e = 193.2 (p + 1).

### Step 3

### 7-Bromo-6-methoxy-1-methyl-3,4-dihydro-1H-[1,5]naphthyridin-2-one.

To a solution of 0.6 g (3.1 mmol) 6-methoxy-1-methyl-3.4-dihydro-1H-[1,5]naphthyridin-2-one in 8 ml of acetic acid was added 8 ml of water. To this mixture was added 0.32 ml (6.2 mmol) of bromine. The reaction mixture was heated to 60° C for 1 hour. The reaction mixture was cooled to room temperature and poured into 50 ml of water. The suspension was extracted with ethyl acetate. The ethyl acetate extracts were dried (Na₂SO₄) and evaporated. The residue was chromatographed on silica using chloroform as the elutant. Appropriate fractions were combined and evaporated to yield 0.69 g of 7-bromo-6-methoxy-1-methyl-3,4-dihydro-1H-[1,5]naphthyridin-2-one. TLC Rf = 0.8 (10:1 ethyl acetate/chloroform). ¹H NMR (CDCl₃) δ 7.42 (s, 1H). 4.0 (s, 3H), 3.3 (s, 3H), 3.0 (m, 2H), 2.7 (m, 2H). Mass Spectrum: m/e = 271.2, 273.2 (p + 1,3).

### Step 4

### 6-Methoxy-1-methyl-7-vinyl-3,4-dihydro-1H-[1,5]naphthyridin-2-one.

A mixture of 690 mg (2.5 mmol) of 7-bromo-6-methoxy-1-methyl-3,4-dihydro-1H-[1,5]naphthyridin-2-one, 0.8 ml (2.7 mmol) of tri-n-butyl-vinyltin and 100 mg of bis(triphenylphosphine-palladium(II) chloride in 20 ml of dioxane was heated under nitrogen to 100° C for 6 hours. The reaction mixture was cooled to room temperature and diluted with water and ethyl acetate. The ethyl acetate solution was washed with water several times. To this ethyl acetate solution was added 1 ml of saturated K F, and the mixture filtered. The filtrate was dried, evaporated, and the residue chromatographed on silica using 1:5 ethyl acetate/hexanes as the elutant. Appropriate fractions were combined and evaporated to afford 200 mg of 6-methoxy-1-methyl-7-vinyl-3,4-dihydro-1H-[1,5]naphthyridin-2-one. ¹H NMR (COCl₃) δ 7.28 (s, 1 H), 6.90 (d,d 1H), 5.75 (d, 1H), 5.35 (d, 1H), 3.95 (s, 3H), 3.26 (s, 3H), 3.0 (m, 2H), 2.7 (m, 2H) Mass spectrum: m/e = 219.3 (p + 1).

### Step 5

### 2-Methoxy-5-methyl-6-oxo-5,6,7,8-tetrahydro-[1,5]naphthyridine-3-carbaldehyde.

A 1.1 g (5.0 mmol) solution of 6-methoxy-1-methyl-7-vinyl-3,4-dihydro-1H-[1,5]naphthyridin-2-one in 50 ml of methylene chloride and 10 ml of methanol was cooled to - 70° C. Ozone (via an ozone generator) was bubbled into the solution until a blue color was achieved. The reaction mixture was stirred at -70° C for 30 minutes and quenched with 4.0 ml of dimethyl sulfide. The reaction mixture was stirred for 18 hours at room temperature. The reaction mixture was poured into 50 ml of water. The organic layer was dried (Na₂SO₄) and evaporated. This residue was chromatographed on silica using 10:1 chloroform/ethyl acetate as the elutant. Appropriate fractions were combined to yield0.52 g of 2-methoxy-5-methyl-6-oxo-5,6,7,8-tetrahydro-[1,5]naphthyridine-3-carbaldehyde. TLC Rf = 0.4 (1:1 ethyl acetate: hexane). ¹H NMR (CDCl₃) δ 10.18 (s, 1H), 7.7 (s, 1H), 4.05 (s, 3H), 3.3 (s, 3H), 3.0 (m, 2H), 2.7 (m, 2H). Mass spectrum: m/e = 221.1, (p + 1).

### Step 6

### 6-Methoxy-1-methyl-7-[(2(S)-phenyl-piperidin-3(S)-ylamino)-methyl]-3,4-dihydro-1H-[1,5]naphthyridin-2-one.

The 2-methoxy-5-methyl-6-oxo-5,6,7,8-tetrahydro-[1,5]naphthyridine-3-carbaldehyde prepared in step 5 (0.35g; 1.4 mmol) was dissolved in 20 ml of dichloroethane. To this was added 250 mg (1.1 mmol) of 2-(S)-phenyl-piperidin-3(S)-ylamine and 0.4 ml (2.8 mmol) of triethylamine, and the mixture was stirred for 60 minutes at room temperature. To this mixture was added 0.7g (3.3 mmol) of sodium triacetoxyborohydride, and the reaction mixture was stirred at room temperature for 18 hours. The reaction was quenched with water and stirred at room temperature for 30 minutes. The pH of the mixture was adjusted to 8.5 with Na₂CO₃. The organic layer was separated from the water layer. An additional 20 ml of water was added to the organic layer. The pH of the mixture was adjusted to 2 with 1N HCl and extracted with ethyl acetate. The pH of the water layer was adjusted to 7.5 with sodium bicarbonate and extracted with ethyl acetate. The pH = 7.5 ethyl acetate extracts were combined, dried (Na₂SO₄), and evaporated to yield 300 mg of 6-methoxy-1-methyl-7-[(2(S)-phenyl-piperidin-3(S)-ylamino)-methyl]-3,4-dihydro-1H-[1,5]naphthyridin-2-one. ¹H NMR (CDCl₃) δ 7.2-7.4 (m, 5H), 6.85 (s, 1H), 3.90 (s, 1H), 3.85 (s, 3H), 3.60,3.38 (d,d 2H), 3.30 (d. 1H), 3.10 (s, 3H), 3.0 (s, 1H), 2.90 (m, 2H), 2.80 (m, 2H), 2.66 (m, 2H), 2.0-2.2 (m, 3H), 1.9 (m, 1H), 1.6 (m, 1H), 1.45 (m, 1H). Mass spectrum: m/e = 381.1 (p + 1). TLC: Rf = 0.5; (5:1 chloroform/methanol).

### Example 3

### 6-Methoxy-1-methyl-7-[(6(S)-methyl-2(S)-phenyl-piperidin-3(S)-ylamino)-methyl]-3,4-dihydro-1H-[1,5]naphthyridin-2-one:

By a procedure similar to the previous example 2: starting with 6(S)-methyt-2(S)-phenyl-piperidin-3(S)-ylamine (Scheme J) and 2-methoxy-5-methyl-6-oxo-5,6,7,8-tetrahydro-[1.5]naphthyridine-3-carbaldehyde (Example 2, step 5) and using the above coupling procedure (Example 2, step 6) 6-methoxy-1-methyl-7-[(6(S)-methyl-2(S)-phenyl-piperidin-3(S)-ylamino)-methyl]-3,4-dihydro-1H-[1,5]naphthyridin-2-one was obtained. ¹H NMR (CDCl₃) δ 7.2-7.4 (m, 5H), 7.0 (s, 1H), 4.28 (s, 1H), 3.70 (s, 3H), 3.60,3.42 (d,d 2H) 3..45 (m, 1H), 3.15 (s, 3H), 2.90 (m, 3H), 2.60 (m, 2H), 2.0-2.2 (m, 3H), 1.85 (m, 3H), 1.1 (d, 3H). Mass spectrum: m/e = 395.2 (p + 1). TLC: Rf = 0.55; (5:1 chloroform/methanol). [47595-203, 276]

### Example 4

### 7-[(6(S)-Ethyl-2(S)-phenyl-piperidin-3(S)-ylamino)-methyl]-6-methoxy-1-methyl-3,4-dihydro-1H-[1,5]naphthyridin-2-one:

By a procedure similar to the previous example 2: starting with *cis*-2-phenyl-3-amino-*trans*-6-ethyl-piperidine (scheme J) and 2-methoxy-5-methyl-6-oxo-5,6,7,8-tetrahydro-[1,5]naphthyridine-3-carbaldehyde (Example 2, step 5) and using the above coupling procedure (Example 2, step 6) 7-[(6-ethyl-2-phenyl-piperidin-3-ylamino)-methyl]-6-methoxy-1-methyl-3,4-dihydro-1H-[1,5]naphthyridin-2-one was obtained. Using a CHIRALPAK AD (10X50 cm) and a mobil phase of 95:5 heptane/ethanol containing 0.025% diethyl amine (flow rate = 275 ml/min) 7-[(6(S)-ethyl-2(S)-phenyl-piperidin-3(S)-ylamino)-methyl]-6-methoxy-1-methyl-3,4-dihydro-1H-[1,5]naphthyridin-2-one (retention time 13.545 min) was isolated as a pure enantiomer. ¹H NMR (CDCl₃) δ 7.40 (m, 2H), 7.35 (m, 2H), 7.2 (m, 1H), 4.20 (s, 1H), 3.75 (s, 3H), 2.82, 2.42 (d,d, 2H), 3.17 (s, 3H), 3.08 (m, 1H), 2.90 (m, 2H), 2.85 (m, 1H), 2.65 (m, 2H), 1.8-2.2 (m, 6H), 1.7 (m, 1H), 1.55 (m, 1H), 1.38 (m, 1H), 0.9 (t, 3H). Mass spectrum: m/e = 409.2 (p + 1). The corresponding 7-[(6(R)-ethyl-2(R)-phenyl-piperidin-3(R)-ylamino)-methyl]-6-methoxy-1-methyl-3,4-dihydro-1H-[1,5]naphthyridin-2-one enantiomer (retention time 14.539 min) was also isolated. NMR and mass spectrum was identical to the above spectra.

### Example 5

### 6-Methoxy-1-methyl-7-[(2-phenyl-6-propyl-piperidin-3-ylamino)-methyl]-3,4-dihydro-1H-[1,5]naphthyridin-2-one:

By a procedure similar to the previous example 2: starting with *cis*-2-phenyl-3-amino-*trans*-6-(n-propyl)-piperidine (Scheme J) and 2-methoxy-5-methyl-6-oxo-5,6,7,8-tetrahydro-[1,5]naphthyridine-3-carbaldehyde (Example. 2, step 5) and using the above coupling procedure (Example 2, step 6) 7-[(6-ethyl-2-phenyl-piperidin-3-ylamino)-methyl]-6-methoxy-1-methyl-3,4-dihydro-1H-[1,5]naphthyridin-2-one was obtained. Using a CHIRALPAK AD (10X50 cm) and a mobil phase of 9 5:5 heptane/ethanol containing 0.025% diethylamine (flow rate = 275 ml/min) 6-methoxy-1-methyl-7-[(2(S)-phenyl-6(S)-propyl-piperidin-3(S)-ylamino)-methyl]-3,4-dihydro-1H-[1,5]naphthyridin-2-one (retention time 11.479 min) was isolated as a pure enantiomer. ¹H NMR (CDCl₃) δ 7.40 (m, 2H), 7.30 (m, 2H), 7.22 (m, 1H), 6.95 (s, 1H), 4.20 (s, 1H), 3.72 (s, 3H), 3.80, 3.62 (d,d, 2H), 3.20 (m, 1H), 3.10 (s, 3H), 2.90 (m, 2H), 2.85 (m, 1H), 2.62 (m, 2H), 2.10 (m, 2H), 1.90 (m, 3H), 1.70 (m, 1H), 1.42 (m, 1H), 1.30 (m, 3H), 0.9 (t, 3H). Mass spectrum: m/e = 423.2 (p + 1). The corresponding enantiomer 6-methoxy-1-methyl-7-[(2(R)-phenyl-6(R)-propyl-piperidin-3(R)-ylamino)-methyl]-3,4-dihydro-1H-[1,5]naphthyridin-2-one (retention time 11.592 min) was also isolated. NMR and mass spectrum was identical to the above spectra.

### Example 6

### 7-[((2S, 3S, 6S)-6-Isopropyl-2-phenyl-piperidin-3-ylamino)-methyl]-6-methoxy-1-methyl-3,4-dihydro-1H-[1,5]naphthyridin-2-one

By a procedure similar to Reference Examples 3 and 3A: prepared through the reaction of 6-isopropyl-2-phenyl-piperidin-3-ylamine with 2-Methoxy-5-methyl-6-oxo-5,6,7,8-tetrahydro-[1,5]naphthyridine-3-carbaldehyde. Mass spectrum calcd for C25 H34 N4 O2 (M+1) 423; found 423.

## Claims

1. A compound of the formula 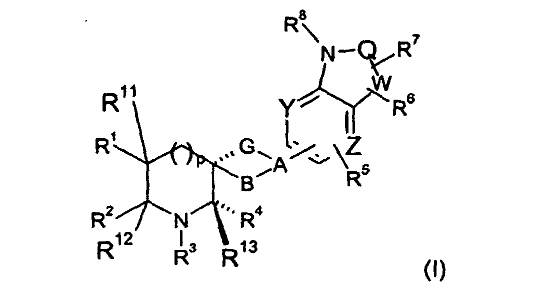
wherein Q is C=S or C=O;
A is CH, CH₂, C(C₁-C₆)alkyl, CH(C₁-C₆)alkyl, C(CF₃) or CH(CF₃), with the proviso that when B is present, A must be either CH, C(C₁-C₆)alkyl or C(CF₃); B is absent or is methylene or ethylene;
Y is N and Z is CH, or Y is CH and Z is N;
G is NH(CH₂)_{q}, S(CH₂)_{q} or O(CH₂)_{q}, wherein q is zero or one;
with the proviso that when q is zero, G is NH, S or O;
W is a one carbon linking group (i.e., methylene) or a saturated or unsaturated two or three carbon linking group, wherein each of the foregoing W groups can optionally be substituted with one substituent R⁷ or two substituents R⁷ and R⁶, or W is a one carbon linking group that forms, together with a 2, 3, 4 or 5 carbon chain, a 3, 4, 5 or 6 membered spiro ring, respectively;
or W is a saturated two carbon chain linking group that forms, together with a separate 1, 2 or 3 carbon chain, a fused 3, 4 or 5 membered ring, respectively;
or W is a saturated two carbon chain linking group, wherein one of the two carbons in the chain forms, together with a separate 2, 3, 4 or 5 carbon chain, a 3, 4, 5 or 6 membered spiro ring, respectively;
p is zero, one or two;
R³ is selected from hydrogen, COR⁹, CO₂R⁹, optionally substituted phenyl, optionally substituted heterocyclic rings, and optionally substituted (C₁-C₈)alkyl wherein one of the CH₂ groups of said (C₁-C₈) alkyl may optionally be replaced with a sulfur, oxygen or carbonyl group and wherein said (C₁-C₈)alkyl can optionally be substituted with from one to three substituents, preferably with zero substituents or one substituent, independently selected from hydroxy, oxo, phenyl-(C₁-C₃)alkoxy, phenyl, cyano, halo, optionally substituted heterocyclic rings, NR⁹COR¹⁰, NR⁹CO₂R¹⁰, CONR⁹R¹⁰, COR⁹, CO₂R⁹, NR⁹R¹⁰, and (C₁-C₆)alkoxy optionally substituted with from one to seven fluorine atoms, preferably with from zero to three fluorine atoms;
and wherein the heterocyclic rings of R³ and the heterocyclic ring substituents on the alkyl groups of R³ are selected, independently, from 3 to 7 membered saturated or unsaturated monocyclic rings containing from 1 to 4 ring heteroatoms, and 8 to 12 membered saturated or unsaturated bicyclic rings containing from 1 to 4 ring heteroatoms, wherein said heteroatoms are selected, independently, from oxygen, nitrogen and sulfur, with the proviso that there can not be two adjacent ring oxygen atoms or two adjacent ring sulfur atoms in either the monocyclic or bicyclic heterocyclic rings, and with the proviso that heterocyclic rings formed from NR⁹R¹⁰ or CONR⁹R¹⁰ must contain at least one nitrogen atom;
and wherein the heterocyclic rings of R³ and the heterocyclic ring substituents on the alkyl groups of R³ can optionally be substituted with one or more substituents, preferably with zero, one or two substituents, independently selected from oxo, hydroxy, thioxo, halo, cyano, phenyl, (CH₂)ₘNR⁹R¹⁰, NR⁹COR¹⁰, (CH₂)ₘOR⁹, wherein m is zero, one or two, and (C₁-C₆)alkyl optionally substituted with one or more substituents, preferably with from zero to two substituents, independently selected from halo, CF₃, methoxy and phenyl;
and wherein the phenyl groups of R³ and the phenyl substituents in the alkyl groups of R³ can optionally be substituted with one or more substitutents, preferably with from zero to two substituents, independently selected from the group consisting of halo, cyano, nitro, CF₃, (CH₂)ₘNR⁹R¹⁰, wherein m is zero, one or two, NR⁹COR¹⁰, NR⁹CO₂R¹⁰, CONR⁹R¹⁰, CO₂NR⁹R¹⁰, COR⁹, CO₂R⁹, (C₁-C₆)alkyl optionally substituted with from one to seven fluorine atoms, preferably with from zero to three fluorine atoms, (C₁-C₆)alkoxy optionally substituted with from one to seven fluorine atoms, preferably with from zero to three fluorine atoms, and (C₂-C₆)alkenyl optionally substituted with from one to seven fluorine atoms, preferably with from zero to three fluorine atoms;
each of R¹, R², R¹¹, R¹² and R¹³ are selected, independently, from hydrogen and (C₁-C₆)alkyl optionally substituted with one or more substituents, preferably with zero, one or two substituents, that are selected, independently, from hydroxy, oxo, (C₁-C₆)alkoxy and cyano;
or R¹ and R², together with the carbon atoms to which they are attached, or R² and R³, together with the carbon and nitrogen to which they are attached, respectively, form a 5 or 6 membered saturated heterocyclic ring containing one or two heteroatoms that are selected, independently, from nitrogen, oxygen and sulfur, with the proviso that said ring can not contain two a djacent oxygen atoms or two adjacent sulfur atoms; or R¹ and R², together with the carbons to which they are attached, form a 5 or 6 membered, saturated or unsaturated carbocyclic ring, and wherein said heterocyclic and carbocyclic rings formed by R¹ and R² or by R² and R³ can be substituted with one or more substituents, preferably with zero substituents or one substituent, independently selected from halo, oxo, NR⁹R¹⁰, (C₁-C₆)alkyl optionally substituted with from one to seven fluorine atoms, preferably with from zero to three fluorine atoms, and (C₁-C₆)alkoxy optionally substituted with from one to seven fluorine atoms, preferably with from zero to three fluorine atoms;
or R¹² and R¹³, together with the carbon atoms to which they are attached, form a 5 or 6 membered saturated heterocyclic ring containing one or two heteroatoms that are selected, independently, from nitrogen, oxygen and sulfur, with the proviso that said ring can not contain two adjacent oxygen atoms or two adjacent sulfur atoms, or R¹² and R¹³, together with the carbons to which they are attached, form a 5 or 6 membered, saturated or unsaturated carbocyclic ring, and wherein said heterocyclic and carbocyclic rings formed by R¹² and R¹³ can be substituted with one or more substituents, preferably with zero substituents or one substituent, independently selected from NR⁹R¹⁰, halo, phenyl-S-, phenyl-SO-, phenyl-SO₂-, oxo, (C₁₋C₆)alkoxy optionally substituted with from one to seven fluorine atoms, preferably with from zero to three fluorine atoms, and (C₁-C₆)alkyl optionally substituted with from one to seven fluorine atoms, preferably with from zero to three fluorine atoms:
with the proviso that no more than one of R¹ and R², R² and R³, and R¹² and R¹³ can form a ring;
R⁴ is selected from phenyl, 2-, 3- or 4-pyridyl, 2- or 3-thienyl, and pyrimidyl, wherein R⁴ can be optionally substituted with one or more substituents, preferably with zero or one substituent, selected, independently, from halo, (C₁-C₆)alkyl optionally substituted with from one to seven fluorine atoms, preferably with from zero to three fluorine atoms, (C₁-C₆)alkoxy optionally substituted with from one to seven fluorine atoms, preferably with from zero to three fluorine atoms, and (C₂-C₆) alkenyl optionally substituted with from one to seven fluorine atoms, preferably with from zero to three fluorine atoms;
R⁵ is selected from hydrogen, -SO(C₁-C₆)alkyl, -SO₂-(C₁₋C₆)alkyl, -SO-aryl, -SO₂-aryl, CF₃, phenyl, phenyl-(C₁-C₂)alkyl, hydroxy, aryloxy, heteroaryloxy, pyridyl, tetrazolyl, oxazolyl, thiazolyl, (C₁-C₆)alkoxy optionally substituted with from one to seven fluorine atoms, preferably with from zero to three fluorine atoms, (C₁-C₆)alkyl optionally substituted with from one to seven fluorine atoms, preferably with from zero to three fluorine atoms, and (C₁-C₆)alkyl substituted with one or more substituents, preferably with from zero to two substituents selected, independently, from hydroxy, oxo, (C₁-C₆)alkoxy, phenyl-(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
R⁶ and R⁷ are selected, independently, from -SO(C₁-C₆)alkyl, -SO₂-(C₁-C₆)alkyl, -SO-aryl, -SO₂-aryl, CF₃, halo, phenyl, phenyl-(C₁-C₂)alkyl, hydroxy, aryloxy, heteroaryloxy, pyridyl, tetrazolyl, oxazolyl, thiazolyl, (C₁-C₆)alkoxy optionally substituted with from one to seven fluorine atoms, preferably with from zero to three fluorine atoms, (C₁-C₆)alkyl optionally substituted with from one to seven fluorine atoms, preferably with from zero to three fluorine atoms, and (C₁-C₆)alkyl substituted with one or more substituents, preferably with from zero to two substituents selected, independently, from hydroxy, oxo, (C₁-C₆)alkoxy, phenyl-(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
R⁶ is selected from hydrogen, -SO(C₁-C₆)alkyl, -SO₂-(C₁-C₆)alkyl, -SO-aryl, - SO₂-aryl, CF₃, phenyl, phenyl-(C₁-C₂)alkyl, pyridyl, tetrazolyl, oxazolyl, thiazolyl, and (C₁-C₆)alkyl substituted with one or more substituents preferably from zero to two substituents selected, independently from hydroxy, oxo, (C₁-C₆)alkoxy, phenyl-(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
each R⁹ and each R¹⁰ is selected, independently, from hydrogen, (C₁-C₆)alkyl, hydroxy(C₁-C₆)alkyl, phenyl and CF₃;
or R⁹ and R¹⁰, when R³ is NR⁹R¹⁰ or CONR⁹R¹⁰, can form, together with the nitrogen to which they are attached, an optionally substituted heterocyclic ring that contains at least one nitrogen atom;
and wherein the phenyl groups in the definition of R⁵, R⁶, R⁷ and R⁸ and the phenyl moiety of phenyl (C₁-C₂)alkyl in the definition of R⁵, R⁶, R⁷ and R⁸ can optionally be substituted with one or more substituents, preferably with from zero to two substituents, that are selected, independently, from halo, hydroxy, (C₁-C₆)alkoxy optionally substituted with from one to seven fluorine atoms, preferably with from zero to three fluorine atoms, and (C₁-C₆)alkyl optionally substituted with from one to seven fluorine atoms, preferably with from zero to three fluorine atoms;
or a pharmaceutically acceptable salt thereof.

2. A compound according to claim 1, wherein R³ is an optionally substituted heterocyclic ring, or an alkyl group substituted with an optionally substituted heterocyclic ring, wherein said heterocyclic ring is selected from the following: pyrimidinyl, benzoxazolyl, 2,3-dihydro-3-oxobenzisosulfonazol-2-yl, morpholin-1-yl, thiomorpholin-1-yl, benzofuranyl, benzothienyl, indolyl, isoindolyl, isoquinolinyl, furyl, pyridyl, isothiazolyl, oxazolyl, triazolyl, tetrazolyl, quinolyl, thiazolyl, and thienyl, and groups of the formulas 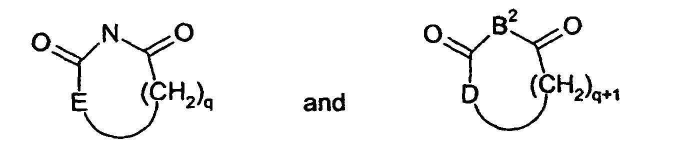 wherein B² and D are selected from carbon, oxygen and nitrogen, and at least one of B² and D is other than carbon; E is carbon or nitrogen; q is an integer from 1 to 5; any one of the carbon atoms of said (CH₂)_{q} and (CH₂)_{q+1} may be optionally substituted with (C₁-C₆)alkyl or (C₁-C₆) spiroalkyl; and either any one pair of the carbon atoms of said (CH₂)_{q} and (CH₂)_{q+1} may be bridged by a one or two carbon atom linkage, or any one pair of adjacent carbon atoms of said (CH₂)_{q} and (CH₂)_{q+1} may form, together with from one to three carbon atoms that are not members of the carbonyl containing ring, a (C₃-C₅) fused carbocyclic ring.

3. A compound according to claim 1, wherein Q is a carbonyl group.

4. A compound according to claim 1, wherein B is ethylene, A is CH and G is NHCH₂.

5. A compound according to claim 1, wherein B is ethylene, A is CH and G is SCH₂.

6. A compound according to claim 1, wherein B is absent, G is NH and A is CH₂.

7. A compound according to claim 1, wherein W is ethylene.

8. A compound according to claim 1, wherein R⁴ is phenyl.

9. A compound according to claim 1, wherein R⁴ is phenyl and R⁸ is hydrogen.

10. A compound according to claim 1, wherein p is one.

11. A compound according to claim 1, wherein R² is (C₁-C₆)alkyl.

12. A compound according to claim 1, wherein R⁴ is 2-, 3- or 4-pyridyl.

13. A compound according to claim 1, wherein R² and R¹² are selected, independently, from methyl and ethyl.

14. A compound according to claim 1, wherein Y is CH and Z is nitrogen.

15. A compound according to claim 1, wherein Y is nitrogen and Z is CH.

16. A compound according to claim 1, wherein Q is C=S.

17. A compound that is selected from isomers and mixtures of isomers of the following compounds, wherein said isomers or mixtures of isomers have the stereochemistry depicted in structural formula (I):
6-Methoxy-1-methyl-7-[(2-phenyl-piperidin-3-ylamino)-methyl]-3,4-dihydro-1H-[1,5]naphthyridin-2-one;
6-Methoxy-1-methyl-7-[(6-methyl-2-phenyl-piperidin-3-ylamino)-methyl]-3,4-dihydro-1H-[1,5]naphthyridin-2-one;
7-[(6-Ethyl-2-phenyl-piperidin-3-ylamino)-methyl]-6-methoxy-1-methyl-3,4-dihydro-1H-[1,5]naphthyridin-2-one;
6-Methoxy-1-methyl-7-[(2-phenyl-6-propyl-piperidin-3-ylamino)-methyl]-3,4-dihydro-1H-[1,5]naphthyridin-2-one;
7-[((2S,3S,6S)-6-isopropyl-2-phenyl-piperidin-3-ylamino)-methyl]-6-methoxy-1-methyl-3,4-dihydro-1H-[1,5]naphthyridin-2-one;
and pharmaceutically acceptable salts thereof.

18. A compound of formula (I) as defined in claim 1 for use as a medicament.

19. Use of a compound of formula (I) as defined in claim 1 in the manufacture of a medicament for the treatment or prevention of a disorder or condition selected from the group consisting of mood disorders, such as depression, or more particularly, depressive disorders, for example, single episodic or recurrent major depressive disorders, dysthymic disorders, depressive neurosis and neurotic depression, melancholic depression, including anorexia, weight loss, insomnia, early morning waking and psychomotor retardation, atypical depression (or reactive depression), including increased appetite, hypersomnia, psychomotor agitation or irritability, seasonal affective disorder and pediatric depression; or bipolar disorders or manic depression, for example, bipolar I disorder, bipolar II disorder and cyclothymic disorder; conduct disorder and disruptive behavior disorder; anxiety disorders, such as panic disorder with or without agoraphobia, agoraphobia without history of panic disorder, specific phobias, for example, specific animal phobias, social anxiety, social phobias, obsessive-compulsive disorder, stress disorders, including post-traumatic stress disorder and acute stress disorder, and generalized anxiety disorders; borderline personality disorder; schizophrenia and other psychotic disorders, for example, schizophreniform disorders, schizoaffective disorders, delusional disorders, brief psychotic disorders, shared psychotic disorders, psychotic disorders with delusions or hallucinations, psychotic episodes of anxiety, anxiety associated with psychosis, psychotic mood disorders such as severe major depressive disorder; mood disorders associated with psychotic disorders such as acute mania and depression associated with bipolar disorder, mood disorders associated with schizophrenia; behavioral disturbances associated with mental retardation, autistic disorder, and conduct disorder.

20. Use of a compound of formula (I) as defined in claim 1 in the manufacture of a medicament for the treatment or prevention of a disorder or condition selected from the group consisting of delerium, dementia, and amnestic and other cognitive or neurodegenerative disorders, such as Parkinson's disease (PD), Huntington's disease (HD), Alzheimer's disease, senile dementia, dementia of the Alzheimer's type, memory disorder, vascular dementia, and other dementias, for example, due to HIV disease, head trauma, Parkinson's disease, Huntington's disease, Pick's disease, Creutzfeldt-Jakob disease, or due to multiple aetiologies; movement disorders such as akinesias, dyskinesias, including familial paroxysmal dyskinesias, spasticities, Tourette's syndrome, Scott syndrome, PALSYS and akinetic-rigid syndrome; extra-pyramidal movement disorders such as medication-induced movement disorders, for example, neuroteptic-induced Parkinsonism, neuroleptic malignant syndrome, neuroleptic-induced acute dystonia, neuroteptic-induced acute akathisia, neurofeptic-induced tardive dyskinesia and medication-induced postural tremour; substance-related disorders arising from the use of alcohol, amphetamines (or amphetamine-like substances) caffeine, cannabis, cocaine, hallucinogens, inhalants and aerosol propellants, nicotine, opioids, phenylglycidine derivatives, sedatives, hypnotics, and anxiolytics, which substance-related disorders include dependence and abuse, intoxication, withdrawal, intoxication delerium and withdrawal delerium; addictive behaviors such as gambling; epilepsy; Down's syndrome; acute pain, chronic pain and migraine; demyelinating diseases such as multiple sclerosis (MS) and amylolateral sclerosis (ALS), peripheral neuropathy, for example diabetic and chemotherapy-induced-neuropathy, and postherpetic neuralgia, trigeminal neuralgia, segmental or intercostal neuralgia and other neuralgias; and cerebral vascular disorders due to acute or chronic cerebrovascular damage such as cerebral infarction, subarachnoid haemorrhage or cerebral oedema.

21. Use of a compound of formula (I) as defined in claim 1 in the manufacture of a medicament for the treatment or prevention of a disorder or condition selected from the group consisting of respiratory diseases, particularly those associated with excess mucus secretion, such as chronic obstructive airways disease, bronchopneumonia, chronic bronchitis, cystic fibrosis, adult respiratory distress syndrome, and bronchospasm; inflammatory diseases such as inflammatory bowel disease, psoriasis, Reiter's syndrome, Raynaud's syndrome, anthropathies, fibrositis, osteoarthritis, rheumatoid arthritis, psoriatic arthritis, asthma, pruritis and sunburn; human immunodeficiency virus (HIV) infections; allergies such as eczema and rhinitis, and other allergies; hypersensitivity disorders such as poison ivy; ophthalmic diseases such as conjunctivitis, vernal conjunctivitis, and the like; ophthalmic conditions associated with cell proliferation such as proliferative vitreoretinopathy; cutaneous diseases such as contact dermatitis, atopic dermatitis, urticaria, and other eczematoid dermatitis.

22. Use of a compound of formula (I) as defined in claim 1 in the manufacture of a medicament for the treatment or prevention of a disorder or condition selected from the group consisting of neoplasms, including breast tumours, gastric carcinomas, gastric lymphomas, neuroganglioblastomas and small cell carcinomas such as small cell lung cancer.

23. Use of a compound of formula (I) as defined in claim 1 in the manufacture of a medicament for the treatment or prevention of a disorder or condition selected from the group consisting of gastrointestinal (GI) disorders, including inflammatory gastrointestinal disorders such as inflammation bowel disease disorders, caused by *helicobacter pylori* and diseases of the GI tract such as gastritis, gastroduodenal ulcers, disorders associated with the neuronal control of viscera, ulcerative colitis, Crohn's disease, irritable bowel syndrome and emesis.

24. Use of a compound of formula (I) as defined in claim 1 in the manufacture of a medicament for the treatment or prevention of a disorder or condition selected from the group consisting of stress related somatic disorders; reflex sympathetic dystrophy such as shoulder/hand syndrome; adverse immunological reactions such as rejection of transplanted tissues and disorders related to immune enhancement or suppression such as systemic lupus erythematosus; plasma extravasation resulting from cytokine chemotherapy; disorders of bladder function such as cystitis, bladder detrusor hyper-reflexia, inflammation of the urinary tract and incontinence, including urinary urge incontinence, overactive bladder, stress incontinence and mixed incontinence; fibrosing and collagen diseases such as scleroderma and eosinophilic fascioliasis; blood flow disorders caused by vasodilation and vasospastic diseases such as angina and Reynaud's disease; angiogenesis; cardiovascular disorders; eating disorders, such as anorexia nervosa and bulimia nervosa; attention deficit hyperactivity disorder; chronic fatigue syndrome; sexual dysfunctions including premature ejaculation and male erectile dysfunction; premenstrual syndrome and premenstrual dysphoric disorder, fibromyalgia; and rheumatic diseases such as fibrositis.

25. A pharmaceutical composition comprising a compound according to claim 1 and a pharmaceutically acceptable carrier.

26. A compound of the formula 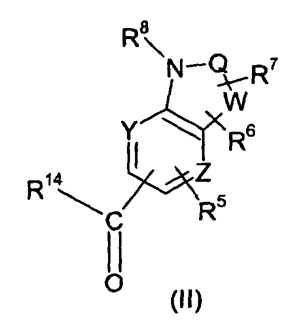
wherein Q is C=S or C=O;
Y is N and Z is CH, or Y is CH and Z is N;
W is a one carbon linking group (i.e., methylene) or a saturated or unsaturated two or three carbon linking group, wherein each of the foregoing W groups can optionally be substituted with one substituent R⁷ or two substituents R⁷ and R⁶, or W is a one carbon linking group that forms, together with a 2, 3, 4 or 5 carbon chain, a 3, 4, 5 or 6 membered spiro ring, respectively;
or W is a saturated two carbon chain linking group that forms, together with a separate 1, 2 or 3 carbon chain, a fused 3, 4 or 5 membered ring, respectively;
or W is a saturated two carbon chain linking group, wherein one of the two carbons in the chain forms, together with a separate 2, 3, 4 or 5 carbon chain, a 3, 4, 5 or 6 membered spiro ring, respectively;
R⁵ is selected from hydrogen, -SO(C₁-C₈)alkyl, -SO₂-(C₁-C₆)alkyl, -SO-aryl, - SO₂-aryl, CF₃, halo, phenyl, phenyl-(C₁-C₂)alkyl, hydroxy, aryloxy, heteroaryloxy, pyridyl, tetrazolyl, oxazolyl, thiazolyl, (C₁-C₆)alkoxy optionally substituted with from one to seven fluorine atoms, preferably with from zero to three fluorine atoms, (C₁-C₆)alkyl optionally substituted with from one to seven fluorine atoms, preferably with from zero to three fluorine atoms, and (C₁-C₆)alkyl substituted with one or more substituents, preferably with from zero to two substituents selected, independently, from hydroxy, oxo, (C₁-C₆)alkoxy, phenyl-(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
R⁶ and R⁷ are selected, independently, from -SO(C₁-C₆)alkyl, -SO₂-(C₁-C ₆)alkyl, -SO-aryl, -SO₂-aryl, CF₃, halo, phenyl, phenyl-(C₁-C₂)alkyl, hydroxy, aryloxy, heteroaryloxy, pyridyl, tetrazolyl, oxazolyl, thiazolyl, (C₁-C₆)alkoxy optionally substituted with from one to seven fluorine atoms, preferably with from zero to three fluorine atoms, (C₁-C₆)alkyl optionally substituted with from one to seven fluorine atoms, preferably with from zero to three fluorine atoms, and (C₁-C₆)alkyl substituted with one or more substituents, preferably with from zero to two substituents selected, independently, from hydroxy, oxo, (C₁-C₆)alkoxy, phenyl-(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
R⁸ is selected from hydrogen, -SO(C₁-C₆)alkyl, -SO₂-(C₁-C₆)alkyl, -SO-aryl, - SO₂-aryl, CF₃, phenyl, phenyl-(C₁-C₂)alkyl, pyridyl, tetrazolyl, oxazolyl, thiazolyl, and (C₁-C₆)alkyl substituted with one or more substituents preferably from zero to two substituents selected, independently from hydroxy, oxo, (C₁-C₆)alkoxy, phenyl-(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
each R⁹ and each R¹⁰ is selected, independently, from hydrogen, (C₁-C₆)alkyl, hydroxy(C₁-C₆)alkyl, phenyl and CF₃;
and wherein the phenyl groups in the definition of R⁵, R⁶, R⁷ and R⁸ and the phenyl moiety of phenyl (C₁-C₂)alkyl in the definition of R⁵, R⁶, R⁷ and R⁸ can optionally be substituted with one or more substituents, preferably with from zero to two substituents, that are selected, independently, from halo, hydroxy, (C₁-C₆)alkoxy optionally substituted with from one to seven fluorine atoms, preferably with from zero to three fluorine atoms, and (C₁-C₆)alkyl optionally substituted with from one to seven fluorine atoms, preferably with from zero to three fluorine atoms; and
R¹⁴ is hydrogen, (C₁-C₆)alkyl or CF₃;
or a pharmaceutically acceptable salt thereof.

27. A compound according to claim 26, selected from the group consisting of: 3-Methoxy-8-methyl-7-oxo-5,6,7,8-tetrahydro-[1,8]naphthyridine-2-carbaldehyde; 2-Methoxy-5-methyl-6-oxo-5,6,7,8-tetrahydro-[1,5]naphthyridine-3-carbaldehyde; and pharmaceutically acceptable salts thereof.

## Patentansprüche

1. Verbindung der Formel 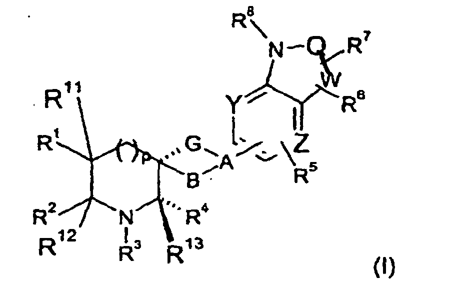 worin
Q die Bedeutungen C=S oder C=O hat;
A CH, CH₂, C(C₁-C₆)-Alkyl, CH(C₁-C₆)-Alkyl, C(CF₃) oder CH(CF₃) bedeutet, mit der Maßgabe, dass dann, wenn B vorhanden ist, A entweder CH, C(C₁-C₆)-Alkyl oder C(CF₃) bedeuten muss;
B abwesend ist oder Methylen oder Ethylen bedeutet;
Y N bedeutet und Z CH bedeutet oder Y CH bedeutet und Z N bedeutet;
G NH(CH₂)_{q}, S(CH₂)_{q} oder O(CH₂)_{q} bedeutet, wobei q den Wert 0 oder 1 hat;
mit der Maßgabe, dass dann, wenn q den Wert 0 hat, G NH, S oder 0 bedeutet;
W eine Verknüpfungsgruppe mit einem Kohlenstoffatom (d. h. Methylen) oder eine gesättigte oder ungesättigte Verknüpfungsgruppe mit 2 oder 3 Kohlenstoffatomen bedeutet, wobei jede der vorstehenden W-Gruppen optional mit einem Substituenten R⁷ oder mit zwei Substituenten R⁷ und R⁶ substituiert sein kann, oder W eine Verknüpfungsgruppe mit einem Kohlenstoffatom bedeutet, die zusammen mit einer Kette mit 2, 3, 4 oder 5 Kohlenstoffatomen einen 3-, 4-, 5- bzw. 6-gliedrigen Spiroring bildet;
oder W eine Verknüpfungsgruppe aus einer gesättigten Kette mit 2 Kohlenstoffatomen bedeutet, die zusammen mit einer getrennten Kette mit 1, 2 oder 3 Kohlenstoffatomen einen kondensierten 3-, 4- bzw. 5-gliedrigen Ring bildet;
oder W eine Verknüpfungsgruppe aus einer gesättigten Kette mit 2 Kohlenstoffatomen bedeutet, wobei eines der beiden Kohlenstoffatome in der Kette zusammen mit einer getrennten Kette mit 2, 3, 4 oder 5 Kohlenstoffatomen einen 3-, 4-, 5- bzw. 6-gliedrigen Spiroring bildet;
p den Wert 0, 1 oder 2 hat;
R³ ausgewählt ist aus Wasserstoff, COR⁹, CO₂R⁹, optional substituiertem Phenyl, optional substituierten heterocyclischen Ringen und optional substituiertem (C₁-C₈)-Alkyl, wobei eine der CH₂-Gruppen des (C₁-C₈)-Alkylrestes optional durch eine Schwefel-, Sauerstoff- oder Carbonylgruppe ersetzt sein kann und wobei der (C₁-C₈)-Alkylrest optional mit 1 bis 3 Substituenten, vorzugsweise mit 0 oder 1 Substituent substituiert sein kann, die unabhängig voneinander ausgewählt sind aus Hydroxy, Oxo, Phenyl-(C₁-C₃)-alkoxy, Phenyl, Cyano, Halogen, optional substituierten heterocyclischen Ringen, NR⁹COR¹⁰, NR⁹CO₂R¹⁰ CONR⁹R¹⁰, COR⁹, CO₂R⁹, NR⁹R¹⁰ und (C₁-C₆)-Alkoxy, optional substituiert mit 1 bis 7 Fluoratomen, vorzugsweise mit 0 bis 3 Fluoratomen;
und wobei die heterocyclischen Ringe von R³ und die heterocyclischen Ringsubstituenten an den Alkylgruppen von R³ unabhängig voneinander aus 3- bis 7-gliedrigen, gesättigten oder ungesättigten, monocyclischen Ringen mit 1 bis 4 Ringheteroatomen und 8- bis 12-gliedrigen, gesättigten oder ungesättigten, bicyclischen Ringen mit 1 bis 4 Ringheteroatomen ausgewählt sind, wobei die Heteroatome unabhängig voneinander aus Sauerstoff, Stickstoff und Schwefel ausgewählt sind, mit der Maßgabe, dass in den monocyclischen oder bicyclischen heterocyclischen Ringen nicht zwei benachbarte Ringsauerstoffatome oder zwei benachbarte Ringschwefelatome vorliegen können, und mit der Maßgabe, dass aus NR⁹R¹⁰ oder CONR⁹R¹⁰ gebildete heterocyclische Ringe mindestens ein Stickstoffatom enthalten müssen;
und wobei die heterocyclischen Ringe von R³ und die heterocyclischen Ringsubstituenten an den Alkylgruppen von R³ optional mit einem oder mehreren Substituenten und vorzugsweise mit 0, 1 oder 2 Substituenten substituiert sein können, die unabhängig voneinander ausgewählt sind aus Oxo, Hydroxy, Thioxo, Halogen, Cyano, Phenyl, (CH₂)ₘNR⁹R¹⁰, NR⁹COR¹⁰, (CH₂)ₘOR⁹, wobei m den Wert 0, 1 oder 2 hat, und (C₁-C₆)-Alkyl, optional substituiert mit einem oder mehreren Substituenten, vorzugsweise mit 0 bis 2 Substituenten, die unabhängig voneinander aus Halogen, CF₃, Methoxy und Phenyl ausgewählt sind;
und wobei die Phenylgruppen von R³ und die Phenylsubstituenten in den Alkylgruppen von R³ optional mit einem oder mehreren Substituenten, vorzugsweise mit 0 bis 2 Substituenten substituiert sein können, die unabhängig voneinander ausgewählt sind aus Halogen, Cyano, Nitro, CF₃, (CH₂)ₘNR⁹R¹⁰, wobei m den Wert 0, 1 oder 2 hat, NR⁹COR¹⁰, NR⁹CO₂R¹⁰, CONR⁹R¹⁰, CO₂NR⁹R¹⁰, COR⁹, CO₂R⁹, (C₁-C₆)-Alkyl, optional substituiert mit 1 bis 7 Fluoratomen, vorzugsweise mit 0 bis 3 Fluoratomen, (C₁-C₆)-Alkoxy, optional substituiert mit 1 bis 7 Fluoratomen, vorzugsweise mit 0 bis 3 Fluoratomen, und (C₂-C₆)-Alkenyl, optional substituiert mit 1 bis 7 Fluoratomen, vorzugsweise mit 0 bis 3 Fluoratomen;
R¹, R², R¹¹, R¹² und R¹³ jeweils unabhängig voneinander ausgewählt sind aus Wasserstoff und (C₁-C₆)-Alkyl, optional substituiert mit einem oder mehreren Substituenten, vorzugsweise mit 0, 1 oder 2 Substituenten, die unabhängig voneinander aus Hydroxy, Oxo, (C₁-C₆)-Alkoxy und Cyano ausgewählt sind;
oder R¹ und R² zusammen mit den Kohlenstoffatomen, an die sie gebunden sind, oder R² und R³ zusammen mit dem Kohlenstoff- bzw. Stickstoffatom, an die sie gebunden sind, einen 5- oder 6-gliedrigen, gesättigten, heterocyclischen Ring mit 1 oder 2 Heteroatomen, die unabhängig voneinander aus Stickstoff, Sauerstoff und Schwefel ausgewählt sind, bilden, mit der Maßgabe, dass der Ring nicht zwei benachbarte Sauerstoffatome oder zwei benachbarte Schwefelatome aufweisen kann; oder R¹ und R² zusammen mit den Kohlenstoffatomen, an die sie gebunden sind, einen 5- oder 6-gliedrigen, gesättigten oder ungesättigten, carbocyclischen Ring bilden, und wobei die durch R¹ und R² oder durch R² und R³ gebildeten heterocyclischen und carbocyclischen Ringe mit einem oder mehreren Substituenten, vorzugsweise mit 0 oder 1 Substituenten substituiert sein können, die unabhängig voneinander ausgewählt sind aus Halogen, Oxo, NR⁹R¹⁰, (C₁-C₆)-Alkyl, optional substituiert mit 1 bis 7 Fluoratomen, vorzugsweise mit 0 bis 3 Fluoratomen, und (C₁-C₆)-Alkoxy, optional substituiert mit 1 bis 7 Fluoratomen, vorzugsweise mit 0 bis 3 Fluoratomen;
oder R¹² und R¹³ zusammen mit den Kohlenstoffatomen, an die sie gebunden sind, einen 5- oder 6-gliedrigen, gesättigten, heterocyclischen Ring mit 1 oder 2 Heteroatomen, die unabhängig voneinander aus Stickstoff, Sauerstoff und Schwefel ausgewählt sind, bilden, mit der Maßgabe, dass der Ring nicht zwei benachbarte Sauerstoffatome oder zwei benachbarte Schwefelatome enthalten kann, oder R¹² und R¹³ zusammen mit den Kohlenstoffatomen, an die sie gebunden sind, einen 5- oder 6-gliedrigen, gesättigten oder ungesättigten carbocyclischen Ring bilden, und wobei die durch R¹² und R¹³ gebildeten heterocyclischen und carbocyclischen Ringe mit einem oder mehreren Substituenten, vorzugsweise mit 0 oder 1 Substituenten substituiert sein können, die unabhängig voneinander ausgewählt sind aus NR⁹R¹⁰, Halogen, Phenyl-S-, Phenyl-SO-, Phenyl-SO₂-, Oxo, (C₁-C₆)-Alkoxy, optional substituiert mit 1 bis 7 Fluoratomen, vorzugsweise mit 0 bis 3 Fluoratomen und, (C₁-C₆)-Alkyl, optional substituiert mit 1 bis 7 Fluoratomen, vorzugsweise mit 0 bis 3 Fluoratomen;
mit der Maßgabe, dass nicht mehr als eines der Paare R¹ und R², R² und R³ sowie R¹² und R¹³ einen Ring bilden können;
R⁴ ausgewählt ist aus Phenyl, 2-, 3- oder 4-Pyridyl, 2- oder 3-Thienyl und Pyrimidyl, wobei R⁴ optional mit einem oder mehreren Substituenten, vorzugsweise mit 0 oder 1 Substituenten substituiert sein kann, die unabhängig voneinander ausgewählt sind aus Halogen, (C₁-C₆)-Alkyl, optional substituiert mit 1 bis 7 Fluoratomen, vorzugsweise mit 0 bis 3 Fluoratomen, (C₁-C₆)-Alkoxy, optional substituiert mit 1 bis 7 Fluoratomen, vorzugsweise mit 0 bis 3 Fluoratomen, und (C₂-C₆)-Alkenyl, optional substituiert mit 1 bis 7 Fluoratomen, vorzugsweise mit 0 bis 3 Fluoratomen;
R⁵ ausgewählt ist aus Wasserstoff, -SO(C₁-C₆)-Alkyl, -SO₂-(C₁-C₆)-Alkyl, -SO-Aryl, -SO₂-Aryl, CF₃, Phenyl, Phenyl-(C₁-C₂)-alkyl, Hydroxy, Aryloxy, Heteroaryloxy, Pyridyl, Tetrazolyl, Oxazolyl, Thiazolyl, (C₁-C₆)-Alkoxy, optional substituiert mit 1 bis 7 Fluoratomen, vorzugsweise mit 0 bis 3 Fluoratomen, (C₁-C₆)-Alkyl, optional substituiert mit 1 bis 7 Fluoratomen, vorzugsweise mit 0 bis 3 Fluoratomen, und (C₁-C₆)-Alkyl, substituiert mit einem oder mehreren Substituenten, vorzugsweise mit 0 bis 2 Substituenten, die unabhängig voneinander ausgewählt sind aus Hydroxy, Oxo, (C₁-C₆)-Alkoxy, Phenyl-(C₁-C₃)-alkoxy, Phenyl, Cyano, Chlor, Brom, Iod, NR⁹R¹⁰, NR⁹COR¹⁰, NR⁹CO₂R¹⁰, CONR⁹R¹⁰, COR⁹ und CO₂R⁹;
R⁶ und R⁷ unabhängig voneinander ausgewählt sind aus -SO(C₁-C₆)-Alkyl, -SO₂-(C₁-C₆)-Alkyl, -SO-Aryl, -SO₂-Aryl, CF₃, Halogen, Phenyl, Phenyl-(C₁-C₂)-alkyl, Hydroxy, Aryloxy, Heteroaryloxy, Pyridyl, Tetrazolyl, Oxazolyl, Thiazolyl, (C₁-C₆)-Alkoxy, optional substituiert mit 1 bis 7 Fluoratomen, vorzugsweise mit 0 bis 3 Fluoratomen, (C₁-C₆)-Alkyl, optional substituiert mit 1 bis 7 Fluoratomen, vorzugsweise mit 0 bis 3 Fluoratomen, und (C₁-C₆)-Alkyl, optional substituiert mit einem oder mehreren Substituenten, vorzugsweise mit 0 bis 2 Substituenten, die unabhängig voneinander ausgewählt sind aus Hydroxy, Oxo, (C₁-C₆)-Alkoxy, Phenyl-(C₁-C₃)-alkoxy, Phenyl, Cyano, Chlor, Brom, Iod, NR⁹R¹⁰, NR⁹COR¹⁰, NR⁹CO₂R¹⁰, CONR⁹R¹⁰, COR⁹ und CO₂R⁹;
R⁸ ausgewählt ist aus Wasserstoff, -SO(C₁-C₆)-Alkyl, -SO₂-(C₁-C₆)-Alkyl, -SO-Aryl, -SO₂-Aryl, CF₃, Phenyl, Phenyl-(C₁-C₂)-alkyl, Pyridyl, Tetrazolyl, Oxazolyl, Thiazolyl und (C₁-C₆)-Alkyl, substituiert mit einem oder mehreren Substituenten, vorzugsweise mit 0 bis 2 Substituenten, die unabhängig voneinander ausgewählt sind aus Hydroxy, Oxo, (C₁-C₆)-Alkoxy, Phenyl-(C₁-C₃)-alkoxy, Phenyl, Cyano, Chlor, Brom, Iod, NR⁹R¹⁰, NR⁹COR¹⁰, NR⁹CO₂R¹⁰, CONR⁹R¹⁰, COR⁹ und CO₂R⁹;
jeder Rest R⁹ und jeder Rest R¹⁰ jeweils unabhängig voneinander ausgewählt ist aus Wasserstoff, (C₁-C₆)-Alkyl, Hydroxy-(C₁-C₆)-alkyl, Phenyl und CF₃;
oder R⁹ und R¹⁰, wenn R³ die Bedeutung NR⁹R¹⁰ oder CONR⁹R¹⁰ hat, zusammen mit dem Stickstoffatom, an das sie gebunden sind, einen optional substituierten heterocyclischen Ring bilden können, der mindestens ein Stickstoffatom enthält;
und wobei die Phenylgruppen in der Definition von R⁵, R⁶, R⁷ und R⁸ und der Phenylrest von Phenyl-(C₁-C₂)-alkyl in der Definition von R⁵, R⁶, R⁷ und R⁸ optional mit einem oder mehreren Substituenten, vorzugsweise mit 0 bis 2 Substituenten substituiert sein können, die unabhängig voneinander aus Halogen, Hydroxy, (C₁-C₆)-Alkoxy, optional substituiert mit 1 bis 7 Fluoratomen, vorzugsweise mit 0 bis 3 Fluoratomen, und (C₁-C₆)-Alkyl, optional substituiert mit 1 bis 7 Fluoratomen, vorzugsweise mit 0 bis 3 Fluoratomen, ausgewählt sind;
oder ein pharmazeutisch verträgliches Salz davon.

2. Verbindung nach Anspruch 1, wobei R³ einen optional substituierten heterocyclischen Ring oder eine Alkylgruppe, die mit einem optional substituierten heterocyclischen Ring substituiert ist, bedeutet, wobei der heterocyclische Ring ausgewählt ist aus Pyrimidinyl, Benzoxazolyl, 2,3-Dihydro-3-oxobenzisosulfonazol-2-yl, Morpholin-1-yl, Thiomorpholin-1-yl, Benzofuranyl, Benzothienyl, Indolyl, Isoindolyl, Isochinolinyl, Furyl, Pyridyl, Isothiazolyl, Oxazolyl, Triazolyl, Tetrazolyl, Chinolyl, Thiazolyl und Thienyl, und Gruppen der Formeln 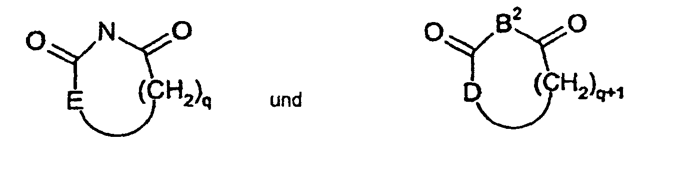 wobei B² und D unabhängig voneinander aus Kohlenstoff, Sauerstoff und Stickstoff ausgewählt sind und mindestens einer der Reste B² und D eine von Kohlenstoff abweichende Bedeutung hat; E Kohlenstoff oder Stickstoff bedeutet; q eine ganze Zahl mit einem Wert von 1 bis 5 ist; jedes beliebige der Kohlenstoffatome von (CH₂)_{q} und (CH₂)_{q+1} optional mit (C₁-C₆)-Alkyl oder (C₁-C₆)-Spiroalkyl substituiert sein kann; und entweder jedes beliebige Paar der Kohlenstoffatome von (CH₂)_{q} und (CH₂)_{q+1} durch 1 oder 2 Kohlenstoffatom-Verknüpfungen überbrückt sein kann oder jedes beliebige Paar von benachbarten Kohlenstoffatomen von (CH₂)_{q} und (CH₂)_{q+1} zusammen mit 1 bis 3 Kohlenstoffatomen, die nicht Bestandteile des carbonylhaltigen Rings sind, einen (C₃-C₅)-kondensierten carbocyclischen Ring bilden kann.

3. Verbindung nach Anspruch 1, wobei Q eine Carbonylgruppe bedeutet.

4. Verbindung nach Anspruch 1, wobei B Ethylen bedeutet, A CH bedeutet und G NHCH₂ bedeutet.

5. Verbindung nach Anspruch 1, wobei B Ethylen bedeutet, A CH bedeutet und G SCH₂ bedeutet.

6. Verbindung nach Anspruch 1, wobei B abwesend ist, G NH bedeutet und A CH₂ bedeutet.

7. Verbindung nach Anspruch 1, wobei W Ethylen bedeutet.

8. Verbindung nach Anspruch 1, wobei R⁴ Phenyl bedeutet.

9. Verbindung nach Anspruch 1, wobei R⁴ Phenyl bedeutet und R⁸ Wasserstoff bedeutet.

10. Verbindung nach Anspruch 1, wobei p den Wert 1 hat.

11. Verbindung nach Anspruch 1, wobei R² (C₁-C₆)-Alkyl bedeutet.

12. Verbindung nach Anspruch 1, wobei R⁴ 2-, 3- oder 4-Pyridyl bedeutet.

13. Verbindung nach Anspruch 1, wobei R² und R¹² unabhängig voneinander aus Methyl und Ethyl ausgewählt sind.

14. Verbindung nach Anspruch 1, wobei Y CH bedeutet und Z Stickstoff bedeutet.

15. Verbindung nach Anspruch 1, wobei Y Stickstoff bedeutet und Z CH bedeutet.

16. Verbindung nach Anspruch 1, wobei Q C=S bedeutet.

17. Verbindung, ausgewählt aus Isomeren und Gemischen von Isomeren der folgenden Verbindungen, wobei die Isomeren oder Gemische von Isomeren die in der Strukturformel (I) dargestellte Stereochemie aufweisen:
6-Methoxy-1-methyl-7-[(2-phenylpiperidin-3-ylamino)-methyl]-3,4-dihydro-1H-[1,5]naphthyridin-2-on;
6-Methoxy-1-methyl-7-[(6-methyl-2-phenylpiperidin-3-ylamino)-methyl]-3,4-dihydro-1H-[1,5]naphthyridin-2-on;
7-[(6-Ethyl-2-phenylpiperidin-3-ylamino)-methyl]-6-methoxy-1-methyl-3,4-dihydro-1H-[1,5]naphthyridin-2-on;
6-Methoxy-1-methyl-7-[(2-phenyl-6-propylpiperidin-3-ylamino)-methyl]-3,4-dihydro-1H-[1,5]naphthyridin-2-on;
7-[((2S,3S,6S)-6-Isopropyl-2-phenylpiperidin-3-ylamino)-methyl]-6-methoxy-1-methyl-3,4-dihydro-1H-[1,5]naphthyridin-2-on;
und pharmazeutisch verträgliche Salze davon.

18. Verbindung der Formel (I) gemäß der Definition in Anspruch 1 zur Verwendung als Arzneimittel.

19. Verwendung einer Verbindung der Formel (I) gemäß der Definition in Anspruch 1 bei der Herstellung eines Arzneimittels zur Therapie oder Prophylaxe einer Störung oder eines Zustands, die aus folgender Gruppe ausgewählt sind: Gemütsstörungen, wie Depressionen oder insbesondere depressive Störungen, z. B. einzelne episodische oder wiederkehrende größere depressive Störungen ("major depressive disorders"), dysthymische Störungen, depressive Neurose und neurotische Depressionen, melancholische Depressionen, einschließlich Anorexie, Gewichtsverlust, Schlaflosigkeit, frühzeitiges Erwachen am Morgen und psychomotorische Verzögerungen, atypische Depressionen (oder reaktive Depressionen), einschließlich verstärkter Appetit, Schlafsucht, psychosomatische Reizung oder Erregbarkeit, saisonale affektive Störungen und pädiatrische Depressionen; oder bipolare Störungen oder manische Depressionen, z. B. bipolare Störung I, bipolare Störung II und zyklothymische Störung; Verhaltensstörungen (conduct disorder) und Zerstörungs-Verhaltensstörungen (disruptive behavior disorder); Angststörungen, wie panische Störungen mit oder ohne Platzangst, Platzangst ohne Anamnese einer panischen Störung, spezielle Phobien, z. B. spezielle Tierphobien, soziale Angstgefühle, soziale Phobien, obsessiv-kompulsive Störungen, Stressstörungen, einschließlich posttraumatische Stressstörungen und akute Stressstörungen und allgemeine Angststörungen; Grenzfall-Persönlichkeitsstörungen (borderline personality disorder); Schizophrenie und andere psychotische Störungen, z. B. schizophreniforme Störungen, schizoaffektive Störungen, Delusionsstörungen (delusional disorders), kurze psychotische Störungen, geteilte psychotische Störungen, psychotische Störungen mit Delusionen oder Halluzinationen, psychotische Angstepisoden, Angst in Verbindung mit Psychosen, psychotische Stimmungsstörungen, wie schwere "Major depressive"-Störung (severe major depressive disorder); Stimmungsstörungen in Verbindung mit psychotischen Störungen, wie akute Manie und Depression in Verbindung mit bipolaren Störungen, Stimmungsstörungen in Verbindung mit Schizophrenie; Verhaltensstörungen in Verbindung mit geistiger Minderentwicklung, autistische Störungen und Verhaltensstörungen.

20. Verwendung einer Verbindung der Formel (I) gemäß der Definition in Anspruch 1 bei der Herstellung eines Arzneimittels zur Therapie oder Prophylaxe einer Störung oder eines Zustands, die aus folgender Gruppe ausgewählt sind: Delirium, Demenz und amnesische und andere kognitive oder neurodegenerative Störungen, wie Parkinson-Krankheit (PD), Huntington-Krankheit (HD), Alzheimer-Krankheit, senile Demenz, Demenz vom Alzheimer-Typ, Gedächtnisstörungen, vaskuläre Demenz und andere Demenzarten, z. B. aufgrund von HIV-Krankheit, Kopftrauma, Parkinson-Krankheit, Huntington-Krankheit, Pick-Krankheit, Creutzfeldt-Jakob-Krankheit oder aufgrund von multipler Ätiologie; Bewegungsstörungen, wie Akinesien, Dyskinesien, einschließlich familiäre, paroxysmale Dyskinesien, spastische Zustände, Tourette-Syndrom, Scott-Syndrom, PALSYS- und akinetisches-Starresyndrom (akinetic-rigid syndrome); extrapyramidale Bewegungsstörungen, wie durch Medikationen induzierte Bewegungsstörungen, z. B. neuroleptisch induzierter Parkinsonismus, neuroleptisches malignes Syndrom, neuroleptisch induzierte akute Dystonie, neuroleptisch induzierte akute Akathisie, neuroleptisch induzierte tardive Dyskinesie und durch Medikation induzierter posturaler Tremor; substanzbezogene Störungen, die sich aus dem Gebrauch von Alkohol, Amphetaminen (oder amphetaminartigen Substanzen), Koffein, Cannabis, Kokain, Halluzinogenen, Inhalationsmitteln und Aerosol-Treibmitteln, Nikotin, Opioiden, Phenylglycidinderivaten, Sedativa, Hypnotika und Anxiolytika ergeben, wobei diese substanzbezogenen Störungen Abhängigkeit und Missbrauch, Intoxikation, Entzug, Intoxikationsdelirium und Entzugsdelirium umfassen; Suchtverhalten, wie Spielsucht; Epilepsie; Down-Syndrom; akute Schmerzen, chronische Schmerzen und Migräne; Demyelinierungskrankheiten, wie multiple Sklerose (MS) und amylolaterale Sklerose (ALS), periphere Neuropathie, z. B. diabetische und durch Chemotherapie induzierte Neuropathie und postherpetische Neuralgie, Trigeminus-Neuralgie, segmentale und interkostale Neuralgie und andere Neuralgien; und zerebrale Gefäßstörungen aufgrund einer akuten oder chronischen zerebrovaskulären Schädigung, wie zerebraler Infarkt, subarachnoide Hämorrhagie oder zerebrales Ödem.

21. Verwendung einer Verbindung der Formel (I) gemäß der Definition in Anspruch 1 bei der Herstellung eines Arzneimittels zur Therapie oder Prophylaxe einer Störung oder eines Zustands, die aus folgender Gruppe ausgewählt sind: respiratorische Krankheiten, insbesondere solche in Verbindung mit übermäßiger Schleimsekretion, wie chronische obstruktive Luftwegerkrankungen, Bronchopneumonie, chronische Bronchitis, zystische Fibrose, Atemnotsyndrom bei Erwachsenen und Bronchospasmus; entzündliche Erkrankungen, wie entzündliche Darmerkrankung, Psoriasis, Reiter-Syndrom, Raynaud-Syndrom, Anthropathien, Fibrositis, Osteoarthritis, rheumatoide Arthritis, psoriatische Arthritis, Asthma, Pruritis und Sonnenbrand; Infektionen durch den humanen Immunschwächevirus (HIV); Allergien, wie Ekzeme und Rhinitis sowie andere Allergien; Überempfindlichkeitsstörungen, wie Giftefeu; ophthalmische Krankheiten, wie Konjunktivitis, vernale Konjunktivitis und dergl.; ophthalmische Zustände in Verbindung mit einer Zellproliferation, wie proliferative Vitreoretinopathie; Hautkrankheiten, wie Kontaktdermatitis, atopische Dermatitis, Nesselsucht und andere ekzematoide Dermatitisarten.

22. Verwendung einer Verbindung der Formel (I) gemäß der Definition in Anspruch 1 bei der Herstellung eines Arzneimittels zur Therapie oder Prophylaxe einer Störung oder eines Zustands, die aus folgender Gruppe ausgewählt sind: Neoplasmen, einschließlich Brusttumoren, Magenkarzinome, Magenlymphome, Neuroganglioblastome und kleinzellige Karzinome, wie kleinzelliges Lungenkarzinom.

23. Verwendung einer Verbindung der Formel (I) gemäß der Definition in Anspruch 1 bei der Herstellung eines Arzneimittels zur Therapie oder Prophylaxe einer Störung oder eines Zustands, die aus folgender Gruppe ausgewählt sind: gastrointestinale (GI) Störungen, einschließlich entzündliche gastrointestinale Störungen, wie entzündliche Darmerkrankungsstörungen, die durch *Helicobacter pylori* verursacht sind, und Erkrankungen des gastrointestinalen Trakts, wie Gastritis, gastroduodenale Geschwüre, Störungen in Verbindung mit der neuronalen Kontrolle von Eingeweiden, ulzerative Kolitis, Crohn-Krankheit, reizbares Darmsyndrom und Erbrechen.

24. Verwendung einer Verbindung der Formel (I) gemäß der Definition in Anspruch 1 bei der Herstellung eines Arzneimittels zur Therapie oder Prophylaxe einer Störung oder eines Zustands, die aus folgender Gruppe ausgewählt sind: somatische Störungen in Verbindung mit Stress; reflexsympathetische Dystrophie, wie Schulter/Hand-Syndrom; ungünstige immunologische Reaktionen, wie Abstoßung von transplantierten Geweben und Störungen in Verbindung mit verstärkten oder unterdrückten Immunreaktionen, wie systemischer Lupus erythematosus; Plasma-Extravasation aufgrund einer Cytokin-Chemotherapie; Störungen der Blasenfunktion, wie Zystitis, Blasen-Detrusor-Hyperreflexie, Entzündungen des Harntrakts und Inkontinenz, einschließlich Harndrang-Inkontinenz, Blasenüberaktivität, Stressinkontinenz und gemischte Inkontinenz; fibrosierende und Kollagen-Krankheiten, wie Sklerodermie und eosinophile Faszioliasis; Durchblutungsstörungen aufgrund von Vasodilatationen und vasospastische Krankheiten, wie Angina und Reynaud-Krankheit; Angiogenese; kardiovaskuläre Störungen; Essstörungen, wie nervöse Anorexie und nervöse Bulimie; Aufmerksamkeitsdefizit-Hyperaktivitätsstörungen; chronisches Müdigkeitssyndrom; sexuelle Fehlfunktionen, einschließlich vorzeitige Ejakulation und Erektionsfehlfunktionen beim Mann; prämenstruelles Syndrom und prämenstruelle dysphorische Störungen; Fibromyalgie; und rheumatische Krankheiten, wie Fibrositis.

25. Pharmazeutische Zusammensetzung, enthaltend eine Verbindung nach Anspruch 1 und einen pharmazeutisch verträglichen Träger.

26. Verbindung der Formel 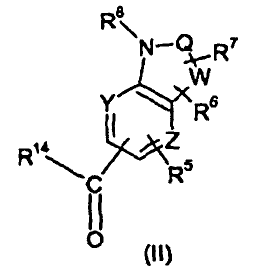 worin
Q C=S oder C=O bedeutet;
Y N bedeutet und Z CH bedeutet oder Y CH bedeutet und Z N bedeutet;
W eine Verknüpfungsgruppe mit einem Kohlenstoffatom (d. h. Methylen) oder eine gesättigte oder ungesättigte Verknüpfungsgruppe mit 2 oder 3 Kohlenstoffatomen bedeutet, wobei jede der vorstehenden W-Gruppen optional mit einem Substituenten R⁷ oder mit zwei Substituenten R⁷ und R⁶ substituiert sein kann, oder W eine Verknüpfungsgruppe mit einem Kohlenstoffatom bedeutet, die zusammen mit einer Kette mit 2, 3, 4 oder 5 Kohlenstoffatomen einen 3-, 4-, 5- bzw. 6-gliedrigen Spiroring bildet;
oder W eine Verknüpfungsgruppe aus einer gesättigten Kette mit 2 Kohlenstoffatomen bedeutet, die zusammen mit einer getrennten Kette mit 1, 2 oder 3 Kohlenstoffatomen einen kondensierten 3-, 4- bzw. 5-gliedrigen Ring bildet;
oder W eine Verknüpfungsgruppe aus einer gesättigten Kette mit 2 Kohlenstoffatomen bedeutet, wobei eines der beiden Kohlenstoffatome in der Kette zusammen mit einer getrennten Kette mit 2, 3, 4 oder 5 Kohlenstoffatomen einen 3-, 4-, 5- bzw. 6-gliedrigen Spiroring bildet;
R⁵ ausgewählt ist aus Wasserstoff, -SO(C₁-C₆)-Alkyl, -SO₂-(C₁-C₆)-Alkyl, -SO-Aryl, -SO₂-Aryl, CF₃, Halogen, Phenyl, Phenyl-(C₁-C₂)-alkyl, Hydroxy, Aryloxy, Heteroaryloxy, Pyridyl, Tetrazolyl, Oxazolyl, Thiazolyl, (C₁-C₆)-Alkoxy, optional substituiert mit 1 bis 7 Fluoratomen, vorzugsweise mit 0 bis 3 Fluoratomen, (C₁-C₆)-Alkyl, optional substituiert mit 1 bis 7 Fluoratomen, vorzugsweise mit 0 bis 3 Fluoratomen, und (C₁-C₆)-Alkyl, substituiert mit einem oder mehreren Substituenten, vorzugsweise mit 0 bis 2 Substituenten, die unabhängig voneinander ausgewählt sind aus Hydroxy, Oxo, (C₁-C₆)-Alkoxy, Phenyl-(C₁-C₃)-alkoxy, Phenyl, Cyano, Chlor, Brom, Iod, NR⁹R¹⁰, NR⁹COR¹⁰, NR⁹CO₂R¹⁰, CONR⁹R¹⁰, COR⁹ und CO₂R⁹;
R⁶ und R⁷ unabhängig voneinander ausgewählt sind aus -SO(C₁-C₆)-Alkyl, -SO₂-(C₁-C₆)-Alkyl, -SO-Aryl, -SO₂-Aryl, CF₃, Halogen, Phenyl, Phenyl-(C₁-C₂)-alkyl, Hydroxy, Aryloxy, Heteroaryloxy, Pyridyl, Tetrazolyl, Oxazolyl, Thiazolyl, (C₁-C₆)-Alkoxy, optional substituiert mit 1 bis 7 Fluoratomen, vorzugsweise mit 0 bis 3 Fluoratomen, (C₁-C₆)-Alkyl, optional substituiert mit 1 bis 7 Fluoratomen, vorzugsweise mit 0 bis 3 Fluoratomen, und (C₁-C₆)-Alkyl, substituiert mit einem oder mehreren Substituenten, vorzugsweise mit 0 bis 2 Substituenten, die unabhängig voneinander ausgewählt sind aus Hydroxy, Oxo, (C₁-C₆)-Alkoxy, Phenyl-(C₁-C₃)-alkoxy, Phenyl, Cyano, Chlor, Brom, Iod, NR⁹R¹⁰, NR⁹COR¹⁰, NR⁹CO₂R¹⁰, CONR⁹R¹⁰, COR⁹ und CO₂R⁹;
R⁸ ausgewählt ist aus Wasserstoff, -SO(C₁-C₆)-Alkyl, -SO₂-(C₁-C₆)-Alkyl, -SO-Aryl, -SO₂-Aryl, CF₃, Phenyl, Phenyl-(C₁-C₂)-alkyl, Pyridyl, Tetrazolyl, Oxazolyl, Thiazolyl und (C₁-C₆)-Alkyl, substituiert mit einem oder mehreren Substituenten, vorzugsweise mit 0 bis 2 Substituenten, die unabhängig voneinander ausgewählt sind aus Hydroxy, Oxo, (C₁-C₆)-Alkoxy, Phenyl-(C₁-C₃)-alkoxy, Phenyl, Cyano, Chlor, Brom, Iod, NR⁹R¹⁰, NR⁹COR¹⁰, NR⁹CO₂R¹⁰, CONR⁹R¹⁰, COR⁹ und CO₂R⁹;
jeder Rest R⁹ und jeder Rest R¹⁰ jeweils unabhängig voneinander aus Wasserstoff, (C₁-C₆)-Alkyl, Hydroxy-(C₁-C₆)-alkyl, Phenyl und CF₃ ausgewählt ist ;
und wobei die Phenylgruppen in der Definition von R⁵, R⁶, R⁷ und R⁸ und der Phenylrest von Phenyl-(C₁-C₂)-alkyl in der Definition von R⁵, R⁶, R⁷ und R⁸ optional mit einem oder mehreren Substituenten, vorzugsweise mit 0 bis 2 Substituenten substituiert sein können, die unabhängig voneinander aus Halogen, Hydroxy, (C₁-C₆)-Alkoxy, optional substituiert mit 1 bis 7 Fluoratomen, vorzugsweise mit 0 bis 3 Fluoratomen, und (C₁-C₆)-Alkyl, optional substituiert mit 1 bis 7 Fluoratomen, vorzugsweise mit 0 bis 3 Fluoratomen, ausgewählt sind; und
R¹⁴ Wasserstoff, (C₁-C₆)-Alkyl oder CF₃ bedeutet;
oder ein pharmazeutisch verträgliches Salz davon.

27. Verbindung nach Anspruch 26, ausgewählt aus der folgenden Gruppe:
3-Methoxy-8-methyl-7-oxo-5,6,7,8-tetrahydro-[1,8]naphthyridin-2-carbaldehyd;
2-Methoxy-5-methyl-6-oxo-5,6,7,8-tetrahydro-[1,5]naphthyridin-3-carbaldehyd;
und pharmazeutisch verträgliche Salze davon.

## Revendications

1. Composé de formule 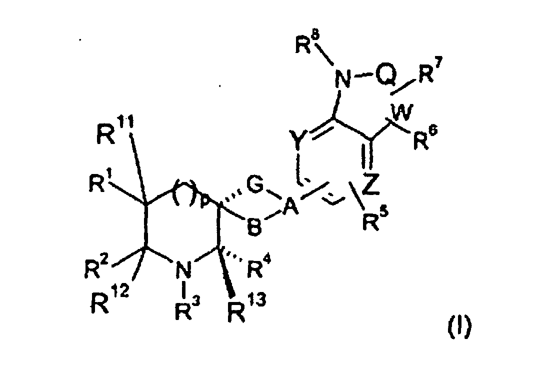
dans laquelle Q représente un groupe C=S ou C=O ;
A représente un groupe CH, CH₂, C (alkyle en C₁ à C₆), CH(alkyle en C₁ à C₆), C(CF₃) ou CH(CF₃), sous réserve que, lorsque B est présent, A représente un groupe CH, C(alkyle en C₁ à C₆) ou C(CF₃) ;
B est absent ou représente un groupe méthylène ou éthylène ;
Y représente un atome de N et Z représente un groupe CH, ou Y représente un groupe CH et Z représente un atome de N ;
G représente un groupe NH(CH₂)_{q}, S(CH₂)_{q} ou O(CH₂)_{q}, dans lequel q est égal à zéro ou un ;
sous réserve que, lorsque g est égal à zéro, G représente un groupe NH ou un atome de S ou O ;
W représente un groupe de liaison carboné (c'est-à-dire méthylène) ou un groupe de liaison saturé ou insaturé de deux ou trois atomes de carbone, chacun des groupes W précités pouvant être facultativement substitué avec un substituant R⁷ ou deux substituants R⁷ et R⁶, ou bien W représente un groupe de liaison carboné qui forme, conjointement avec une chaîne de 2, 3, 4 ou 5 atomes de carbone, un noyau spiro tri-, tétra-, penta- ou hexagonal, respectivement ;
ou W représente un groupe de liaison à chaîne saturée de deux atomes de carbone qui forme, conjointement avec une chaîne de 1, 2 ou 3 atomes de carbone distincte, un noyau condensé tri-, tétra- ou pentagonal, respectivement ;
ou W représente un groupe de liaison à chaîne saturée de deux atomes de carbone, dans lequel un des deux atomes de carbone dans la chaîne forme, conjointement avec une chaîne de 2, 3, 4 ou 5 atomes de carbone distincte, un noyau spiro tri-, tétra-, penta- ou hexagonal, respectivement ;
p est égal à 0, 1 ou 2 ;
R³ est choisi entre un atome d'hydrogène, des groupes COR⁹, CO₂R⁹, phényle facultativement substitué, des noyaux hétérocycliques facultativement substitués et un groupe alkyle en C₁ à C₈ facultativement substitué, un des groupes CH₂ dudit groupe alkyle en C₁ à C₈ pouvant être facultativement remplacé par un atome de soufre ou d'oxygène ou un groupe carbonyle et ledit groupe alkyle en C₁ à C₈ pouvant être facultativement substitué avec un à trois substituants, de préférence avec zéro substituant ou un substituant, choisis indépendamment entre des substituants hydroxy, oxo, phényl(alkoxy en C₁ à C₃), phényle, cyano, halogéno, des noyaux hétérocycliques facultativement substitués, NR⁹COR¹⁰, NR⁹CO₂R¹⁰, CONR⁹R¹⁰, COR⁹, CO₂R⁹, NR⁹R¹⁰ et un groupe alkoxy en C₁ à C₆ facultativement substitué avec un à sept atomes de fluor, de préférence avec zéro à trois atomes de fluor ;
et les noyaux hétérocycliques de R³ et les substituants à noyau hétérocyclique sur les groupes alkyle de R³ sont choisis, indépendamment, parmi des noyaux monocycliques tri- à heptagonaux saturés ou insaturés contenant 1 à 4 hétéroatomes dans le noyau, et des noyaux bicycliques de 8 à 12 membres saturés ou insaturés contenant 1 à 4 hétéroatomes dans les noyaux, lesdits hétéroatomes étant choisis, indépendamment, entre des atomes d'oxygène, d'azote et de soufre, sous réserve qu'il ne puisse pas exister deux atomes d'oxygène de noyau adjacents ou deux atomes de soufre de noyau adjacents dans les noyaux hétérocycliques, monocycliques ou bicycliques, et sous réserve que les noyaux hétérocycliques formés par NR⁹R¹⁰ ou CONR⁹R¹⁰ contiennent au moins un atome d'azote ;
et les noyaux hétérocycliques de R³ et les substituants à noyau hétérocyclique sur les groupes alkyle de R³ peuvent être facultativement substitués avec un ou plusieurs substituants, de préférence avec zéro, un ou deux substituants, choisis indépendamment entre des substituants oxo, hydroxy, thioxo, halogéno, cyano, phényle, (CH₂)ₘNR⁹R¹⁰, NR⁹R¹⁰, NR⁹COR¹⁰, (CH₂)ₘOR⁹, dans lequel m est égal à zéro, un ou deux, et alkyle en C₁ à C₆ facultativement substitué avec un ou plusieurs substituants, de préférence avec zéro à deux substituants, choisis indépendamment entre des substituants halogéno, CF₃, méthoxy et phényle ;
et les groupes phényle de R³ et les substituants phényle dans les groupes alkyle de R³ peuvent être facultativement substitués avec un ou plusieurs substituants, de préférence avec zéro à deux substituants, choisis indépendamment dans le groupe consistant en des substituants halogéno, cyano, nitro, CF₃, (CH₂)ₘNR⁹R¹⁰ dans lequel m est égal à zéro, un ou deux, NR⁹COR¹⁰, NR⁹CO₂R¹⁰, CONR⁹R¹⁰, CO₂NR⁹R¹⁰, COR⁹, CO₂R⁹, alkyle en C₁ à C₆ facultativement substitué avec un à sept atomes de fluor, de préférence avec zéro à trois atomes de fluor, alkoxy en C₁ à C₆ facultativement substitué avec un à sept atomes de fluor, de préférence avec zéro à trois atomes de fluor, et alcényle en C₂ à C₆ facultativement substitué avec un à sept atomes de fluor, de préférence avec zéro à trois atomes de fluor ;
chacun des groupes R¹, R², R¹¹, R¹² et R¹³ est choisi, indépendamment, entre un atome d'hydrogène et un groupe alkyle en C₁ à C₆ facultativement substitué avec un ou plusieurs substituants, de préférence avec zéro, un ou deux substituants qui sont choisis, indépendamment, entre des substituants hydroxy, oxo, alkoxy en C₁ à C₆ et cyano ;
ou bien R¹ et R², conjointement avec les atomes de carbone auxquels ils sont fixés, ou R² et R³, conjointement avec les atomes de carbone et d'azote auxquels ils sont fixés, respectivement, forment un noyau hétérocyclique penta- ou hexagonal saturé contenant un ou deux hétéroatomes qui sont choisis, indépendamment, entre des atomes d'azote, d'oxygène et de soufre, sous réserve que ledit noyau ne puisse pas contenir deux atomes d'oxygène adjacents ou deux atomes de soufre adjacents ; ou bien R¹ et R², conjointement avec les atomes de carbone auxquels ils sont fixés, forment un noyau carbocyclique penta- ou hexagonal saturé ou insaturé, lesdits noyaux hétérocycliques et carbocycliques formés par R¹ et R² ou R² et R³ pouvant être substitués avec un ou plusieurs substituants, de préférence avec zéro substituant ou un substituant, choisis indépendamment entre des substituants halogéno, oxo, NR⁹R¹⁰, alkyle en C₁ à C₆ facultativement substitué avec un à sept atomes de fluor, de préférence avec zéro à trois atomes de fluor, et alkoxy en C₁ à C₆ facultativement substitué avec un à sept atomes de fluor, de préférence avec zéro à trois atomes de fluor ;
ou bien R¹² et R¹³, conjointement avec les atomes de carbone auxquels ils sont fixés, forment un noyau hétérocyclique penta- ou hexagonal saturé contenant un ou deux hétéroatomes qui sont choisis, indépendamment, entre des atomes d'azote, d'oxygène et de soufre, sous réserve que ledit noyau ne puisse pas contenir deux atomes d'oxygène adjacents ou deux atomes de soufre adjacents, ou bien R¹² et R¹³, conjointement avec les atomes de carbone auxquels ils sont fixés, forment un noyau carbocyclique penta- ou hexagonal saturé ou insaturé, et lesdits noyaux hétérocycliques et carbocycliques formés par R¹² et R¹³ peuvent être substitués avec un ou plusieurs substituants, de préférence avec zéro substituant ou un substituant, choisis indépendamment entre des substituants NR⁹R¹⁰, halogéno, phényl-S-, phényl-SO-, phényl-SO₂-, oxo, alkoxy en C₁ à C₆ facultativement substitué avec un à sept atomes de fluor, de préférence avec zéro à trois atomes de fluor, et alkyle en C₁ à C₆ facultativement substitué avec un à sept atomes de fluor, de préférence avec zéro à trois atomes de fluor ;
sous réserve que pas plus d'un des groupes R¹ et R², R² et R³ et R¹² et R¹³ ne puisse former un noyau ;
R⁴ est choisi entre des groupes phényle, 2-, 3- ou 4-pyridyle, 2- ou 3-thiényle et pyrimidyle, R⁴ pouvant être facultativement substitué avec un ou plusieurs substituants, de préférence avec zéro ou un substituant, choisis, indépendamment, entre des substituants halogéno, alkyle en C₁ à C₆ facultativement substitué avec un à sept atomes de fluor, de préférence avec zéro à trois atomes de fluor, alkoxy en C₁ à C₆ facultativement substitué avec un à sept atomes de fluor, de préférence avec zéro à trois atomes de fluor, et alcényle en C₂ à C₆ facultativement substitué avec un à sept atomes de fluor, de préférence avec zéro à trois atomes de fluor ;
R⁵ est choisi entre un atome d'hydrogène, des groupes -SO(alkyle en C₁ à C₆), -SO₂-(alkyle en C₁ à C₆), -SO-aryle, -SO₂-aryle, CF₃, phényle, phényl-(alkyle en C₁ ou C₂), hydroxy, aryloxy, hétéroaryloxy, pyridyle, tétrazolyle, oxazolyle, thiazolyle, alkoxy en C₁ à C₆ facultativement substitué avec un à sept atomes de fluor, de préférence avec zéro à trois atomes de fluor, alkyle en C₁ à C₆ facultativement substitué avec un à sept atomes de fluor, de préférence avec zéro à trois atomes de fluor, et alkyle en C₁ à C₆ substitué avec un ou plusieurs substituants, de préférence avec zéro à deux substituants choisis, indépendamment, entre des substituants hydroxy, oxo, alkoxy en C₁ à C₆ , phényl-(alkoxy en C₁ à C₃), phényle, cyano, chloro, bromo, iodo, NR⁹R¹⁰, NR⁹COR¹⁰, NR⁹CO₂R¹⁰, CONR⁹R¹⁰, COR⁹ et CO₂R⁹ ;
R⁶ et R⁷ sont choisis, indépendamment, entre des groupes -SO (alkyle en C₁ à C₆), -SO₂-(alkyle en C₁ à C₆), -SO-aryle, -SO₂-aryle, CF₃, halogéno, phényle, phényl(alkyle en C₁ ou C₂), hydroxy, aryloxy, hétéroaryloxy, pyridyle, tétrazolyle, oxazolyle, thiazolyle, alkoxy en C₁ à C₆ facultativement substitué avec un à sept atomes de fluor, de préférence avec zéro à trois atomes de fluor, alkyle en C₁ à C₆ facultativement substitué avec un à sept atomes de fluor, de préférence avec zéro à trois atomes de fluor, et alkyle en C₁ à C₆ substitué avec un ou plusieurs substituants, de préférence avec zéro à deux substituants choisis, indépendamment, entre des substituants hydroxy, oxo, alkoxy en C₁ à C₆, phényl-(alkoxy en C₁ à C₃), phényle, cyano, chloro, bromo, iodo, NR⁹R¹⁰, NR⁹COR¹⁰, NR⁹CO₂R¹⁰, CONR⁹R¹⁰, COR⁹ et CO₂R⁹ ;
R⁸ est choisi entre un atome d'hydrogène, des groupes -SO (alkyle en C₁ à C₆), -SO₂-(alkyle en C₁ à C₆), -SO-aryle, SO₂-aryle, CF₃, phényle, phényl-(alkyle en C₁ ou C₂), pyridyle, tétrazolyle, oxazolyle, thiazolyle et alkyle en C₁ à C₆ substitué avec un ou plusieurs substituants, de préférence avec zéro à deux substituants choisis, indépendamment, entre des substituants hydroxy, oxo, alkoxy en C₁ à C₆, phényl-(alkoxy en C₁ à C₃), phényle, cyano, chloro, bromo, iodo, NR⁹R¹⁰, NR₉COR₁₀, NR⁹CO₂R₁₀, CONR⁹R¹⁰, COR⁹ et CO₂R⁹ ;
chaque groupe R⁹ et chaque groupe R¹⁰ sont choisis, indépendamment, entre un atome d'hydrogène, des groupes alkyle en C₁ à C₆, hydroxyalkyle en C₁ à C₆, phényle et CF₃ ;
ou bien R⁹ et R¹⁰, lorsque R³ représente un groupe NR⁹R¹⁰ ou CONR⁹R¹⁰, peuvent former, conjointement avec l'atome d'azote auquel ils sont fixés, un noyau hétérocyclique facultativement substitué qui contient au moins un atome d'azote ;
et les groupes phényle dans la définition de R⁵, R⁶, R⁷ et R⁸ et le groupement phényle du groupe phényl-(alkyle en C₁ ou C₂) dans la définition de R⁵, R⁶, R⁷ et R⁸ peuvent être facultativement substitués avec un ou plusieurs substituants, de préférence avec zéro à deux substituants, qui sont choisis, indépendamment, entre des substituants halogéno, hydroxy, alkoxy en C₁ à C₆ facultativement substitué avec un à sept atomes de fluor, de préférence avec zéro à trois atomes de fluor, et alkyle en C₁ à C₆ facultativement substitué avec un à sept atomes de fluor, de préférence avec zéro à trois atomes de fluor ;
ou un de ses sels pharmaceutiquement acceptables.

2. Composé suivant la revendication 1, dans lequel R³ représente un noyau hétérocyclique facultativement substitué, ou un groupe alkyle substitué avec un noyau hétérocyclique facultativement substitué, dans lequel ledit noyau hétérocyclique est choisi parmi les suivants : pyrimidinyle, benzoxazolyle, 2,3-dihydro-3-oxobenzisosulfonazol-2-yle, morpholine-1-yle, thiomorpholine-1-yle, benzofurannyle, benzothiényle, indolyle, isoindolyle, isoquinolinyle, furyle, pyridyle, isothiazolyle, oxazolyle, triazolyle, tétrazolyle, quinolyle, thiazolyle et thiényle, et les groupes de formules 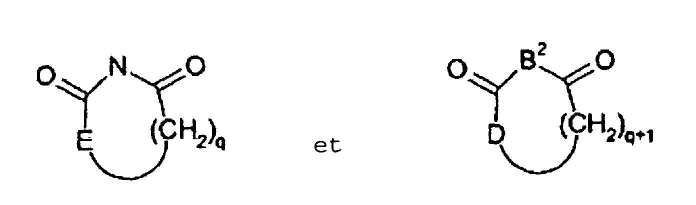 dans lesquelles B² et D sont choisis entre les atomes de carbone, d'oxygène et d'azote, et au moins l'un de B² et D est autre qu'un atome de carbone ; E représente un atome de carbone ou d'azote ; q représente un nombre entier de 1 à 5 ; n'importe lequel des atomes de carbone desdits groupes (CH₂)_{q} et (CH₂)_{q+1} peut être facultativement substitué avec un substituant alkyle en C₁ à C₆ ou spiroalkyle en C₁ à C₆ ; et n'importe quelle paire des atomes de carbone desdits groupes (CH₂)_{q} et (CH₂)_{q+1} peut être pontée par une liaison d'un ou deux atomes de carbone,
ou n'importe quelle paire d'atomes de carbone adjacents desdits groupes (CH₂)_{q} et (CH₂)_{q+1} peut former, conjointement avec un à trois atomes de carbone qui ne sont pas des membres du noyau contenant un groupe carbonyle, un noyau carbocyclique condensé en C₃ à C₅.

3. Composé suivant la revendication 1, dans lequel Q représente un groupe carbonyle.

4. Composé suivant la revendication 1, dans lequel B représente un groupe éthylène, A représente un groupe CH et G représente un groupe NHCH₂.

5. Composé suivant la revendication 1, dans lequel B représente un groupe éthylène, A représente un groupe CH et G représente un groupe SCH₂.

6. Composé suivant la revendication 1, dans lequel B est absent, G représente un groupe NH et A représente un groupe CH₂.

7. Composé suivant la revendication 1, dans lequel W représente un groupe éthylène.

8. Composé suivant la revendication 1, dans lequel R⁴ représente un groupe phényle.

9. Composé suivant la revendication 1, dans lequel R⁴ représente un groupe phényle et R⁸ représente un atome d'hydrogène.

10. Composé suivant la revendication 1, dans lequel p est égal à 1.

11. Composé suivant la revendication 1, dans lequel R² représente un groupe alkyle en C₁ à C₆.

12. Composé suivant la revendication 1, dans lequel R⁴ représente un groupe 2-, 3- ou 4-pyridyle.

13. Composé suivant la revendication 1, dans lequel R² et R¹² sont choisis, indépendamment, entre des groupes méthyle et éthyle.

14. Composé suivant la revendication 1, dans lequel Y représente un groupe CH et Z représente un atome d'azote.

15. Composé suivant la revendication 1, dans lequel Y représente un atome d'azote et Z représente un groupe CH.

16. Composé suivant la revendication 1, dans lequel Q représente un groupe C=S.

17. Composé qui est choisi parmi les isomères et mélanges d'isomères des composés suivants, lesdits isomères ou mélanges d'isomères ayant la stéréochimie représentée dans la formule structurale (I) :
6-méthoxy-1-méthyl-7-[(2-phénylpipéridine-3-ylamino)-méthyl]-3,4-dihydro-1H-[1,5]naphthyridine-2-one ;
6-méthoxy-1-méthyl-7-[(6-méthyl-2-phénylpipéridine-3-ylamino)-méthyl]-3,4-dihydro-1H-[1,5]naphthyridine-2-one ;
7-[(6-éthyl-2-phénylpipéridine-3-ylamino)-méthyl]-6-méthoxy-1-méthyl-3,4-dihydro-1H-[1,5]naphthyridine-2-one ;
6-méthoxy-1-méthyl-7-[(2-phényl-6-propylpipéridine-3-ylamino)-méthyl]-3,4-dihydro-1H-[1,5]naphthyridine-2-one ;
7-[((2S,3S,6S)-6-isopropyl-2-phénylpipéridine-3-ylamino)-méthyl]-6-méthoxy-1-méthyl-3,4-dihydro-1H-[1,5]naphthyridine-2-one ;
et leurs sels pharmaceutiquement acceptables.

18. Composé de formule (I) suivant la revendication 1, destiné à être utilisé comme médicament.

19. Utilisation d'un composé de formule (I) suivant la revendication 1 dans la production d'un médicament destiné au traitement ou à la prévention d'un trouble ou d'une affection choisi dans le groupe consistant en des troubles de l'humeur, tels que la dépression, ou plus particulièrement, des troubles dépressifs, par exemple des troubles dépressifs majeurs épisodiques uniques ou récidivants, des troubles dysthymiques, la névrose dépressive et la dépression névrotique, la dépression mélancolique, comprenant l'anorexie, la perte de poids, l'insomnie, l'éveil matinal précoce et un retard psychomoteur, la dépression atypique (ou dépression réactive), comprenant une augmentation de l'appétit, l'hypersomnie, l'agitation psychomotrice ou l'irritabilité, un trouble affectif saisonnier et la dépression pédiatrique ; ou des troubles bipolaires ou la dépression maniaque, par exemple le trouble bipolaire I, le trouble bipolaire II et un trouble cyclothymique, un trouble de la conduite et un trouble de comportement disruptif, des troubles du type de l'anxiété tels que la panique avec ou sans agoraphobie, l'agoraphobie sans antécédents de troubles de panique, des phobies spécifiques, par exemple des phobies vis-à-vis d'animaux spécifiques, l'anxiété sociale, les phobies sociales, le syndrome obsessionnel compulsif, des troubles dus à un stress comprenant des troubles dus à un stress post-traumatique et un trouble dû à un stress aigu et des troubles d'anxiété généralisée ; un comportement de personnalité à la limite de la normale ; la schizophrénie et d'autres troubles psychotiques, par exemple des troubles schizophréniformes, des troubles schizoaffectifs, des troubles délirants, des troubles psychotiques brefs, des troubles psychotiques partagés, des troubles psychotiques avec des délires ou des hallucinations, des épisodes psychotiques d'anxiété, l'anxiété associée à la psychose, des troubles psychotiques de l'humeur tels que le trouble dépressif majeur grave ; des troubles de l'humeur associés à des troubles psychotiques tels que la manie aiguë et la dépression associée à un trouble bipolaire, des troubles de l'humeur associés à la schizophrénie ; des perturbations comportementales associées au retard mental, à un trouble autiste et un trouble de la conduite.

20. Utilisation d'un composé de formule (I) suivant la revendication 1 dans la production d'un médicament destiné au traitement ou à la prévention d'un trouble ou d'une affection choisie dans le groupe consistant en le délire, la démence et des troubles amnésiques et d'autres troubles cognitifs ou neurodégénératifs, tels que la maladie de Parkinson (PD), la maladie de Huntington (HD), la maladie d'Alzheimer, la démence sénile, la démence de type Alzheimer, des troubles de la mémoire, la démence vasculaire et d'autres démences, par exemple dus à la maladie provoquée par le VIH, un traumatisme crânien, la maladie de Parkinson, la maladie de Huntington, la maladie de Pick, la maladie de Creutzfeldt-Jakob ou dus à des étiologies multiples ; des troubles du mouvement tels que des akinésies, des dyskinésies, y compris des dyskinésies paroxystiques familiales, les spasticités, le syndrome de Tourette, le syndrome de Scott, la paralysie et le syndrome akinésique rigide ; des troubles du mouvement extrapyramidaux tels que des troubles du mouvement induits par des médicaments, par exemple parkinsonisme induit par les neuroleptiques, le syndrome malin des neuroleptiques, la dystonie aiguë induite par les neuroleptiques, l'akathisie aiguë induite par les neuroleptiques, la dyskinésie tardive induite par les neuroleptiques et les tremblements posturaux induits par des médicaments, des troubles dus à des substances provenant de l'utilisation de l'alcool, d'amphétamines (ou de substances analogues aux amphétamines), de la caféine, du cannabis, de la cocaïne, d'hallucinogènes, d'agents à inhaler et d'agents propulseurs d'aérosols, de la nicotine, d'opioïdes, de dérivés de phénylglycidine, de sédatifs, d'hypnotiques et d'anxiolytiques, troubles dus à des substances qui comprennent la dépendance et un abus, l'intoxication, le sevrage, le délire dû à l'intoxication et le délire dû au sevrage d'accoutumances telles que le jeu ; l'épilepsie ; le syndrome de Down ; la douleur aiguë, la douleur chronique et la migraine ; des maladies démyélinisantes telles que la sclérose en plaques (MS) et la sclérose amylolatérale (ALS), la neuropathie périphérique, par exemple la neuropathie diabétique et la neuropathie induite par la chimiothérapie et les névralgies postherpétiques, les névralgies du trijumeau, les névralgies segmentaires ou intercostales et d'autres névralgies ; et des troubles vasculaires cérébraux dus à une altération vasculaire cérébrale aiguë ou chronique telle qu'un infractus cérébral, une hémorragie sous-arachnoïdienne ou un oedème cérébral.

21. Utilisation d'un composé de formule (I) suivant la revendication 1 dans la production d'un médicament destiné au traitement ou à la prévention d'un trouble ou d'une affection choisi dans le groupe consistant en des maladies respiratoires, en particulier celles associées à un excès de sécrétion de mucus, telles qu'une maladie obstructive chronique des voies aériennes, la bronchopneumonie, la bronchite chronique, la fibrose kystique, le syndrome de détresse respiratoire de l'adulte et un bronchospasme ; des maladies inflammatoires telles qu'une maladie intestinale inflammatoire, le psoriasis, le syndrome de Reiter, le syndrome de Raynaud, des anthropathies, la fibrosite, l'ostéoarthrite, la polyarthrite rhumatoide, l'arthrite psoriasique, l'asthme, le prurit et les érythèmes solaires ; des infections par le virus de l'immunodéficience humaine (VIH), des allergies telles que l'eczéma, la rhinite et d'autres allergies, des troubles d'hypersensibilité telles que l'empoisonnement par le sumac vénéneux ; des maladies ophtalmiques telles que la conjonctivite, la conjonctivite printanière, etc. ; des affections ophtalmiques associées à une prolifération cellulaire telles que la vitrorétinopathie proliférative ; des maladies cutanées telles que la dermatite de contact, la dermatite atopique, l'urticaire et d'autres dermatites eczématoïdes.

22. Utilisation d'un composé de formule (I) suivant la revendication 1 dans la production d'un médicament destiné au traitement ou à la prévention d'un trouble ou d'une affection choisie dans le groupe consistant en des néoplasmes, comprenant des tumeurs du sein, des carcinomes gastriques, des lymphomes gastriques, des neuroganglioblastomes et des carcinomes à petites cellules telles que le cancer du poumon à petites cellules.

23. Utilisation d'un composé de formule (I) suivant la revendication 1 dans la production d'un médicament destiné à traitement ou à la prévention d'un trouble ou d'une affection choisi dans le groupe consistant en des troubles gastro-intestinaux (GI), comprenant des troubles gastro-intestinaux inflammatoires tels que des troubles intestinaux inflammatoires provoqués par *Helicobacter pylori* et des maladies du tractus GI telles que la gastrite, les ulcères gastroduodénaux, des troubles associés au contrôle neuronal des viscères, la colite ulcérative, la maladie de Crohn, le syndrome du colon irritable et les vomissements.

24. Utilisation d'un composé de formule (I) suivant la revendication 1 dans la production d'un médicament destiné au traitement ou à la prévention d'un trouble ou d'une affection choisi dans le groupe consistant en des troubles somatiques dus à un stress, la dystrophie sympathique réflexe telle que le syndrome épaule/main ; des réactions immunologiques néfastes telles que le rejet de tissus transplantés et des troubles dus à une simulation ou suppression immunitaire telle que le lupus érythémateux disséminé ; une extravasation plasmatique résultant de la chimiothérapie avec une cytokine ; des troubles de la fonction de la vessie tels que la cystite, l'hyperréflexie du muscle vésical, l'inflammation du tractus urinaire et l'incontinence, comprenant l'incontinence urinaire d'effort, l'hyperactivité de la vessie, l'incontinence due à un stress et l'incontinence mixte ; des maladies comportant une fibrose et des maladies du collagène telles que la sclérodermie et la fasciolase à cellules oesinophiles ; des troubles du flux sanguin provoqués par une vasodilatation et des maladies angiospastiques telles que l'angine de poitrine et la maladie de Raynaud ; l'angiogenèse ; des troubles cardiovasculaires ; des troubles de l'alimentation tels que l'anorexie mentale et la boulimie mentale ; un trouble d'hyperactivité avec déficit d'attention ; le syndrome de fatigue chronique ; des dysfonctionnements sexuels comprenant l'éjaculation précoce et un dysfonctionnement de l'érection masculine ; le syndrome prémenstruel et un trouble dysphorique prémenstruel ; la fibromyalgie ; et des maladies rhumatismales telles que la fibrosite.

25. Composition pharmaceutique comprenant un composé suivant la revendication 1 et un support pharmaceutiquement acceptable.

26. Composé de formule 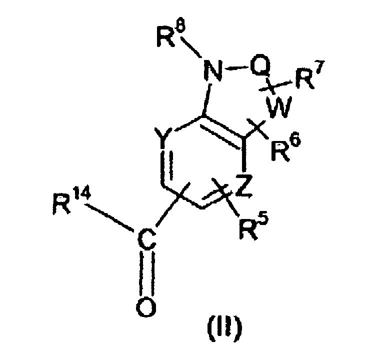
dans laquelle Q représente un groupe C=S ou C=O ;
Y représente un atome de N et Z représente un groupe CH ou Y représente un groupe CH et Z représente un atome de N ;
W représente un groupe de liaison carboné (c'est-à-dire méthylène) ou un groupe de liaison saturé ou insaturé de deux ou trois atomes de carbone, chacun des groupes W précités pouvant être facultativement substitué avec un substituant R⁷ ou avec deux substituants R⁷ et R⁶, ou bien W représente un groupe de liaison d'un atome de carbone qui forme, conjointement avec une chaîne de 2, 3, 4 ou 5 atomes de carbone, un noyau spiro tri-, tétra-, penta- ou hexagonal, respectivement ;
ou W représente un groupe de liaison à chaîne saturée de deux atomes de carbone qui forment, conjointement avec une chaîne de 1, 2 ou 3 atomes de carbone distincts, un noyau condensé tri-, tétra- ou pentagonal, respectivement ;
ou W représente un groupe de liaison à chaîne de deux atomes de carbone saturée, dans lequel un des deux atomes de carbone de la chaîne forme, conjointement avec une chaîne de 2, 3, 4 ou 5 atomes de carbone distincts, un noyau spiro tri-, tétra-, penta- ou hexagonal, respectivement ;
R⁵ est choisi entre un atome d'hydrogène, des groupes -SO(alkyle en C₁ à C₆), -SO₂-(alkyle en C₁ à C₆), -SO-aryle, -SO₂-aryle, CF₃, halogéno, phényle, phényl-(alkyle en C₁ ou C₂), hydroxy, aryloxy, hétéroaryloxy, pyridyle, tétrazolyle, oxazolyle, thiazolyle, alkoxy en C₁ à C₆ facultativement substitué avec un à sept atomes de fluor, de préférence avec zéro à trois atomes de fluor, alkyle en C₁ à C₆ facultativement substitué avec un à sept atomes de fluor, de préférence avec zéro à trois atomes de fluor, et alkyle en C₁ à C₆ substitué avec un ou plusieurs substituants, de préférence avec zéro à deux substituants choisis, indépendamment, entre des substituants hydroxy, oxo, alkoxy en C₁ à C₆, phényl-(alkoxy en C₁ à C₃), phényle, cyano, chloro, bromo, iodo, NR⁹R¹⁰, NR⁹COR¹⁰, NR⁹CO₂R¹⁰, CONR⁹R¹⁰, COR⁹ et CO₂R⁹ ;
R⁶ et R⁷ sont choisis, indépendamment, entre des groupes -SO (alkyle en C₁ à C₆), -SO₂-(alkyle en C₁ à C₆), -SO-aryle, -SO₂-aryle, CF₃, halogéno, phényle, phényl-(alkyle en C₁ ou C₂), hydroxy, aryloxy, hétéroaryloxy, pyridyle, tétrazolyle, oxazolyle, thiazolyle, alkoxy en C₁ à C₆ facultativement substitué avec un à sept atomes de fluor, de préférence avec zéro à trois atomes de fluor, alkyle en C₁ à C₆ facultativement substitué avec un à sept atomes de fluor, de préférence avec zéro à trois atomes de fluor, et alkyle en C₁ à C₆ substitué avec un ou plusieurs substituants, de préférence avec zéro à deux substituants choisis, indépendamment, entre des substituants hydroxy, oxo, alkoxy en C₁ à C₆, phényl-(alkoxy en C₁ à C₃), phényle, cyano, chloro, bromo, iodo, NR⁹R¹⁰, NR⁹COR¹⁰, NR⁹CO₂R¹⁰, CONR⁹R¹⁰, COR⁹ et CO₂R⁹ ;
R⁸ est choisi entre un atome d'hydrogène, des groupes -SO(alkyle en C₁ à C₆), -SO₂-(alkyle en C₁ à C₆), -SO-aryle, -SO₂-aryle, CF₃, phényle, phényl-(alkyle en C₁ ou C₂), pyridyle, tétrazolyle, oxazolyle, thiazolyle et alkyle en C₁ à C₆ substitué avec un ou plusieurs substituants, de préférence avec zéro à deux substituants choisis, indépendamment, entre des substituants hydroxy, oxo, alkoxy en C₁ à C₆, phényl-(alkoxy en C₁ à C₃), phényle, cyano, chloro, bromo, iodo, NR⁹R¹⁰, NR⁹COR¹⁰, NR⁹CO₂R¹⁰, CONR⁹R¹⁰, COR⁹ et CO₂R⁹ ;
chaque groupe R⁹ et chaque groupe R¹⁰ sont choisis, indépendamment, entre un atome d'hydrogène et des groupes alkyle en C₁ à C₆, hydroxy-(alkyle en C₁ à C₆), phényle et CF₃ ;
et les groupes phényle dans la définition de R⁵, R⁶, R⁷ et R⁸ et le groupement phényle du groupe phényl-(alkyle en C₁ ou C₂) dans la définition de R⁵, R⁶, R⁷ et R⁸, peuvent être facultativement substitués avec un ou plusieurs substituants, de préférence avec zéro à deux substituants, qui sont choisis, indépendamment, entre des substituants halogéno, hydroxy, alkoxy en C₁ à C₆ facultativement substitué avec un à sept atomes de fluor, de préférence avec zéro à trois atomes de fluor, et alkyle en C₁ à C₆ facultativement substitué avec un à sept atomes de fluor, de préférence avec zéro à trois atomes de fluor ; et
R¹⁴ représente un atome d'hydrogène, un groupe alkyle en C₁ à C₆ ou CF₃ ;
ou un de ses sels pharmaceutiquement acceptables.

27. Composé suivant la revendication 26, choisi dans le groupe consistant en :
3-méthoxy-8-méthyl-7-oxo-5,6,7,8-tétrahydro-[1,8]naphthyridine-2-carbaldéhyde ;
2-méthoxy-5-méthyl-6-oxo-5,6,7,8-tétrahydro-[1,5]naphthyridine-3-carbaldéhyde ;
et leurs sels pharmaceutiquement acceptables.
